# EUROPEAN PATENT SPECIFICATION

(11) **EP 3 168 221 B1**
(45) Date of publication and mention of the grant of the patent: **28.06.2023**
(21) Application number: 15819320.1
(22) Date of filing: 18.03.2015
(51) Int. Cl.: C07D 487/04, C07D 491/04, C07D 495/04, C07D 471/04, H10K 85/60

(54) **COMPOUND, ORGANIC OPTOELECTRONIC DIODE COMPRISING SAME, AND DISPLAY**
VERBINDUNG, ORGANISCHE OPTOELEKTRONISCHE DIODE DAMIT UND ANZEIGE
COMPOSÉ, DISPOSITIF OPTOÉLECTRONIQUE ORGANIQUE COMPRENANT LEDIT COMPOSÉ ET DISPOSITIF D'AFFICHAGE

(30) Priority: 09.07.2014 KR 20140086108
(43) Date of publication of application: 17.05.2017
(73) Proprietor: Samsung SDI Co., Ltd., Gyeonggi-do 446-902 (KR)
(72) Inventor: KIM, Byung-Ku, Suwon-si Gyeonggi-do 443-803 (KR); KANG, Dong-Min, Suwon-si Gyeonggi-do 443-803 (KR); KO, Chae-Hyuk, Suwon-si Gyeonggi-do 443-803 (KR); KIM, Young-Kwon, Suwon-si Gyeonggi-do 443-803 (KR); KIM, Chang-Woo, Suwon-si Gyeonggi-do 443-803 (KR); YU, Eun-Sun, Suwon-si Gyeonggi-do 443-803 (KR); JEONG, Soo-Young, Suwon-si Gyeonggi-do 443-803 (KR)
(74) Representative: Michalski Hüttermann & Partner Patentanwälte mbB
(86) International application number: PCT/KR2015/002638
(87) International publication number: WO 2016/006791

(56) References cited:
- CN-A- 102 863 451
- JP-A- 2006 151 930
- US-A1- 2009 004 485
- VISHNU BASETTI ET AL.: 'A facile synthesis of tetracyclic benzo-pyridonaphthyridines by domino reaction' TETRAHEDRON LETTERS vol. 54, 2013, pages 2014 - 2017, XP028998230
- LAURENT BOUFFEIER ET AL.: 'Electrochemistry and bioactivity relationshio of 6-substituted -4H-Pyrido[4,3,2-kl] acridin-4-one antitumor drug candidates' BIOELECTROCHEMISTRY vol. 88, 2012, pages 103 - 109, XP055383159
- LAURENT BOUFFIER ET AL.: 'Reactivity of Pyrido[4,3,2-kl]acridines: Regioselectivity Formulation of 6-Substituted Derivatives' J. ORG. CHEM. vol. 69, 2004, pages 8144 - 8147, XP055383161
- GRZEGORZ CHOLEWINSKI ET AL.: 'Natural and synthetic acridines/acridones as antitumor agents: their biological activities and methods of synthesis' PHARMACOLOGICAL REPORTS vol. 63, 2011, pages 305 - 336, XP055383164
- BASETTI VISHNU ET AL: "A facile synthesis of tetracyclic benzo-pyridonaphthyridines by domino reaction", TETRAHEDRON LETTERS, ELSEVIER LTD, AMSTERDAM, NL, vol. 54, no. 15, 13 February 2013 (2013-02-13), pages 2014-2017, XP028998230, ISSN: 0040-4039, DOI: 10.1016/J.TETLET.2013.02.016
- Evelyne Delfourne ET AL: "The First Synthesis of the Pentacyclic Pyridoacridine Marine Alkaloids: Arnoamines A and B", The Journal of Organic Chemistry, vol. 65, no. 18, 1 September 2000 (2000-09-01), pages 5476-5479, XP055281574, US ISSN: 0022-3263, DOI: 10.1021/jo000011a

## Description

### [Technical Field]

A compound, an organic optoelectronic device and a display device are disclosed.

### [Background Art]

An organic optoelectronic device is a device that converts electrical energy into photoenergy, and vice versa.

An organic optoelectronic device may be classified as follows in accordance with its driving principles. One is an optoelectronic device where excitons are generated by photoenergy, separated into electrons and holes, and the electrons and holes are transferred to different electrodes to generate electrical energy, and the other is a light emitting device where a voltage or a current is supplied to an electrode to generate photoenergy from electrical energy.

US 2009/0004485 A1 refers to an organic material having a 6-member ring structure represented
by the following formulae (I), 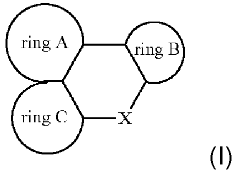 wherein:
ring A, ring B, and ring C each include substituted or un-substituted aromatic rings comprising 6 to 60 carbon atoms, or substituted or un-substituted heteroaromatic rings comprising 4 to 60 carbon atoms, and ring A and ring C form a fused aromatic or heteroaromatic structure; X is a carbon atom, a nitrogen atom, a sulfur atom, a silicon atom, an oxygen atom, a phosphorus atom, a selenium atom, or a germanium atom.

CN 102863451 A refers to a chromene and naphthyridine fluorescent compound, in particular to a preparation method and the usage of a triphenylamine molecule-containing chromene and naphthyridine fluorescent compound, belonging to the field of chemical technologies. Based on the preparation of the chromene and naphthyridine, a series of triphenylamine molecule-containing chromene and naphthyridine derivatives by introducing the triphenylamine molecules, and the fluorescence of the derivatives is tested; and the chromene and naphthyridine fluorescent compound can be applied to the technology for dyeing, marking and/ or detecting organic molecules, and used for the application of fluorescent molecules in fluorescent material. BASETTI VISHNU ET AL: "A facile synthesis of tetracyclic benzo-pyridonaphthyridines by domino reaction", TETRAHEDRON LETTERS, ELSEVIER LTD, AMSTERDAM, NL, vol. 54, no. 15, 13 February 2013, pages 2014-2017, refers inter alia to a methodology for the synthesis of compounds having the following structure:

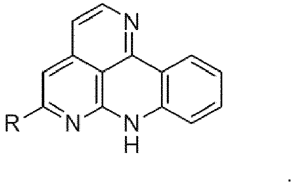

EVELYNE DELFOURNE ET AL: "The First Sythesis of the Pentacyclic Pyridoacridine Marine Alkaloids: Arnoamines A and B", The Journal of Organic Chemistry, vol. 65, no. 18, 1 September 2000, pages 5476-5479, refers inter alia to the sythesis of compounds having the following structures:

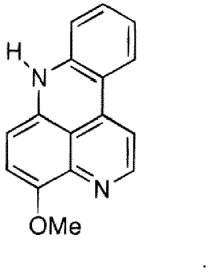

Examples of an organic optoelectronic device may be an organic photoelectric device, an organic light emitting diode, an organic solar cell and an organic photo conductor drum.

Of these, an organic light emitting diode (OLED) has recently drawn attention due to an increase in demand for flat panel displays. Such an organic light emitting diode converts electrical energy into light by applying current to an organic light emitting material. It has a structure in which an organic layer is interposed between an anode and a cathode. Herein, an organic layer may include an emission layer and optionally an auxiliary layer, and the auxiliary layer may include, for example at least one selected from a hole injection layer, a hole transport layer, an electron blocking layer, an electron transport layer, an electron injection layer and a hole blocking layer in order increase efficiency and stability of an organic light emitting diode.

Performance of an organic light emitting diode may be affected by characteristics of the organic layer, and among them, may be mainly affected by characteristics of an organic material of the organic layer.

Particularly, development for an organic material being capable of increasing hole and electron mobility and simultaneously increasing electrochemical stability is needed so that the organic light emitting diode may be applied to a large-size flat panel display.

### [DISCLOSURE]

### [Technical Problem]

A compound for an organic optoelectronic device being capable of realizing an organic optoelectronic device having characteristics such as high efficiency, a long life-span, and the like is provided.

An organic optoelectronic device and a display device including the compound for an organic optoelectronic device are provided.

### [Technical Solution]

In one embodiment of the present invention, a compound represented by
one of the following Chemical Formulae 15 to 27 is provided wherein the compound is used for an organic optoelectronic device: 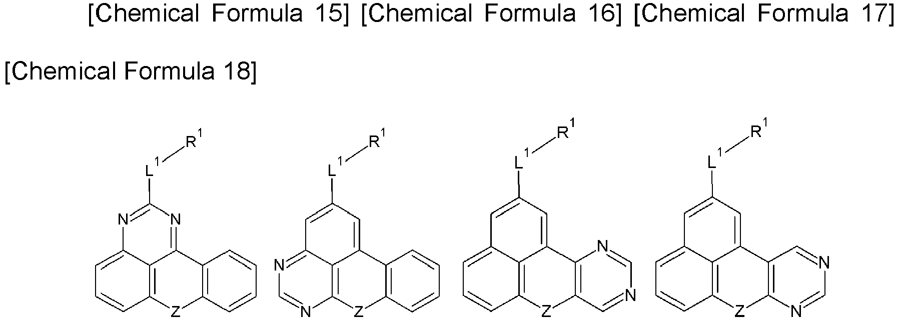 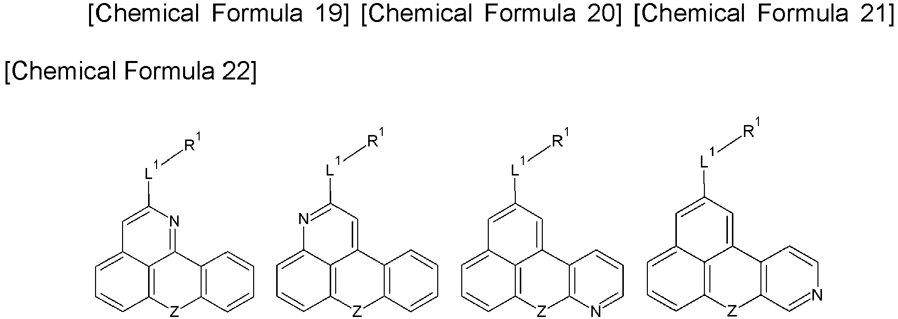 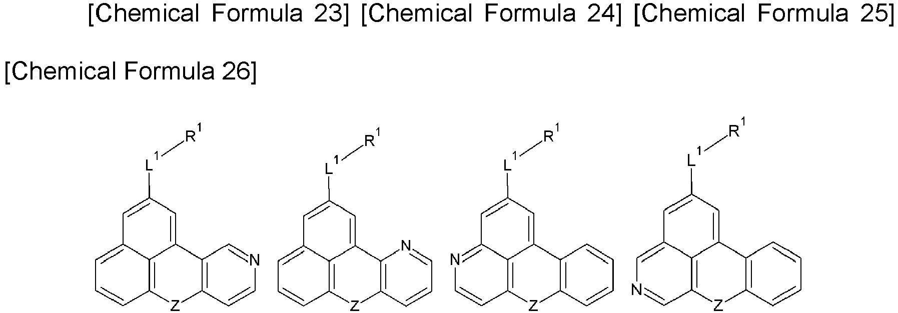 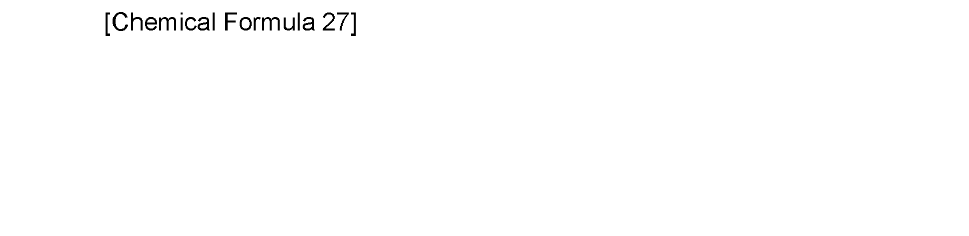 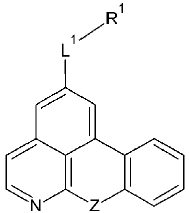
wherein, in the Chemical Formulae 15 to 27,
Z is N-L⁴-R^{b},
L¹ and L⁴ are independently, a single bond, a substituted or unsubstituted C6 to C30 arylene group, a substituted or unsubstituted C2 to C30 heterocyclic group, or a combination thereof, and
wherein R¹ and R^{b} are independently a group selected from substituted or unsubstituted groups listed in the following Group II: 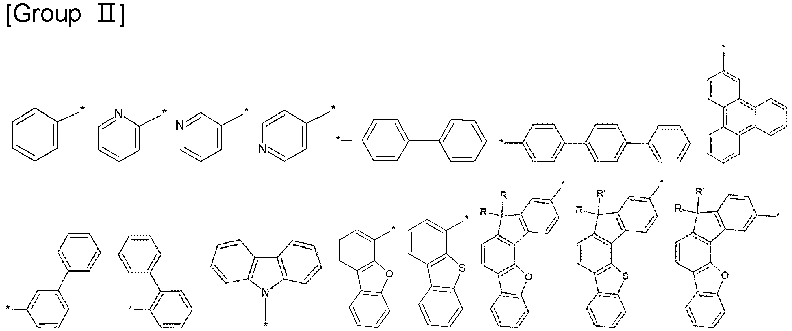 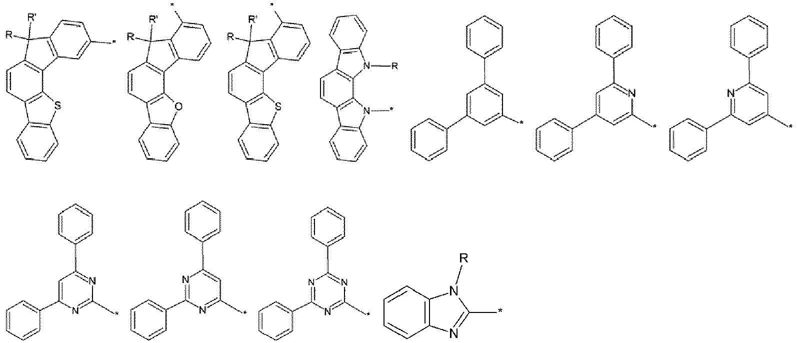
wherein in Group II,
R and R' are independently hydrogen, deuterium, a substituted or unsubstituted C1 to C30 alkyl group, a substituted or unsubstituted C6 to C30 aryl group, a substituted or unsubstituted C2 to C30 heteroaryl group, or a combination thereof, and * is a linking point.

In another embodiment of the present invention, a compound represented by one of the following compounds listed in the following Group III as given in the specification is provided.

The compound according to one embodiment of the present invention is used for an organic optoelectronic device.

In another embodiment of the present invention, an organic optoelectronic device includes an anode and a cathode facing each other and at least one organic layer positioned between the anode and the cathode, wherein the organic layer includes the compound.

In yet another embodiment of the present invention, a display device including the organic optoelectronic device is provided.

### [Advantageous Effects]

An organic optoelectronic device having high efficiency and long life-span may be realized.

### [Description of the Drawings]

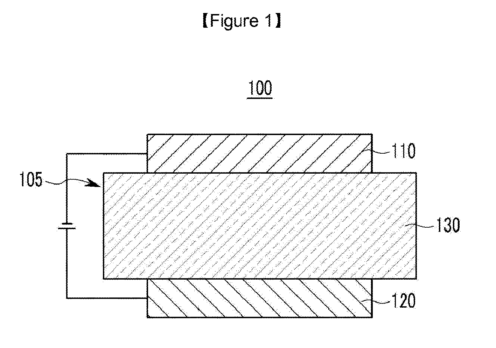

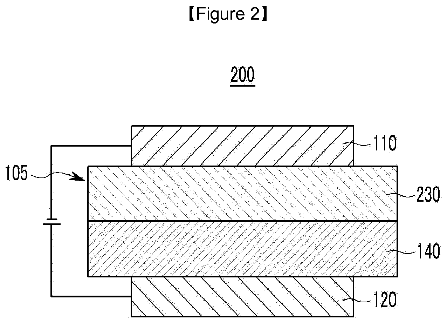

FIGS. 1 and 2 are cross-sectional views showing organic light emitting diodes according to various embodiments.

### <Description of Symbols>

| | | | |
|---|---|---|---|
| 100: | organic light emitting diode | 200: | organic light emitting diode |
| 105: | organic layer | | |
| 110: | cathode | | |
| 120: | anode | | |
| 130: | emission layer | 230: | emission layer |
| 140: | hole auxiliary layer | | |

### [Best Mode]

Hereinafter, embodiments of the present invention are described in detail. However, these embodiments are exemplary, and this disclosure is not limited thereto.

As used herein, when a definition is not otherwise provided, the term "substituted" refers to one substituted with a substituent selected from deuterium, a halogen, a hydroxy group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C3 to C30 cycloalkyl group, a C6 to C30 aryl group, a C1 to C20 alkoxy group, a fluoro group, a C1 to C10 trifluoroalkyl group such as a trifluoromethyl group and the like or a cyano group, instead of at least one hydrogen of a substituent or a compound.

In the present specification, when specific definition is not otherwise provided, "hetero" refers to one including 1 to 3 hetero atoms selected from N, O, S, P, and Si, and remaining carbons in one functional group.

In the present specification, when a definition is not otherwise provided, "alkyl group" refers to an aliphatic hydrocarbon group. The alkyl group may be "a saturated alkyl group" without any double bond or triple bond.

The alkyl group may be a C1 to C20 alkyl group. More specifically, the alkyl group may be a C1 to C10 alkyl group or a C1 to C6 alkyl group. For example, a C1 to C4 alkyl group may have 1 to 4 carbon atoms in an alkyl chain which may be selected from methyl, ethyl, propyl, iso-propyl, n-butyl, iso-butyl, sec-butyl, and t-butyl.

Specific examples of the alkyl group may be a methyl group, an ethyl group, a propyl group, an isopropyl group, a butyl group, an isobutyl group, a t-butyl group, a pentyl group, a hexyl group, a cyclopropyl group, a cyclobutyl group, a cyclopentyl group, a cyclohexyl group, and the like.

As used herein, the term "aryl group" refers to a substituent including all element of the cycle having p-orbitals which form conjugation, and may be monocyclic or fused ring polycyclic (i.e., rings sharing adjacent pairs of carbon atoms) functional group.

As used herein, the term "heterocyclic group" may refer to an aryl group or a cycloalkyl group including 1 to 3 hetero atoms selected from N, O, S, P, and Si and remaining carbons in one functional group. Particularly, when the aryl group includes a heteroatom, it may be referred to as a "heteroaryl group."

When the heterocyclic group is a fused ring, the entire ring or each ring of the heterocyclic group may include a hetero atom.

More specifically, the substituted or unsubstituted C6 to C30 aryl group and/or the substituted or unsubstituted C2 to C30 heteroaryl group may be a substituted or unsubstituted phenyl group, a substituted or unsubstituted naphthyl group, a substituted or unsubstituted anthracenyl group, a substituted or unsubstituted phenanthrylene group, a substituted or unsubstituted naphthacenyl group, a substituted or unsubstituted pyrenyl group, a substituted or unsubstituted biphenyl group, a substituted or unsubstituted p-terphenyl group, a substituted or unsubstituted m-terphenyl group, a substituted or unsubstituted chrysenyl group, a substituted or unsubstituted triphenylenyl group, a substituted or unsubstituted perylenyl group, a substituted or unsubstituted indenyl group, a substituted or unsubstituted furanyl group, a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthyridinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, a substituted or unsubstituted phenoxazinyl group, a substituted or unsubstituted fluorenyl group, a substituted or unsubstituted carbazolyl group, a substituted or unsubstituted dibenzofuranyl group, a substituted or unsubstituted dibenzothiophenyl group, or a combination thereof, but is not limited thereto.

In the present specification, a single bond indicates a directly linking bond not via carbon or a hetero atom other than the carbon, and specifically, when L is the single bond, a substituent linked to the L is directly linked to a core. In other words, the single bond in the present specification does not indicate methylene and the like via carbon.

In the specification, hole characteristics refer to characteristics capable of donating an electron to form a hole when electric field is applied, and characteristics that hole formed in the anode is easily injected into the emission layer and transported in the emission layer due to conductive characteristics according to HOMO level.

In addition, electron characteristics refer to characteristics capable of accepting an electron to form a hole when electric field is applied, and characteristics that electron formed in the cathode is easily injected into the emission layer and transported in the emission layer due to conductive characteristics according to LUMO level.

Hereinafter, a compound according to one embodiment is described.

In one embodiment of the present invention, a compound represented by
one of the following Chemical Formulae 15 to 27 is provided, wherein the compound is used for an organic optoelectronic device:

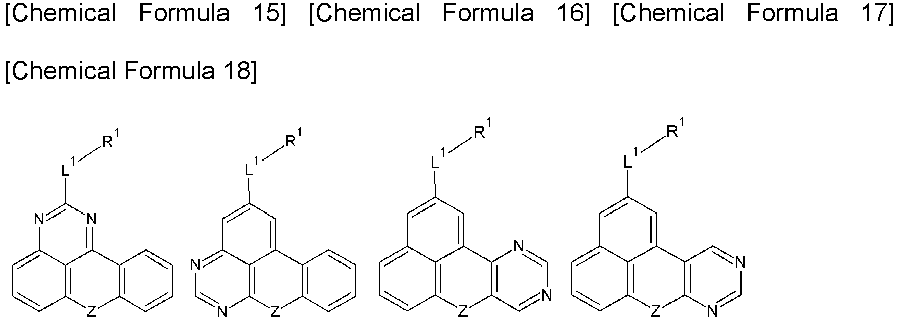

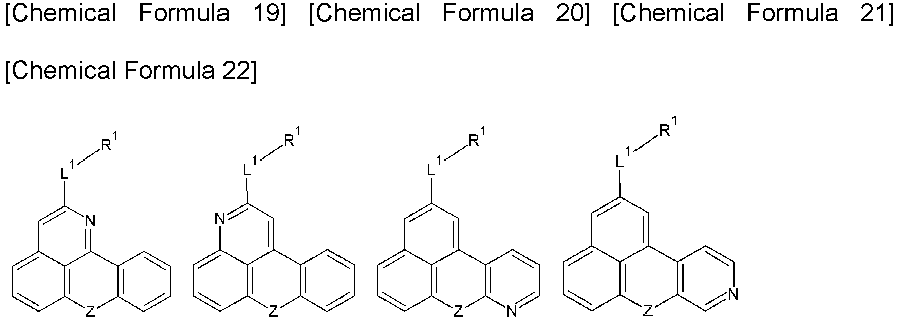

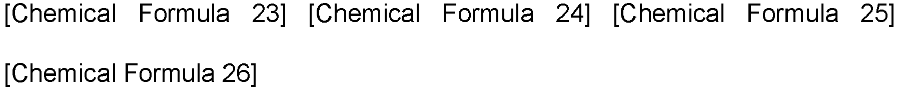

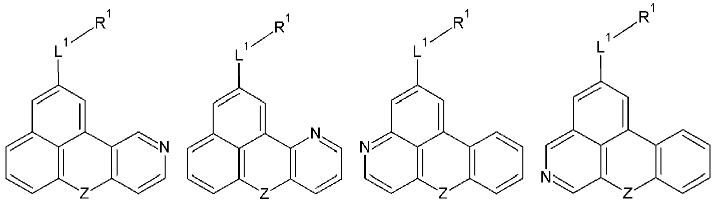

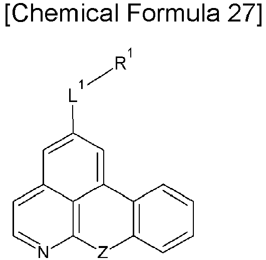

In Chemical Formulae 15 to 27,
Z is N-L⁴-R^{b},
L¹ and L⁴ are independently, a single bond, a substituted or unsubstituted C6 to C30 arylene group, a substituted or unsubstituted C2 to C30 heterocyclic group, or a combination thereof, and
R¹ and R^{b} are according to the claims.
L¹ and L⁴ are independently a single bond or a group selected from substituted or unsubstituted groups listed in the following Group I, but are not limited thereto.

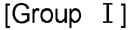

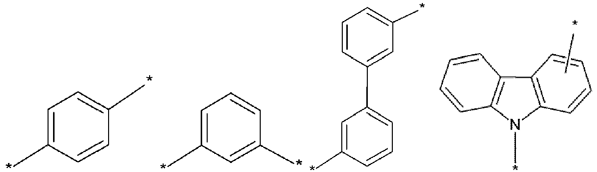

In Group I, * indicates a linking point.

In one embodiment of the present invention, the R¹ and R^{b} are independently a group selected from substituted or unsubstituted groups listed in the following Group II:

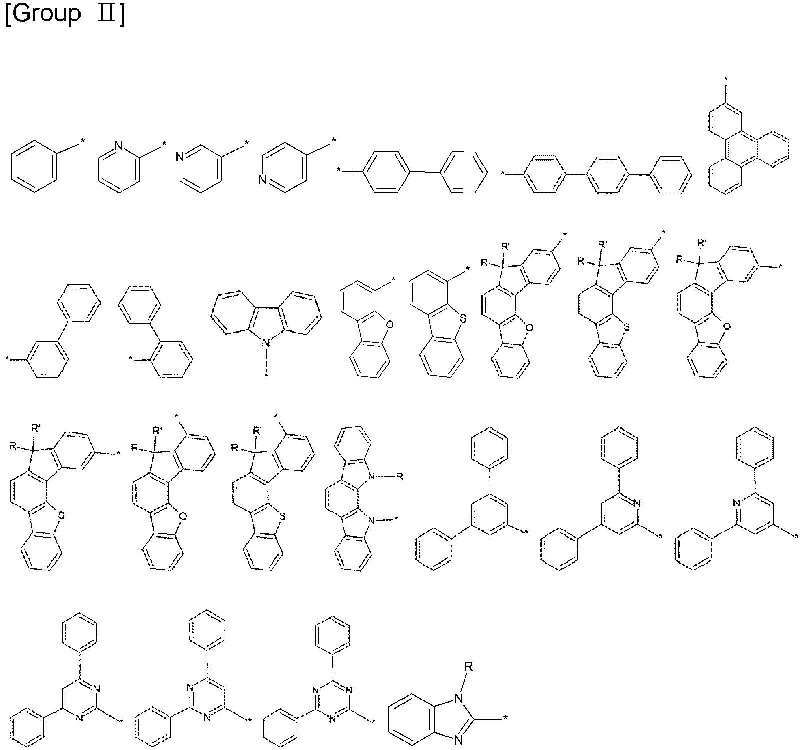

In Group II, R and R' are independently hydrogen, deuterium, a substituted or unsubstituted C1 to C30 alkyl group, a substituted or unsubstituted C6 to C30 aryl group, a substituted or unsubstituted C2 to C30 heteroaryl group, or a combination thereof, and * is a linking point.

In another embodiment of the present invention, the compound is represented by one of the following compounds listed in the following Group III:

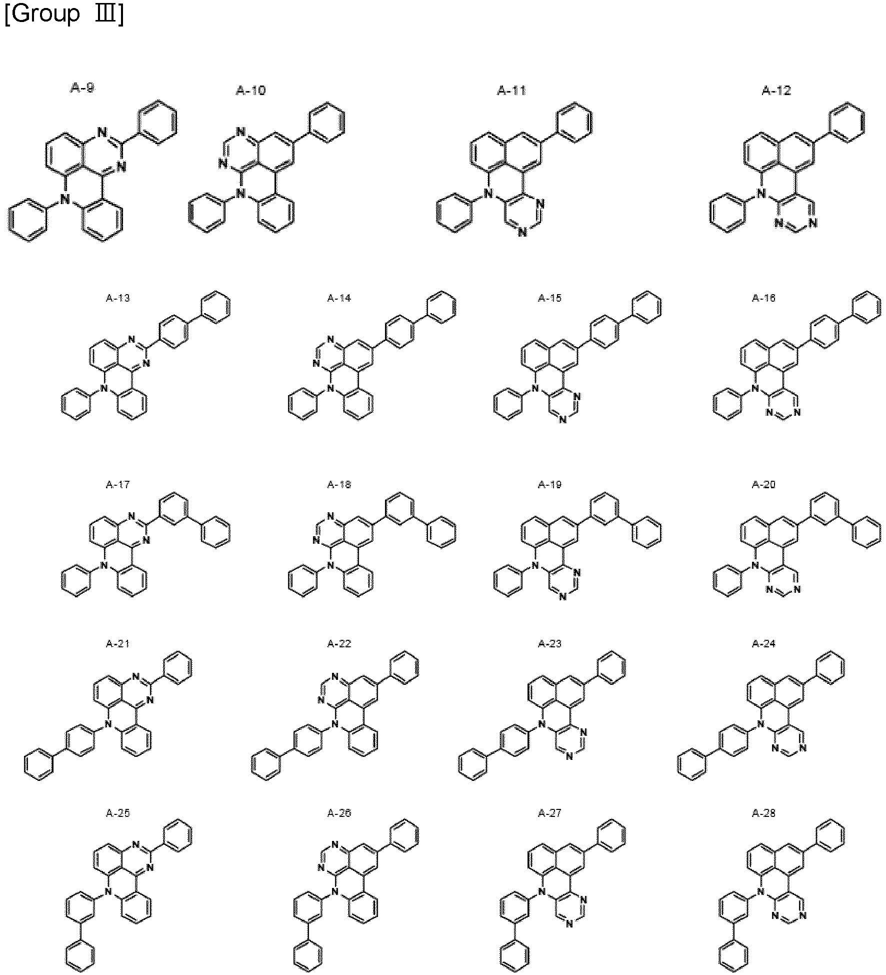

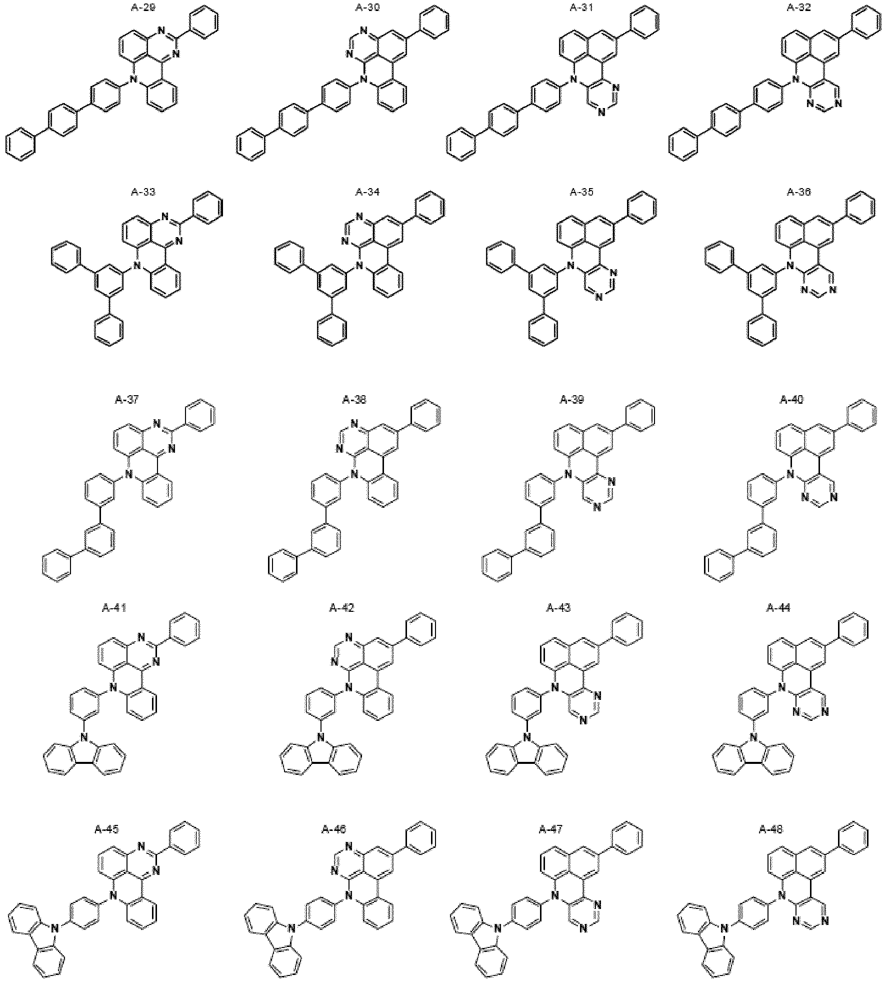

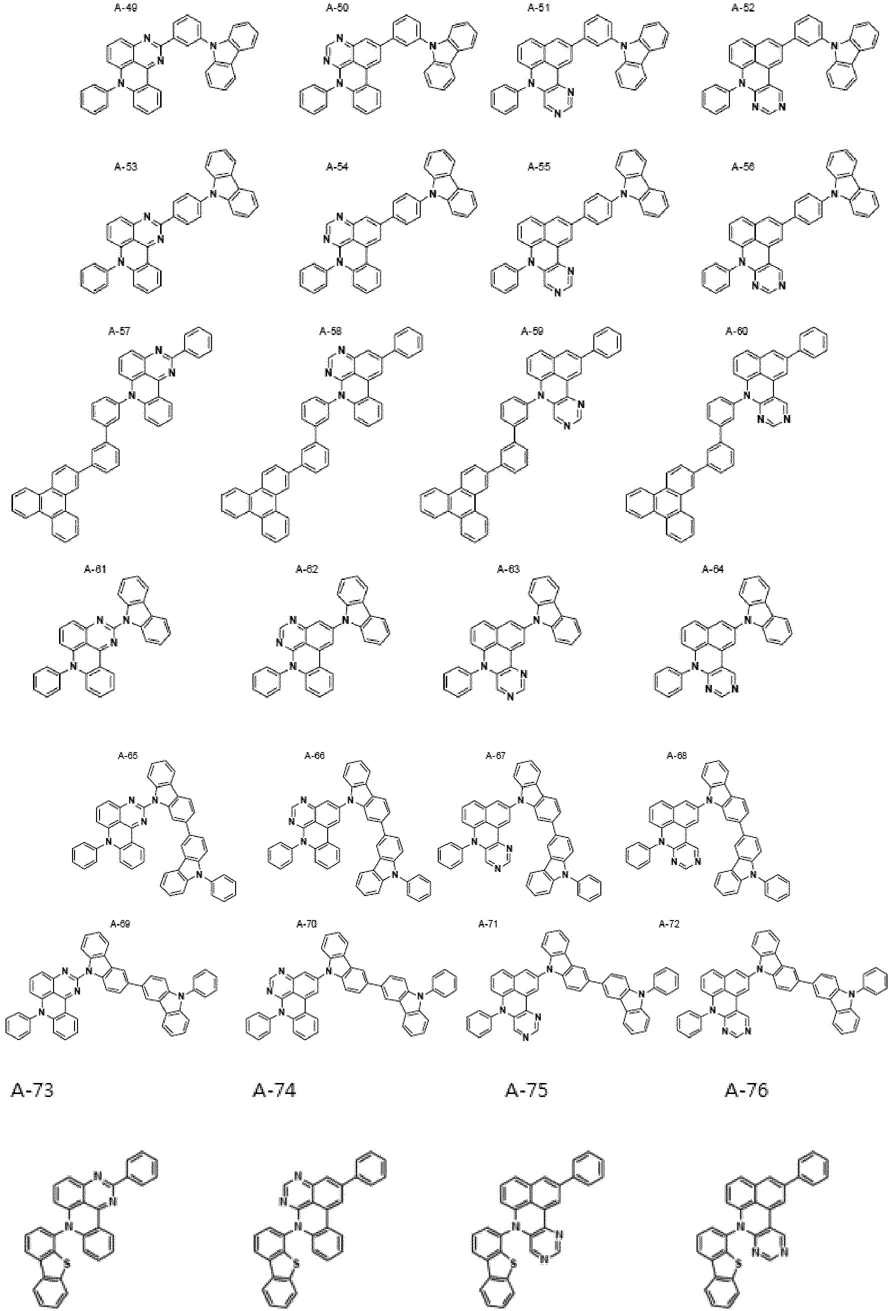

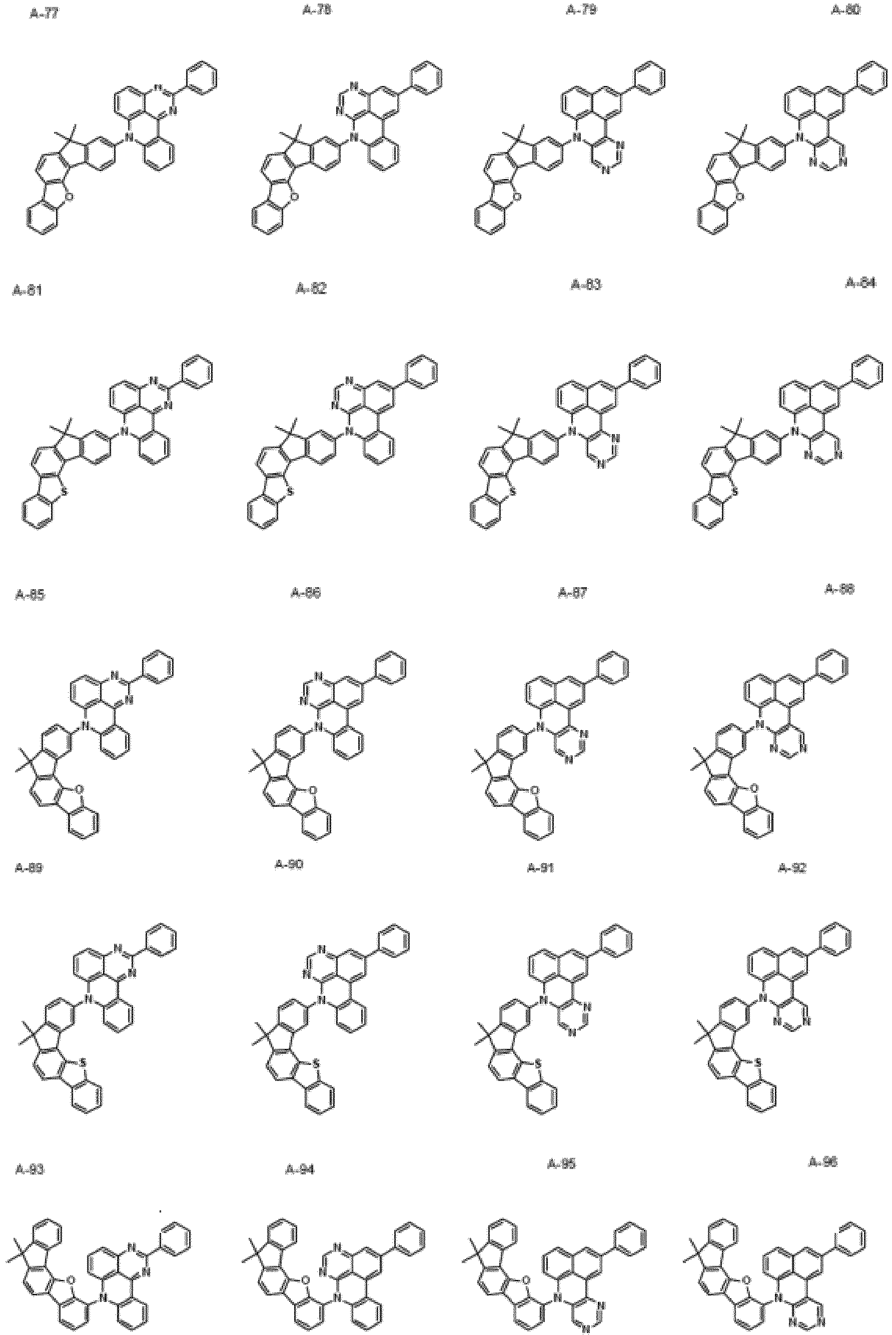

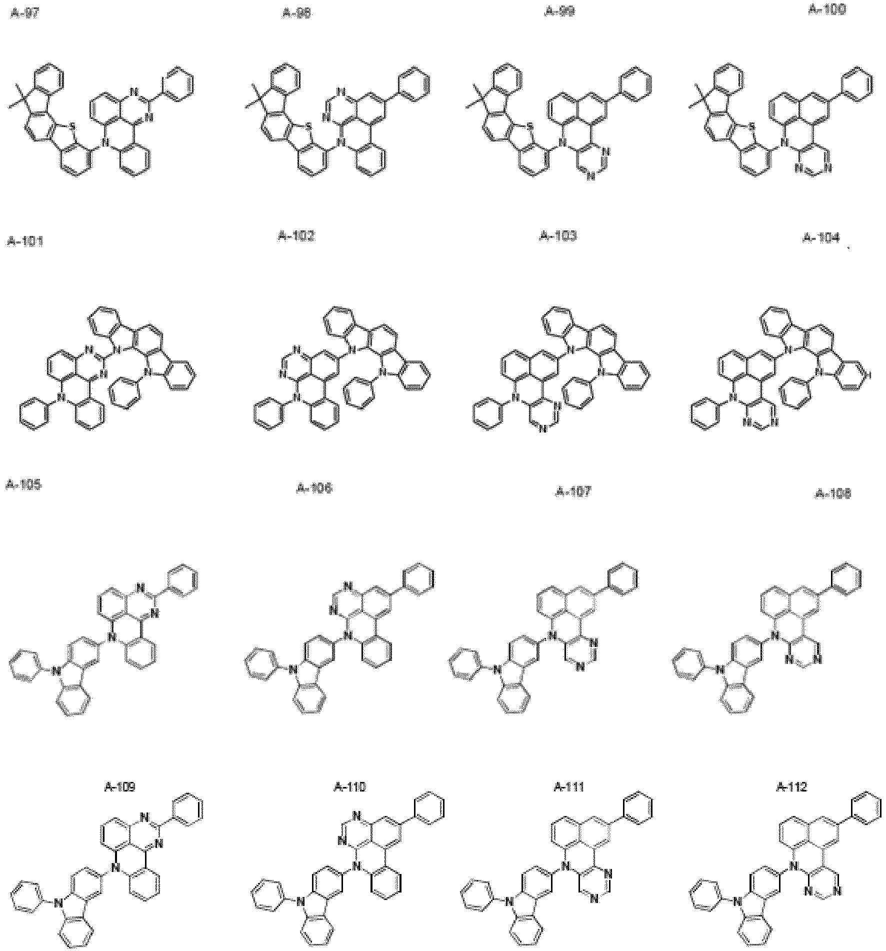

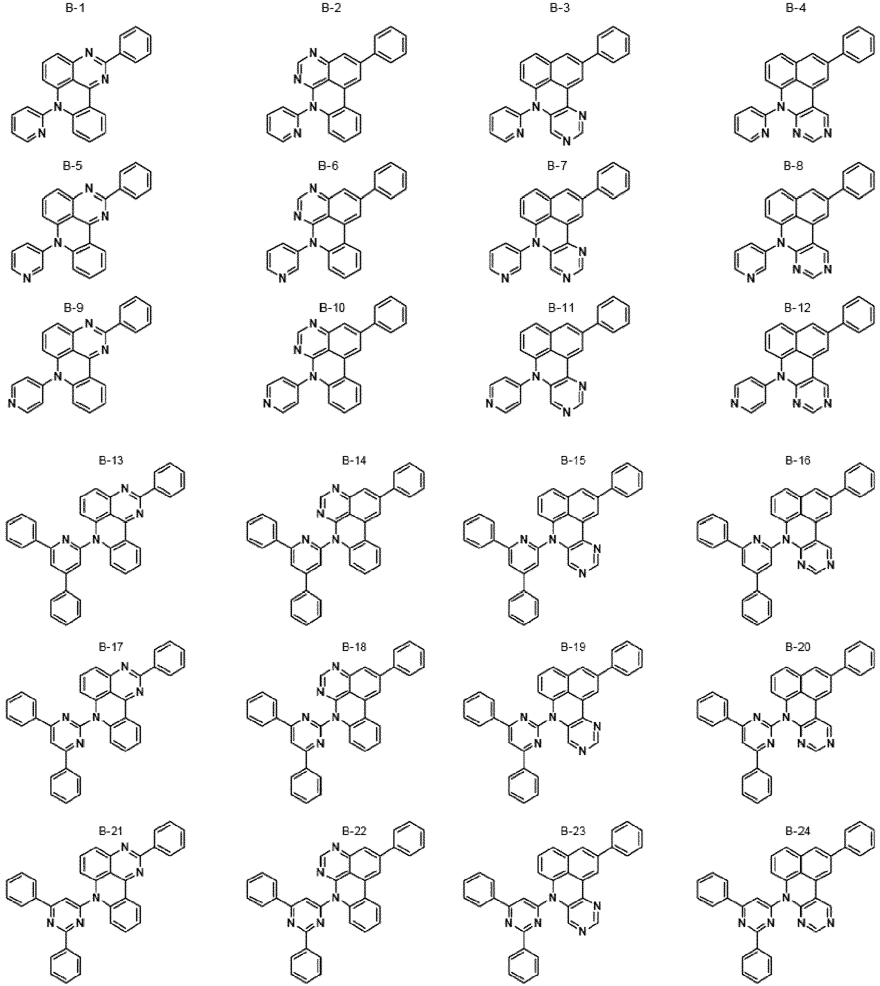

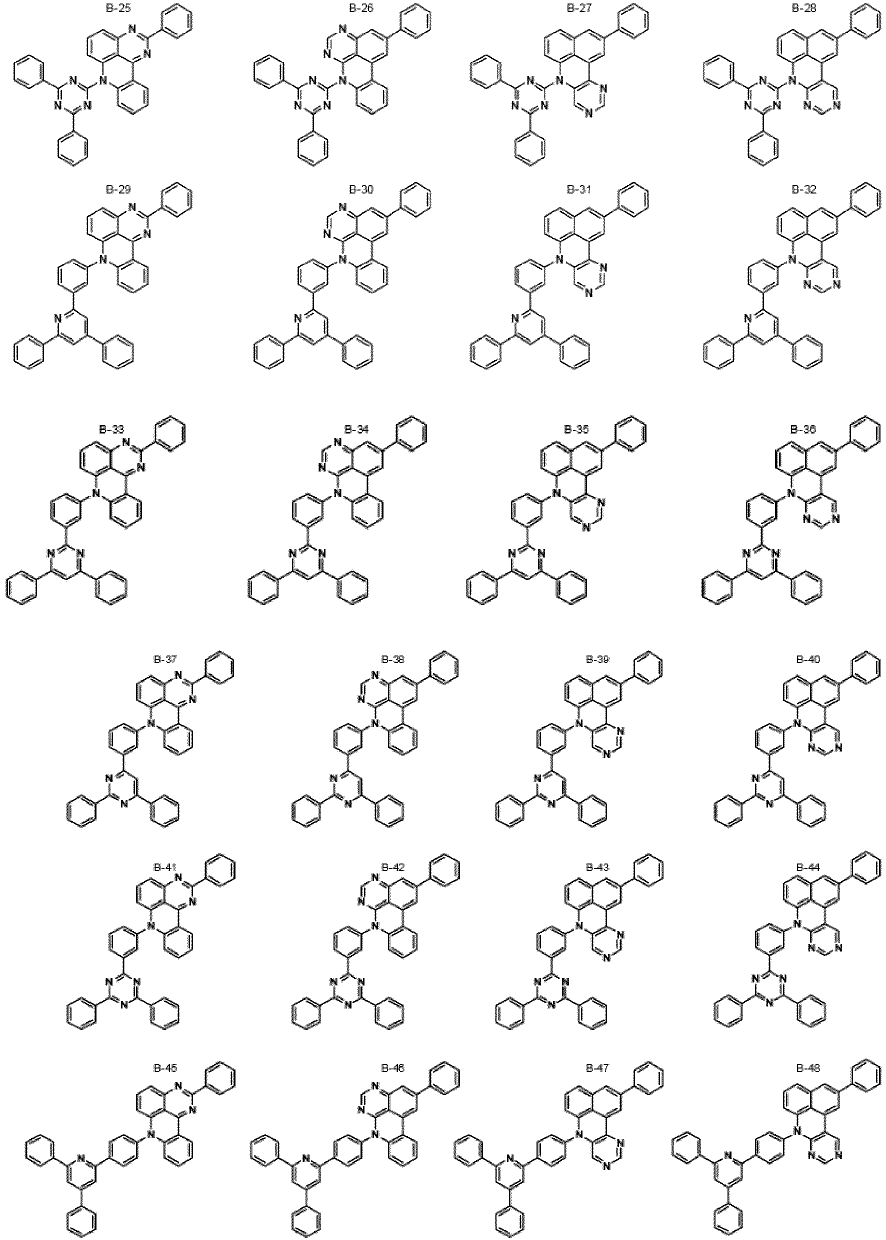

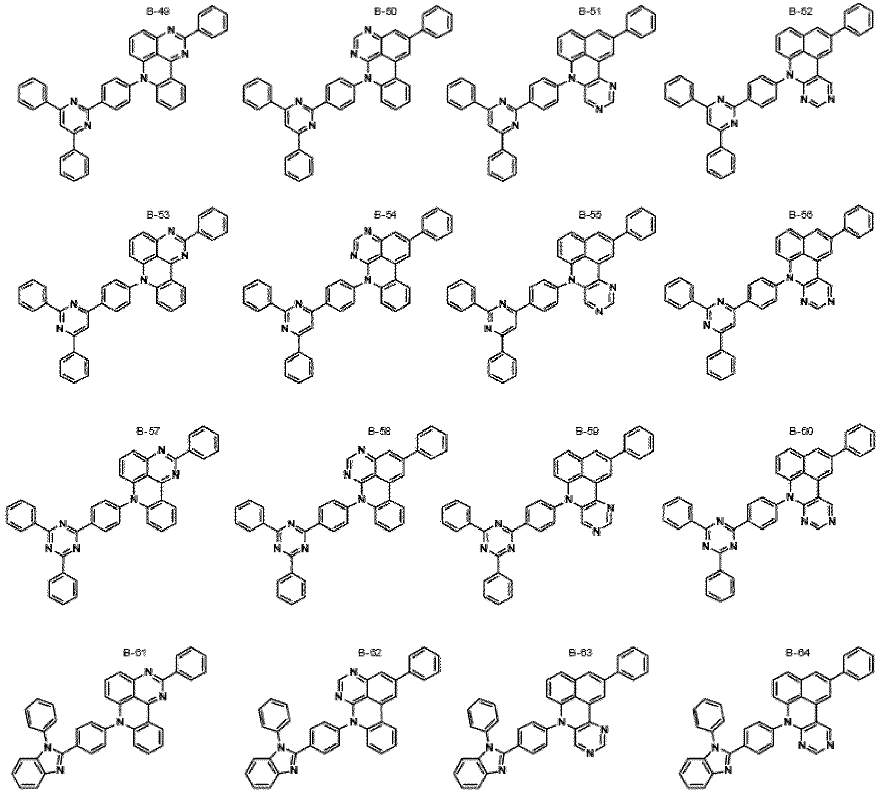

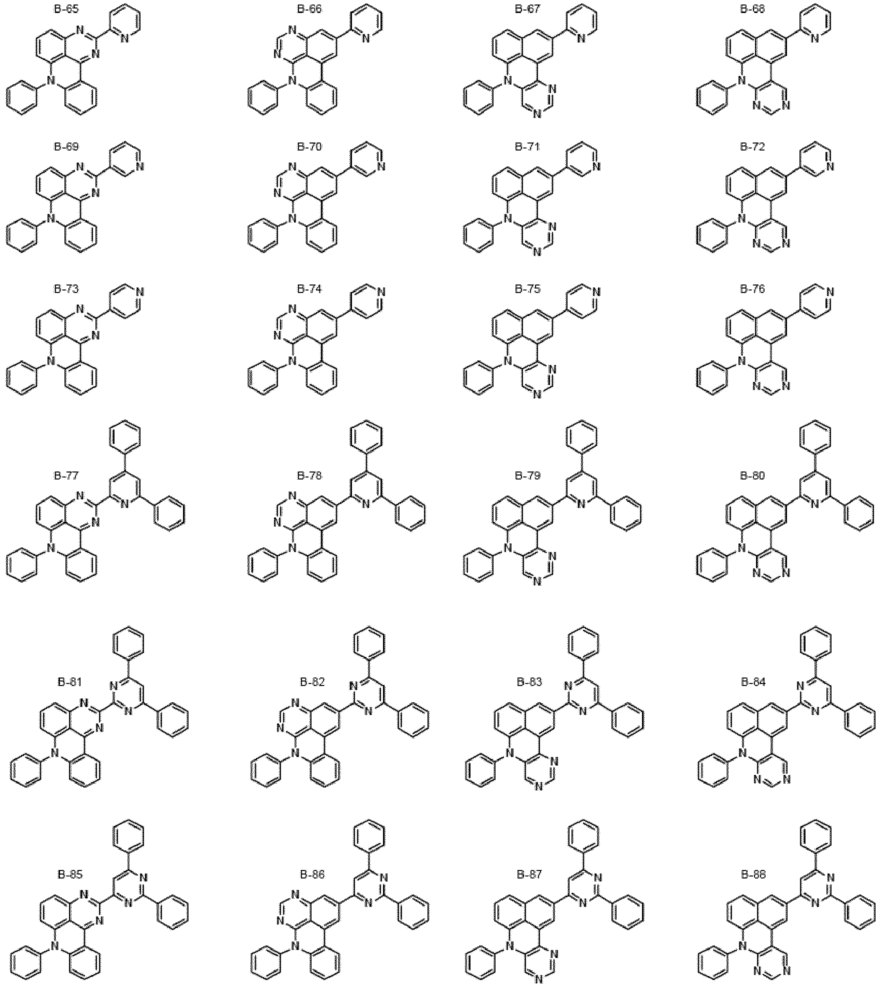

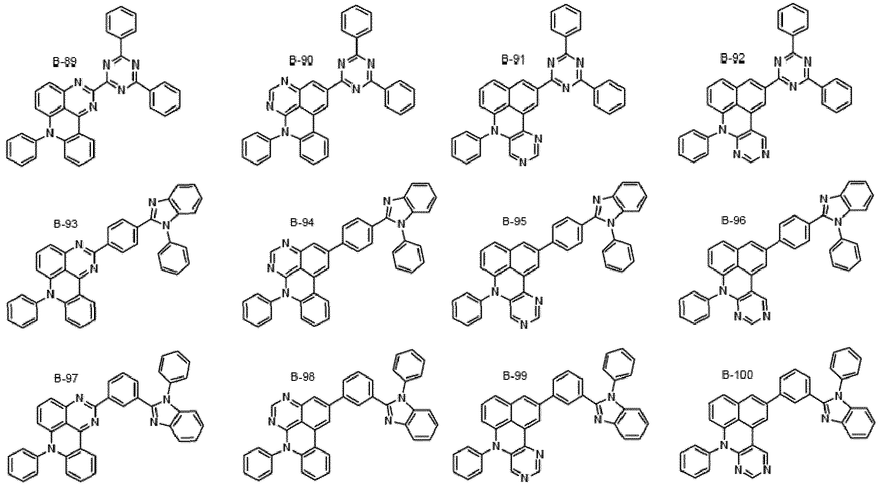

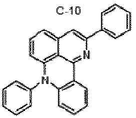

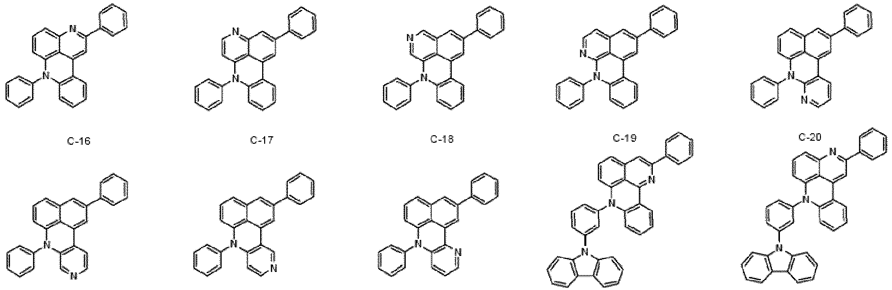

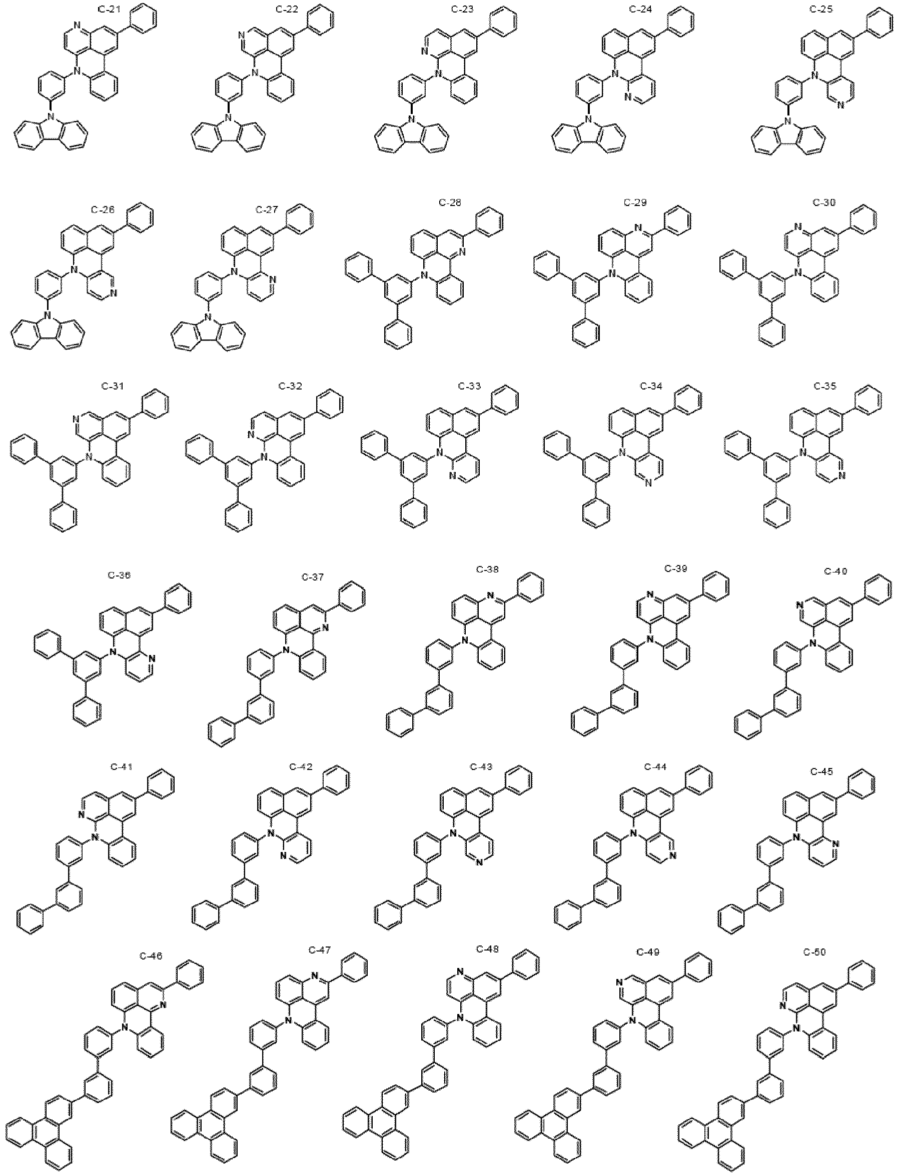

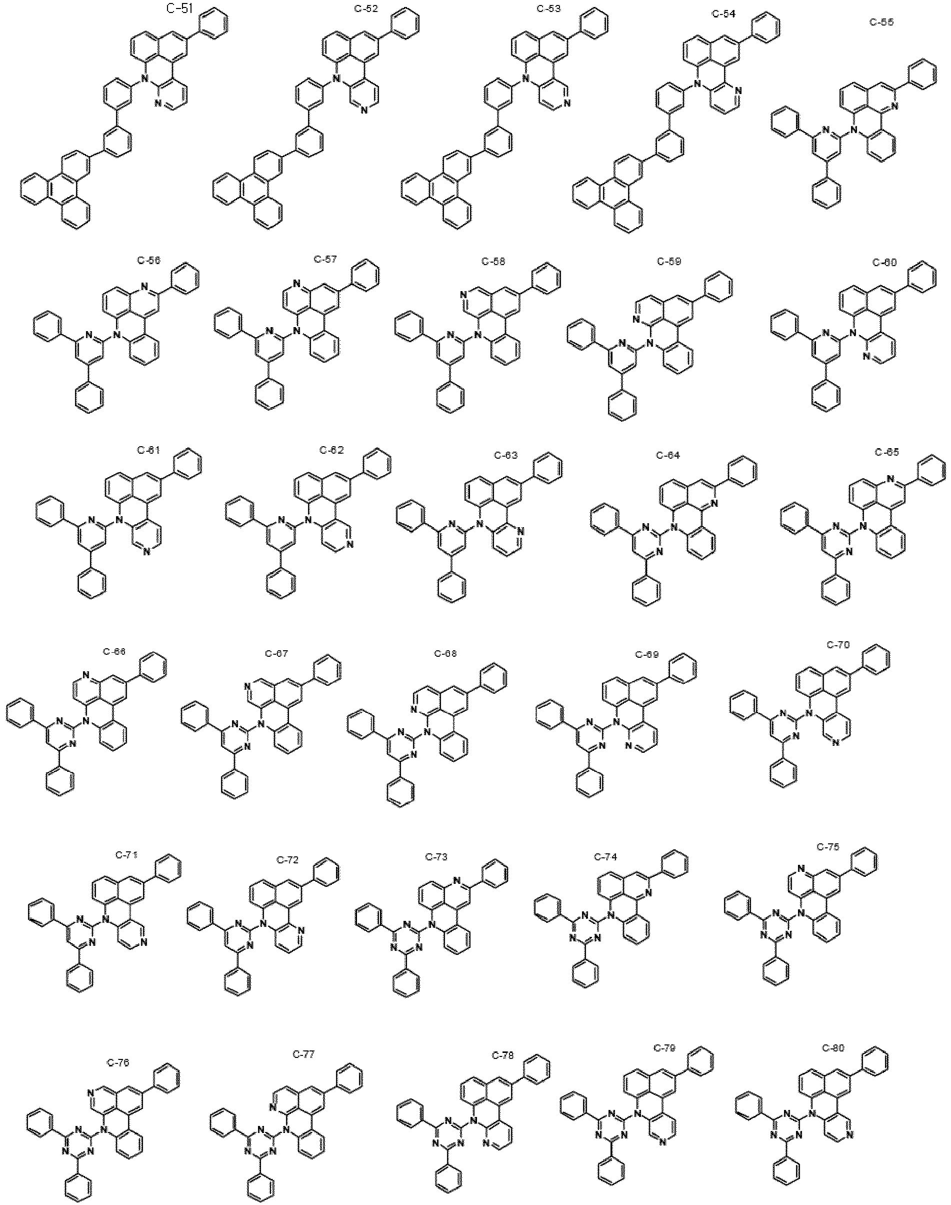

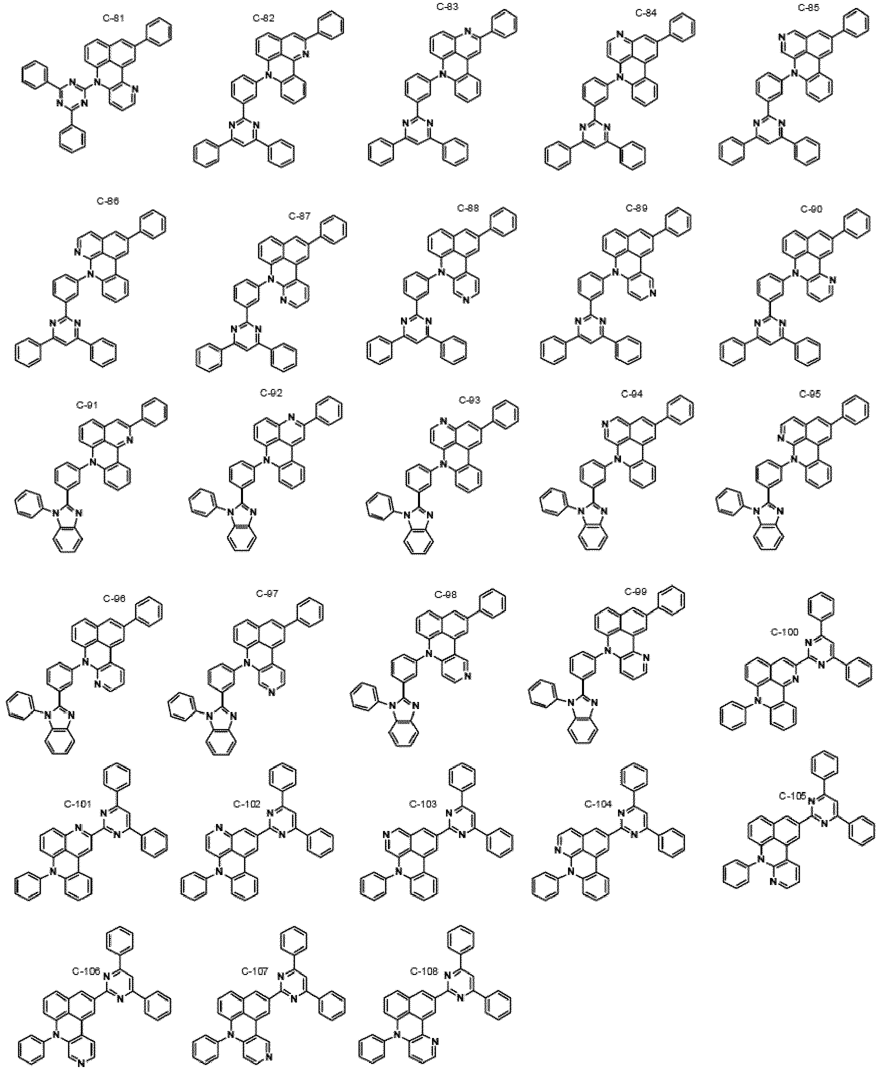

The compound is used for an organic optoelectronic device.

Hereinafter, an organic optoelectronic device to which the compound is applied is described.

In another embodiment of the present invention, the organic optoelectronic device includes an anode and a cathode facing each other, and at least one organic layer interposed between the anode and the cathode, wherein the organic layer includes the compound.

The organic layer may include an emission layer, and the emission layer may include the compound of the present invention.

Specifically, the compound may be included as a host of the emission layer.

In addition, in one embodiment of the present invention, the organic layer includes at least one auxiliary layer selected from a hole injection layer, a hole transport layer, an electron blocking layer, an electron transport layer, an electron injection layer and a hole blocking layer, wherein the auxiliary layer may include the compound.

The organic optoelectronic device may be any device to convert electrical energy into photoenergy and vice versa without particular limitation, and may be, for example an organic photoelectric device, an organic light emitting diode, an organic solar cell, and an organic photo-conductor drum.

Herein, an organic light emitting diode as one example of an organic optoelectronic device is described referring to drawings.

FIGS. 1 and 2 are cross-sectional views of each organic light emitting diode according to one embodiment.

Referring to FIG. 1, an organic optoelectronic device 100 according to one embodiment includes an anode 120 and a cathode 110 facing each other and an organic layer 105 interposed between the anode 120 and cathode 110.

The anode 120 may be made of a conductor having a large work function to help hole injection, and may be for example metal, metal oxide and/or a conductive polymer. The anode 120 may be, for example a metal nickel, platinum, vanadium, chromium, copper, zinc, gold, and the like or an alloy thereof; metal oxide such as zinc oxide, indium oxide, indium tin oxide (ITO), indium zinc oxide (IZO), and the like; a combination of metal and oxide such as ZnO and Al or SnO₂ and Sb; a conductive polymer such as poly(3-methylthiophene), poly(3,4-(ethylene-1,2-dioxy)thiophene) (PEDT), polypyrrole, and polyaniline, but is not limited thereto.

The cathode 110 may be made of a conductor having a small work function to help electron injection, and may be for example metal, metal oxide and/or a conductive polymer. The cathode 110 may be for example a metal or an alloy thereof such as magnesium, calcium, sodium, potassium, titanium, indium, yttrium, lithium, gadolinium, aluminum silver, tin, lead, cesium, barium, and the like; a multi-layer structure material such as LiF/Al, LiO₂/Al, LiF/Ca, LiF/Al and BaF₂/Ca, but is not limited thereto.

The organic layer 105 includes an emission layer 130 including the compound.

The emission layer 130 may include, for example the compound at alone, a mixture of at least two kinds of the compound or a mixture of the compound and another compound. When the mixture of the compound and another compound is used, for example they may be included as a host and a dopant, and the compound may act, for example as a host. The host may be, for example a phosphorescent host or fluorescent host, and for example a phosphorescent host.

When the compound is used as a host, a dopant may be selected from inorganic, organic, organic/inorganic compound, and may be selected from well-known dopants.

Referring to FIG. 2, the organic light emitting diode 200 further include a hole auxiliary layer 140 as well as an emission layer 230. The hole auxiliary layer 140 increases hole injection and/or hole mobility between the anode 120 and the emission layer 230, and blocks electrons. The hole auxiliary layer 140 may be, for example a hole transport layer (HTL), a hole injection layer (HIL) and/or an electron blocking layer, and may include at least one layer. The compound may be included in the emission layer 230 and/or the hole auxiliary layer 140.

Even though not shown in FIG. 1 or FIG. 2, the organic layer 105 may further include an electron injection layer (EIL), an electron transport layer (ETL), an electron transport layer (ETL), an auxiliary hole transport layer (HTL), an auxiliary hole transport layer (HTL), a hole injection layer (HIL) or a combination thereof. The compound of the present invention may be included in these organic layers. The organic light emitting diodes 100 and 200 may be manufactured by forming an anode or a cathode on a substrate, forming an organic layer in accordance with a dry coating method such as evaporation, sputtering, plasma plating, and ion plating or a wet coating method such as spin coating, dipping, flow coating, and forming a cathode or an anode thereon.

The organic light emitting diode may be applied to an organic light emitting diode (OLED) display.

### [Mode for Invention]

Hereinafter, the embodiments are illustrated in more detail with reference to examples. These examples, however, are not in any sense to be interpreted as limiting the scope of the invention.

### (Preparation of Compound)

### Example 1: Synthesis of Compound B-17

A compound B-17 as a specific example of a compound according to the present invention was synthesized through the following four steps.

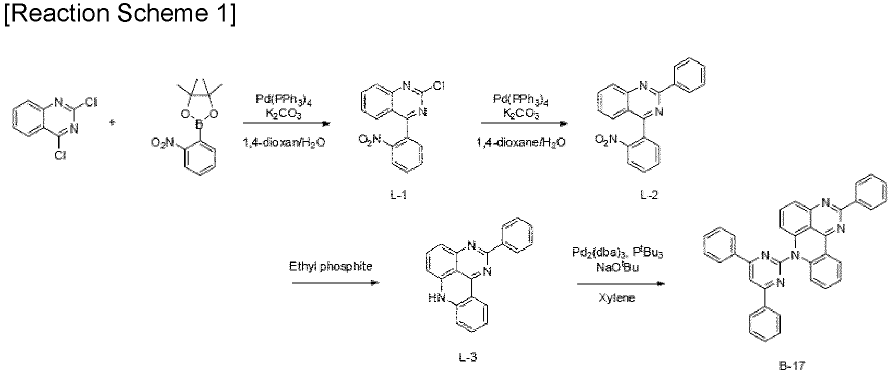

### First Step: Synthesis of Intermediate Product (L-1)

39.0 g (195.9 mmol) of 2,4-dichloroquinazoline, 53.7 g (215.5 mmol) of 4,4,5,5-tetramethyl-2-(2-nitrophenyl)1,3,2-dioxaborolane, 67.7 g (489.9 mmol) of potassium carbonate, and 11.3 g (9.8 mmol) of tetrakis (triphenylphosphine)palladium were added to 650 mL of 1,4-dioxane and 300 mL of water in a 2000 mL flask, and then, the mixture was heated at 60°C for 12 hours under a nitrogen stream. The obtained mixture was added to 2000 mL of methanol, a solid crystallized therein was filtered and then, dissolved in monochlorobenzene and filtered with silica gel/Celite again, and methanol was used to perform recrystallization after removing an organic solvent in an appropriate amount therefrom, obtaining an intermediate L-1 (29.1 g, a yield of 51 %).
calcd. C14H8ClN3O2: C, 58.86; H, 2.82; Cl, 12.41; N, 14.71; O, 11.20; found : C, 58.30; H, 2.92; Cl, 12.13; N, 14.21; O, 10.82

### Second Step; Synthesis of Intermediate Product (L-2)

29.0 g (101.5 mmol) of the intermediateL-1, 14.2 g (116.73 mmol) of phenyl boronic acid, 35.1 g (253.7 mmol) of potassium carbonate, and 5.9 g (5.1 mmol) of tetrakis (triphenylphosphine)palladium were added to 350 mL of 1,4-dioxane and 150 mL of water in a 1000 mL flask, and then, the mixture was heated at 60 °C for 12 hours under a nitrogen stream. The obtained mixture was added to 1000 mL of methanol, a solid crystallized therein was filtered, dissolved in monochlorobenzene and filtered again with silica gel/Celite, and then, methanol was used to perform recrystallization after removing an organic solvent in an appropriate amount therefrom, obtaining an intermediate L-2 (23.6 g, a yield of 71 %).
calcd. C20H13N3O2: C, 73.38; H, 4.00; N, 12.84; O, 9.78; found: C, 72.99; H, 3.96; N, 12.81; O, 9.77

### Third Step; Synthesis of Intermediate Product (L-3)

26.0 g (79.43 mmol) of the intermediate L-2 was added to 200 mL of triethylphosphite in a 500 mL flask, and the mixture was heated at 170 °C for 12 hours under a nitrogen stream. The obtained mixture was fractionally distillated to remove a solvent and then, treated through column chromatography, obtaining an intermediate L-3 (14.1 g, a yield of 60.0 %).
calcd. C20H13N3: C, 81.34; H, 4.44; N, 14.23; found: C, 81.12; H, 4.39; N, 14.16

### Fourth Step: Synthesis of Compound B-17

8.4 g (28.5 mmol) of the intermediate L-3, 8.0 g (29.97 mmol) of 1-chloro-3,5-diphenyl pyrimidine, 5.5 g (57.1 mmol) of sodium t-butoxide, 0.6 g (2.9 mmol) of tris(dibenzylideneacetone)dipalladium(0), 2.3 mL of tri t-butylphosphine (50% in toluene) were added to 200 mL of xylene in a 500 mL round flask, and the mixture was heated and refluxed for 15 hours under a nitrogen stream. Then, 500 mL of methanol was added to the obtained mixture, a solid crystallized therein was filtered, dissolved in dichlorobenzene and filtered with silica gel/Celite, and methanol was used to perform recrystallization to remove an organic solvent in an appropriate amount, obtaining a compound B-17 (10.0 g, a yield of 67 %).
calcd. C36H23N5: C, 82.26; H, 4.41; N, 13.32; found: C, 82.25; H, 4.34; N, 13.02

### Example 2: Synthesis of Compound B-33

A compound B-33 as a specific example of a compound according to the present invention was synthesized through the following one step.

### First Step; Synthesis of Compound B-33

8.3 g (28.3 mmol) of the intermediate L-3, 11.5 g (29.7 mmol) of 2-(3-bromophenyl)-4,6-diphenylpyrimidine, 5.4 g (56.5 mmol) of sodium t-butoxide, 0.6 g (2.8 mmol) of tris(dibenzylideneacetone)dipalladium(0), and 2.3 mL of tri t-butylphosphine (50% in toluene) were added to 200 mL of xylene in a 500 mL round flask, and the mixture was heated and refluxed for 15 hours under a nitrogen stream. The obtained mixture was added to 500 mL of methanol, a solid crystallized therein was filtered, dissolved in dichlorobenzene, and filtered with silica gel/Celite, and methanol was used to perform recrystallization after removing an organic solvent in an appropriate amount therefrom, obtaining a compound B-33 (11.2 g, a yield of 66 %).
calcd. C42H27N5: C, 83.84; H, 4.52; N, 11.64; found: C, 83.57; H, 4.44; N, 11.51

### Example 3: Synthesis of Compound A-105

A compound A-105 as a specific example of a compound according to the present invention was synthesized through the following one step.

### First Step; Synthesis of Compound A-105

8.3 g (28.0 mmol) of the intermediate L-3, 10.8 g (29.4 mmol) of 9-phenyl-3-bromo-carbazole, 5.4 g (56.0 mmol) of sodium t-butoxide, 1.6 g (2.8 mmol) of tris(dibenzylideneacetone)dipalladium(0), and 2.3 mL of tri t-butylphosphine (50 % in toluene) were added to 190 mL of xylene in a 500 mL round flask, and the mixture was heated and refluxed for 15 hours under a nitrogen stream. The obtained mixture was added to 500 mL of methanol, a solid crystallized therein was filtered, dissolved in dichlorobenzene and filtered with silica gel/Celite, and then, methanol was used to perform recrystallization after removing an organic solvent in an appropriate amount therefrom, obtaining a compound A-105 (9.5 g, a yield of 63 %).
calcd. C38H24N4: C, 85.05; H, 4.51; N, 10.44; found: C, 85.01; H, 4.47; N, 10.32

### Comparative Example 1: Synthesis of CBP

A compound represented by the following Chemical Formula a was synthesized in the same method as disclosed in International Publication WO 2013032035.

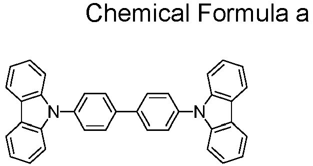

### (Simulation Characteristics Comparison of Prepared Compounds)

The energy level of each material was calculated in a Gaussian 09 method by using a supercomputer GAIA (IBM power 6), and the result is provided in the following Table 1.

**[Table 1]**

| Compounds | Dipole Moment | HOMO (eV) | LUMO (eV) | T1 (eV) | S1 (eV) |
|---|---|---|---|---|---|
| Comparative Example 1 | 0.0829 | -5.319 | -1.231 | 2.971 | 3.560 |
| Example 1 | 8.3085 | -5.075 | -2.198 | 2.302 | 3.145 |
| Example 2 | 8.2130 | -5.082 | -1.904 | 2.324 | 3.167 |
| Example 3 | 8.1971 | -5.052 | -1.476 | 2.321 | 3.203 |

As shown in the Table 1, since desired HOMO/LUMO energy levels in simulation, HOMO of -5.0 to -5.5 and LUMO of -1.4 to -2.2, well show electron transport characteristics, Comparative Example 1 satisfied the HOMO level but did not satisfy the LUMO level and thus, showed an unbalance between holes and electrons compared with Example 1, 2, and 3.

The compound of the present invention has an appropriate energy level compared with Comparative Example 1 and thus, is expected to show an excellent efficiency and life-span.

### (Manufacture of Organic Light Emitting Diode)

### Example 4

An organic light emitting diode was manufactured by using the compound B-17 of Example 1 as a host, and (piq)₂Ir(acac) as a dopant.

As for an anode, 1000 A-thick ITO was used, and as for a cathode, 1000 A-thick aluminum (Al) was used. Specifically, illustrating a method of manufacturing the organic light emitting diode, the anode is manufactured by cutting an ITO glass substrate having 15 Ω/cm² of sheet resistance into a size of 50 mm × 50 mm × 0.7 mm, ultrasonic wave-cleaning them in acetone, isopropylalcohol, and pure water for 15 minutes respectively, and UV ozone cleaning them for 30 minutes.

On the substrate, an 800 A-thick hole transport layer (HTL) was formed by depositing N4,N4'-di(naphthalen-1-yl)-N4,N4'-diphenylbiphenyl-4,4'-diamine (NPB) under a vacuum degree 650×10⁻⁷ Pa at a deposition rate of 0.1 to 0.3 nm/s. Subsequently, A 300 A-thick emission layer was formed by using the compound B-17 of Example 1 under the same vacuum deposition condition, and herein, a phosphorescent dopant of (piq)₂Ir(acac) was simultaneously deposited. Herein, the phosphorescent dopant was deposited to be 3 wt% based on 100 wt% of the total weight of the emission layer by adjusting the deposition rate.

On the emission layer, a 50 A-thick hole blocking layer was formed by depositing bis(2-methyl-8-quinolinolate)-4-(phenylphenolato)aluminium (BAlq) under the same vacuum deposition condition. Subsequently, a 200 A-thick electron transport layer (ETL) was formed by depositing Alq3 under the same vacuum deposition condition. On the electron transport layer (ETL), a cathode is formed by sequentially depositing LiF and Al, manufacturing an organic optoelectronic device.

The organic optoelectronic device has a structure of ITO/ NPB (80 nm)/ EML (B-17 (97wt%) + (piq)₂Ir(acac) (3 wt%), 30nm)/ Balq (5nm)/ Alq3 20nm/ LiF (1nm) / Al (100nm).

### Example 5

An organic light emitting diode was manufactured according to the same method as Example 4 except for using the compound B-33 of Example 2 instead of the compound B-17 of Example 4.

### Example 6

An organic light emitting diode was manufactured according to the same method as Example 4 except for using the compound A-105 of Example 3 instead of the compound B-17 of Example 4.

### Comparative Example 2

An organic light emitting diode was manufactured according to the same method as Example 4 except for using CBP of the following structure instead of the compound B-17 of Example 4.

The structures of NPB, BAlq, CBP and (piq)₂Ir(acac) used to manufacture the organic light emitting diodes are as follows.

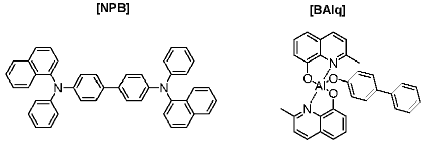

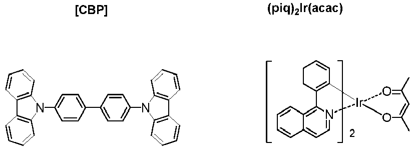

### (Performance Measurement of Organic Light Emitting Diode)

Current density and luminance changes depending on a voltage and luminous efficiency of each organic light emitting diode according to Examples 4, 5, and 6 and Comparative Example 2 were measured.

The measurements were specifically performed in the following method, and the results were provided in the following Table 2.

### (1) Measurement of Current Density Change Depending on Voltage Change

Current values flowing in the unit device of the manufactured organic light emitting diodes were measured for, while increasing the voltage from 0V to 10V using a current-voltage meter (Keithley 2400), and the measured current values were divided by an area to provide the results.

### (2) Measurement of Luminance Change Depending on Voltage Change

Luminance of the manufactured organic light emitting diodes was measured for luminance, while increasing the voltage from 0 V to 10 V using a luminance meter (Minolta Cs-1000A).

### (3) Measurement of Luminous Efficiency

Current efficiency (cd/A) at the same current density (10 mA/cm²) were calculated by using the luminance, current density, and voltages (V) from the items (1) and (2).

### (4) Measurement of Life-span

Life-span was obtained by measuring time taken until current efficiency (cd/A) decreased down to 90 % while luminance (cd/m²) was maintained at 5000 cd/m².

**[Table 2]**

| Nos. | Emission layer | Driving voltage (V) | Color (EL color) | Efficie ncy (cd/A) | 90% life-span (h) at 5000 cd/m² |
|---|---|---|---|---|---|
| Comparative Example 2 | CBP | 6.5 | red | 5.8 | 20 |
| Example 4 | B-17 | 5.0 | red | 13.7 | 101 |
| Example 5 | B-33 | 5.1 | red | 14.3 | 115 |
| Example 6 | A-105 | 5.4 | red | 13.1 | 84 |

Referring to Table 2, the green organic light emitting diodes according to Examples 4 to 6 showed remarkably improved driving voltage, luminous efficiency and/or power efficiency compared with Comparative Example 2.

## Claims

1. A compound represented by
one of the following Chemical Formulae 15 to 27, wherein the compound is used for an organic optoelectronic device: 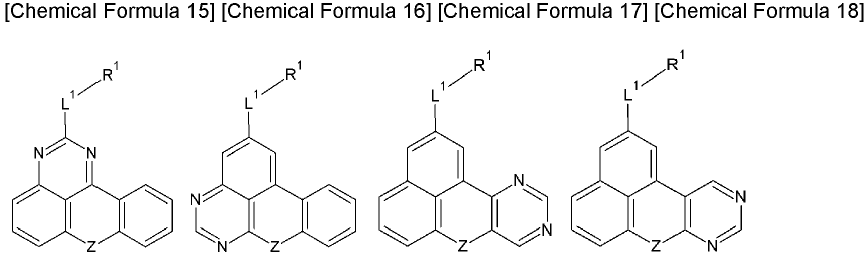 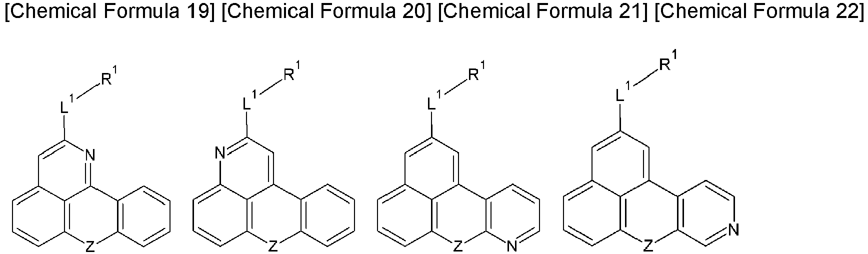 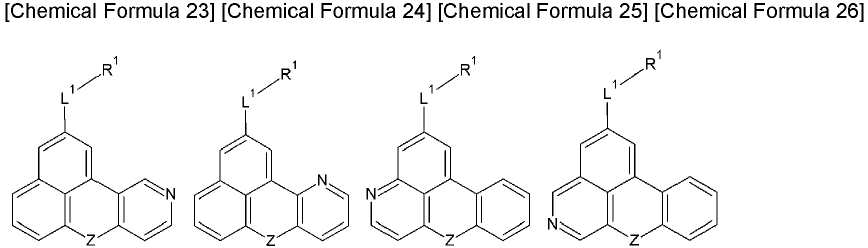 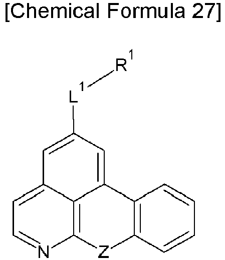
wherein, in the Chemical Formulae 15 to 27,
Z is N-L⁴-R^{b},
L¹ and L⁴ are independently, a single bond, a substituted or unsubstituted C6 to C30 arylene group, a substituted or unsubstituted C2 to C30 heterocyclic group, or a combination thereof, and
wherein the R¹ and R^{b} are independently a group selected from substituted or unsubstituted groups listed in the following Group II: 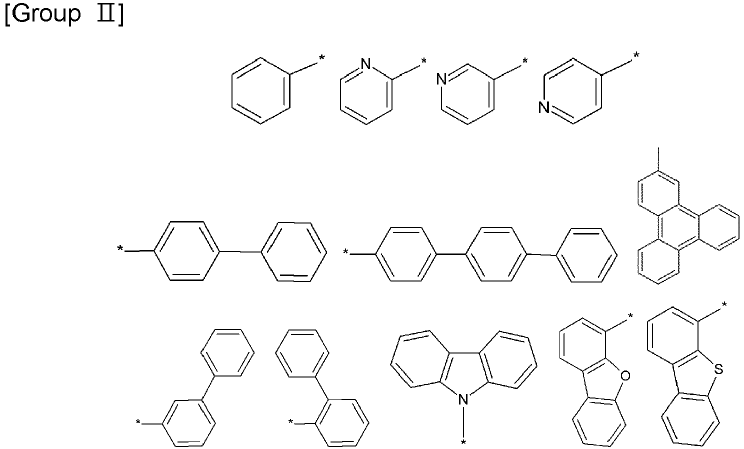 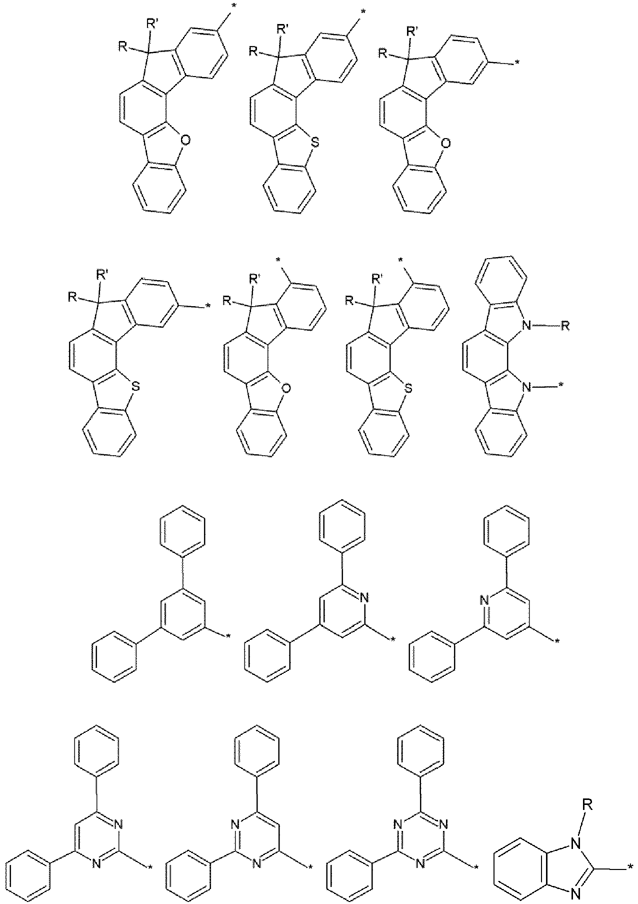
wherein, in Group II,
R and R' are independently hydrogen, deuterium, a substituted or unsubstituted C1 to C30 alkyl group, a substituted or unsubstituted C6 to C30 aryl group, a substituted or unsubstituted C2 to C30 heteroaryl group, or a combination thereof, and
* is a linking point..

2. The compound of claim 1, wherein the L¹ and L⁴ are independently a single bond or a group selected from substituted or unsubstituted groups listed in the following Group I : 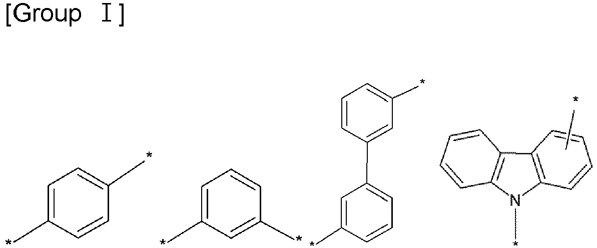
wherein, in Group I,
* indicates a linking point.

3. A compound represented by one of the following compounds listed in the following Group III:

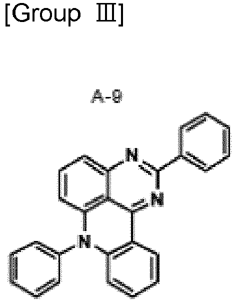

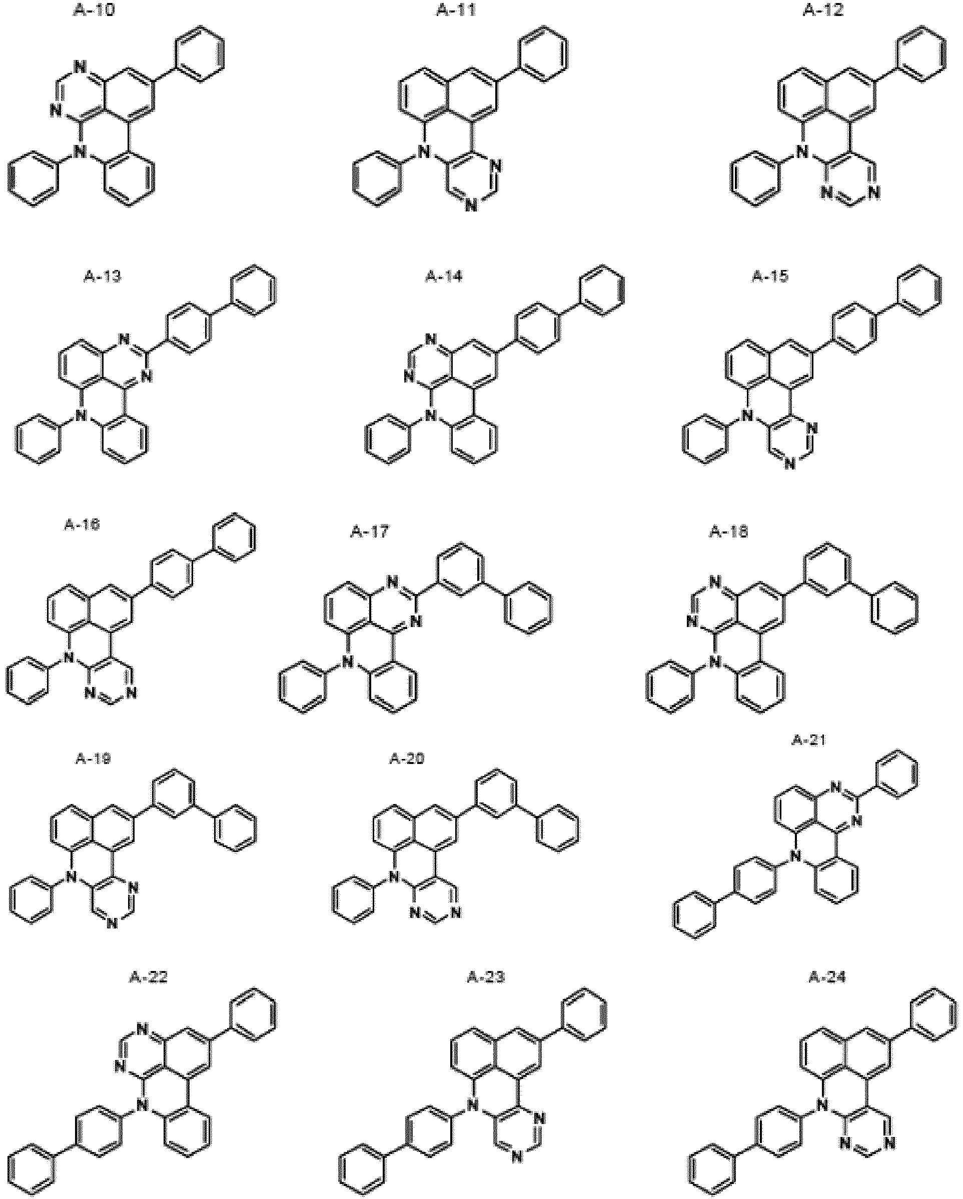

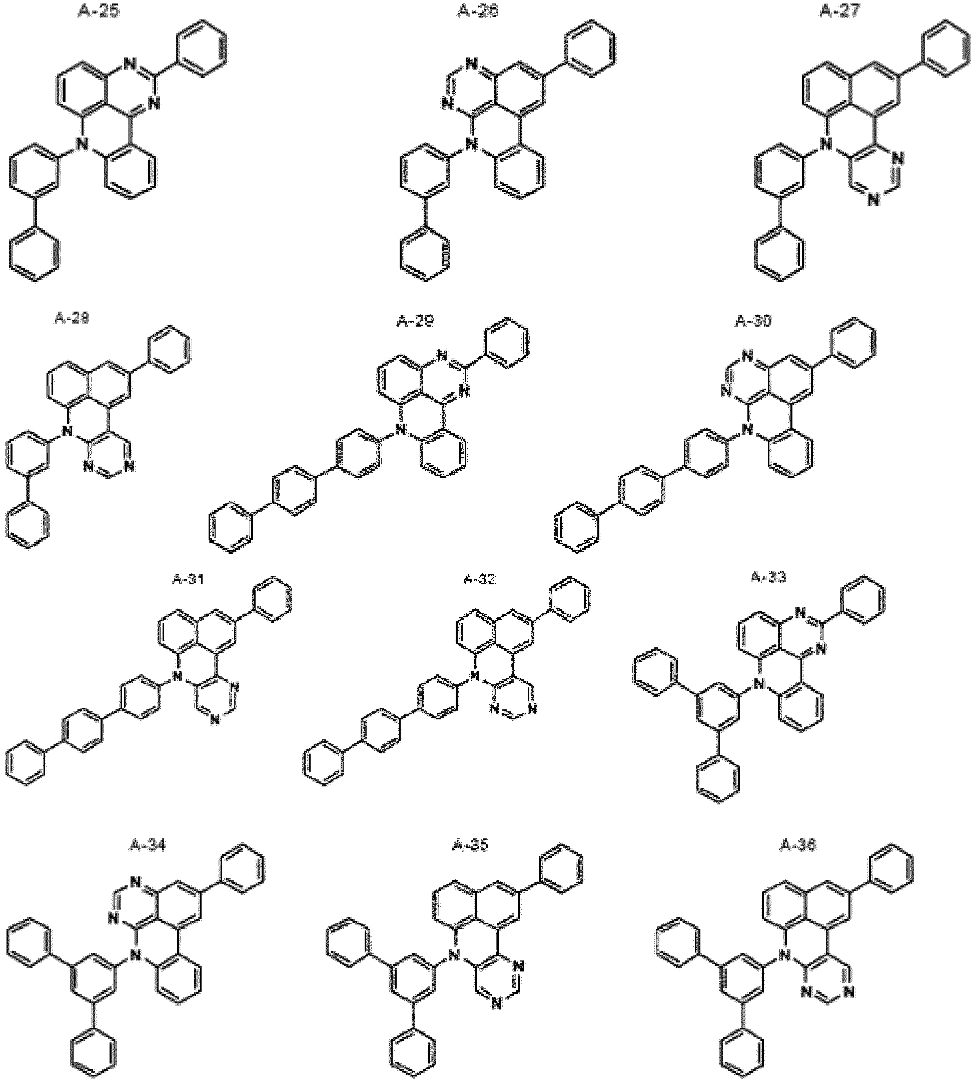

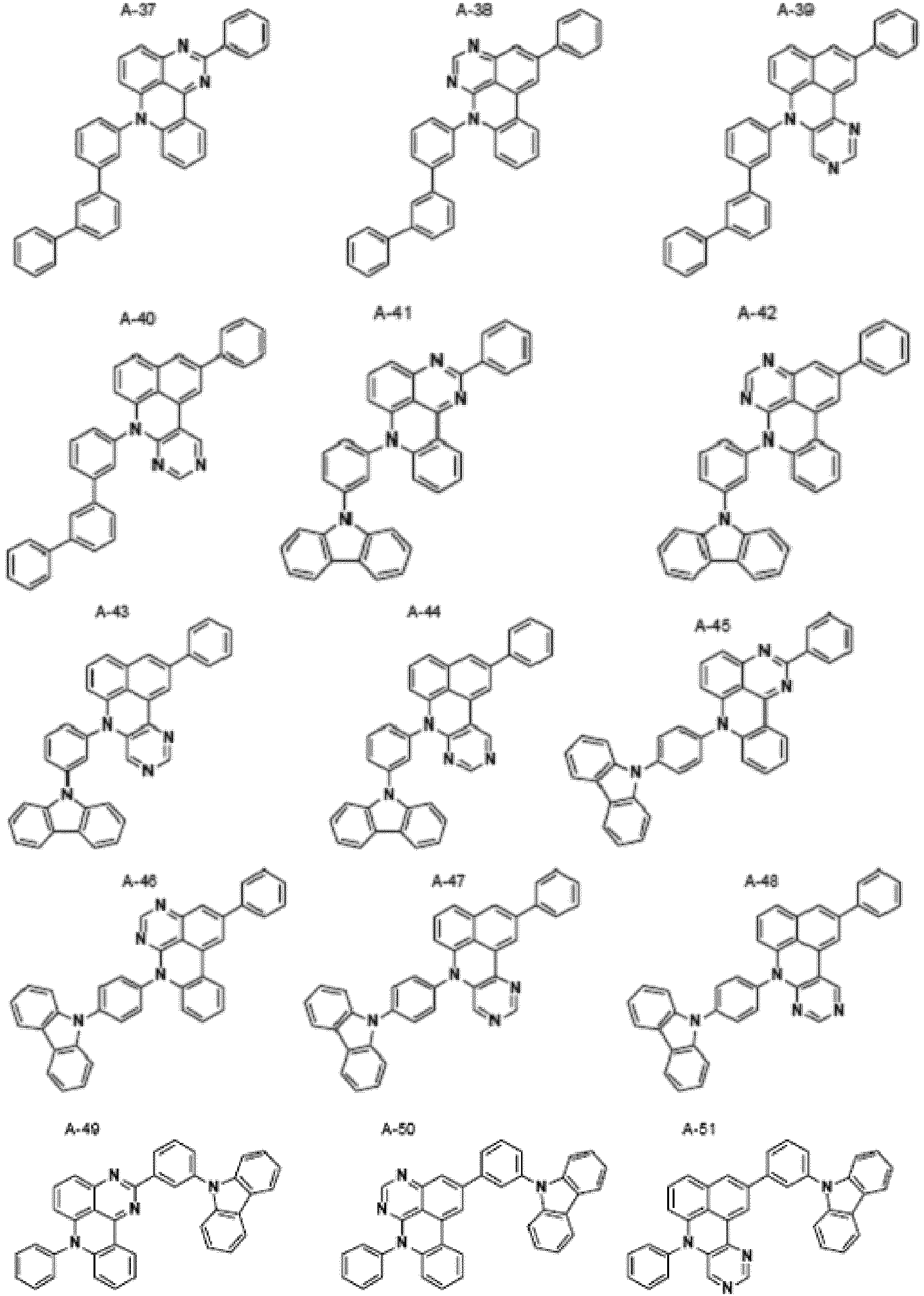

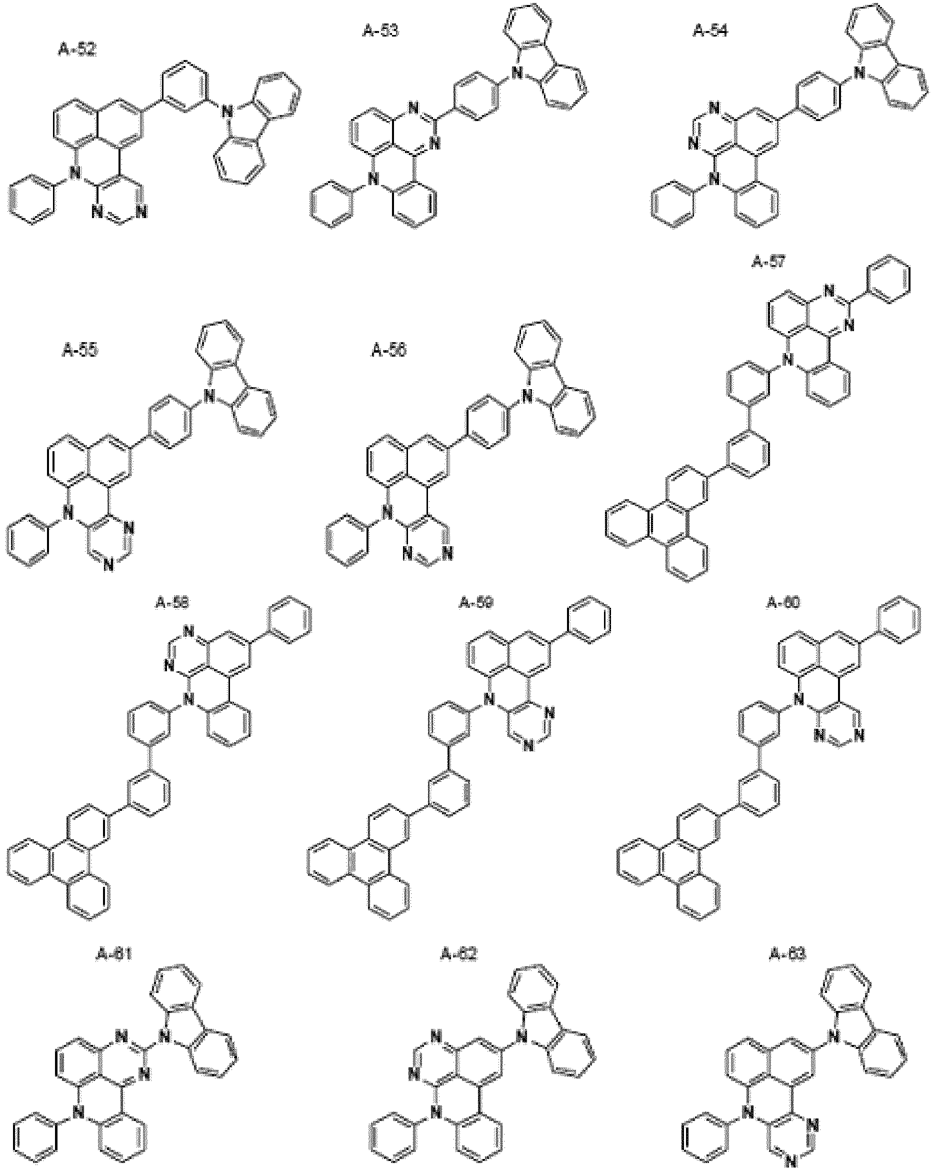

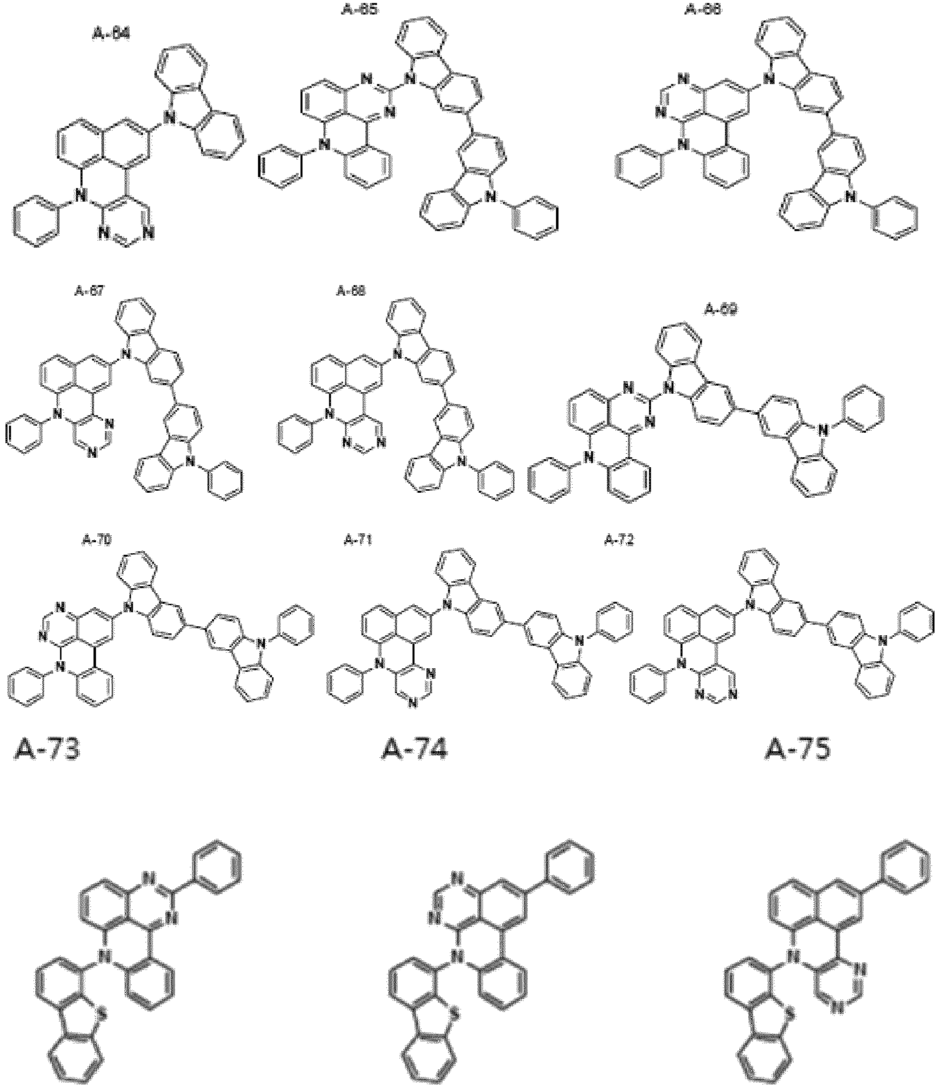

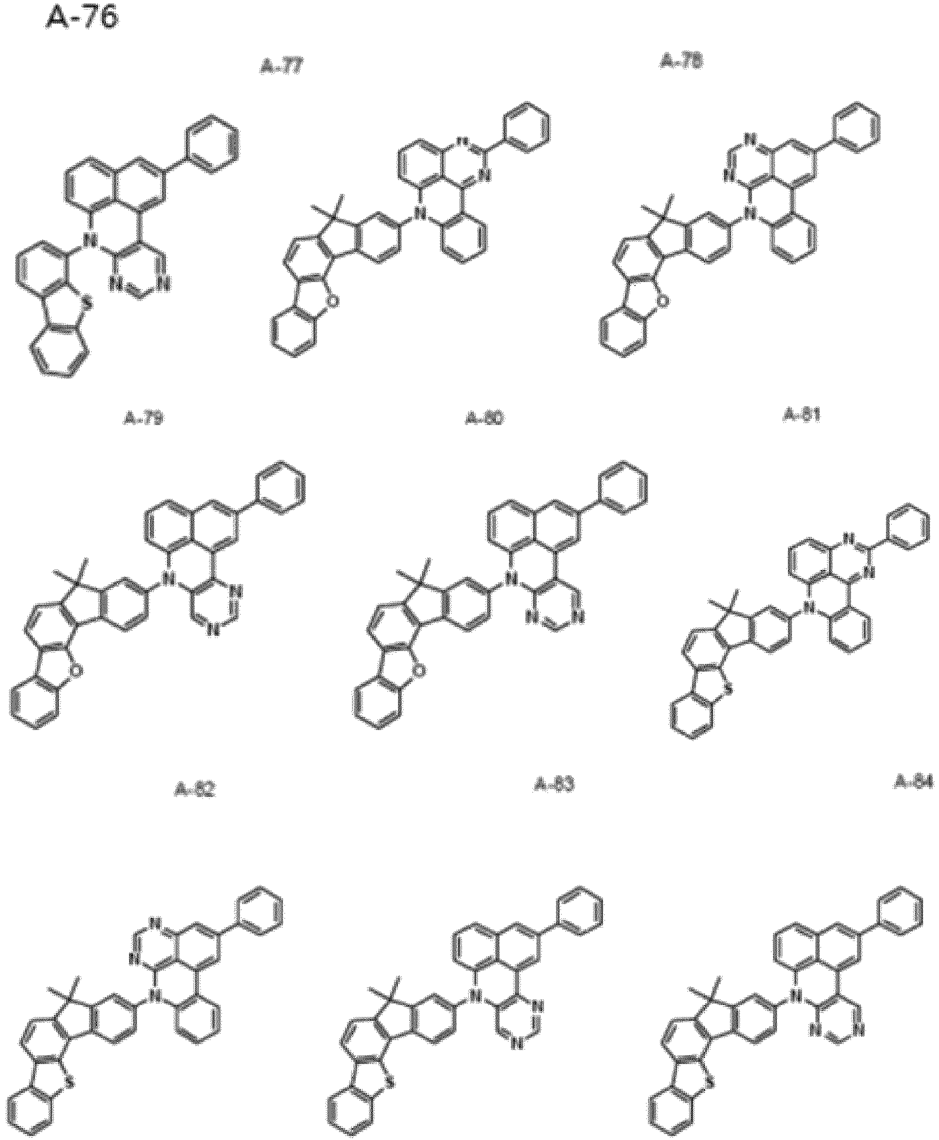

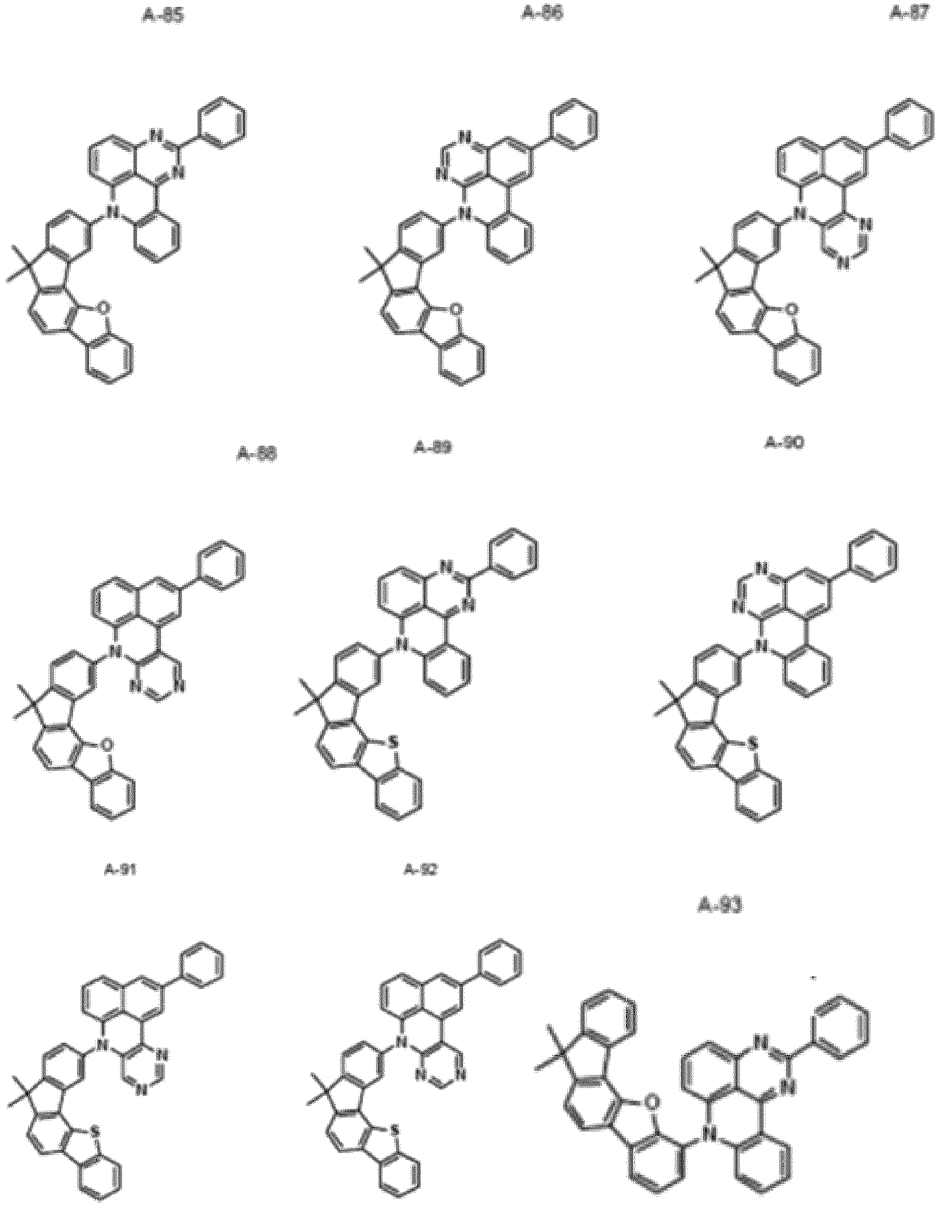

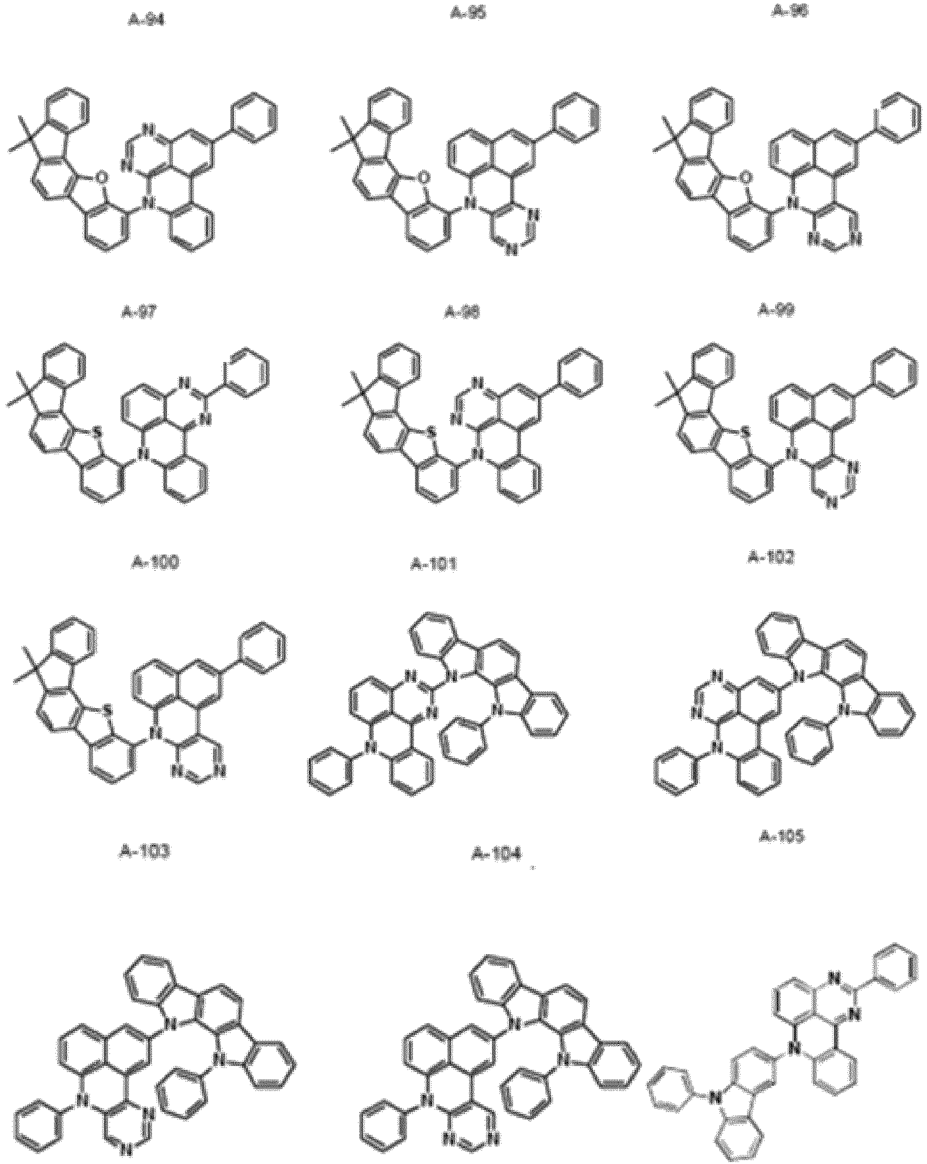

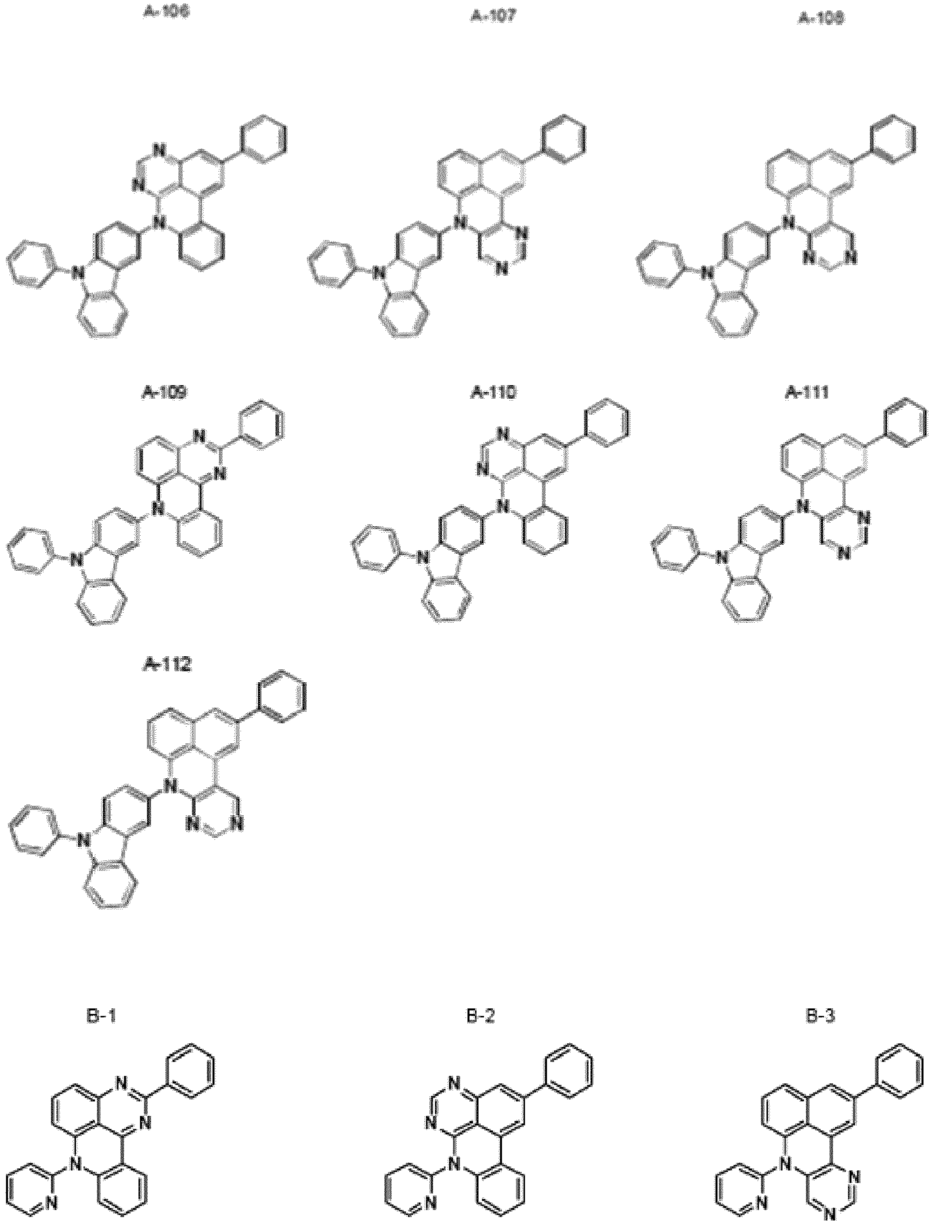

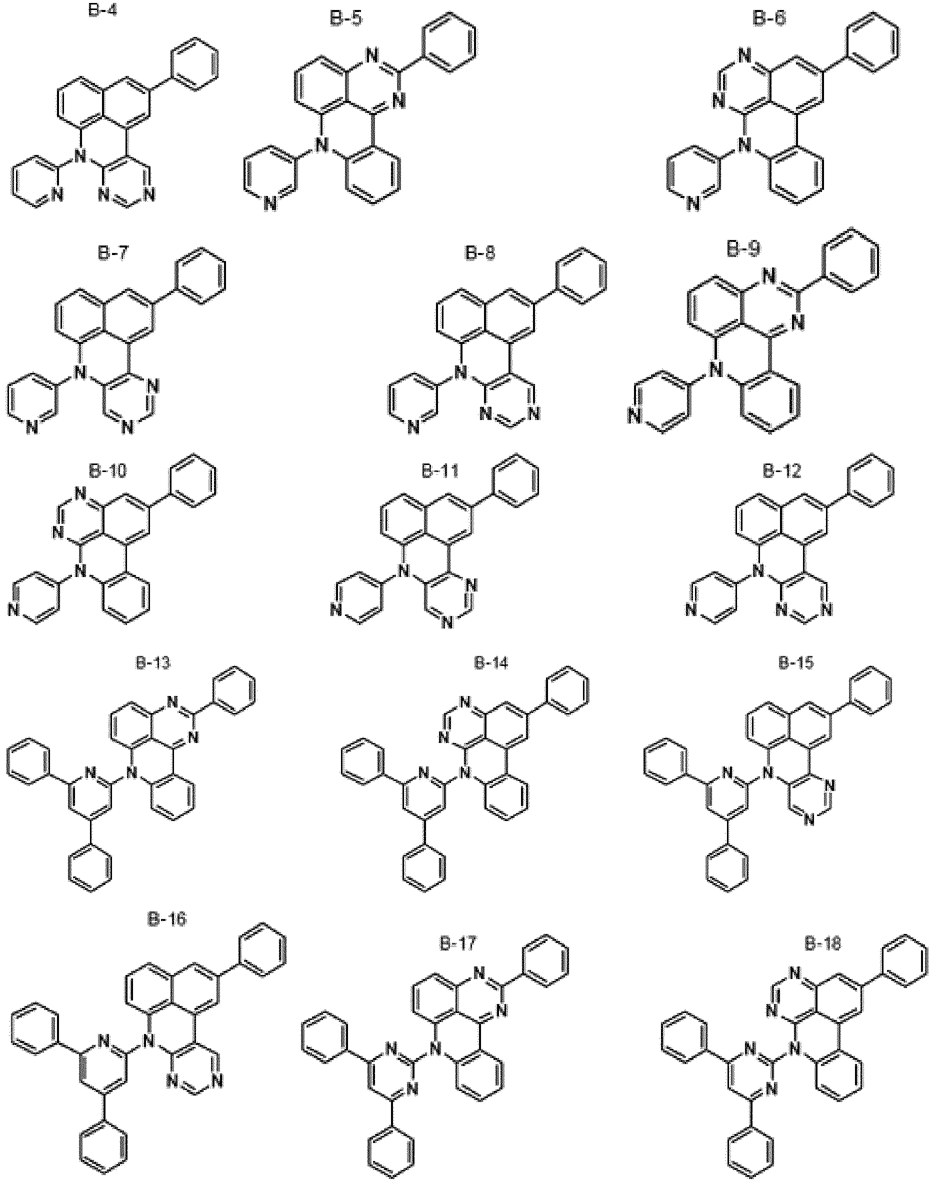

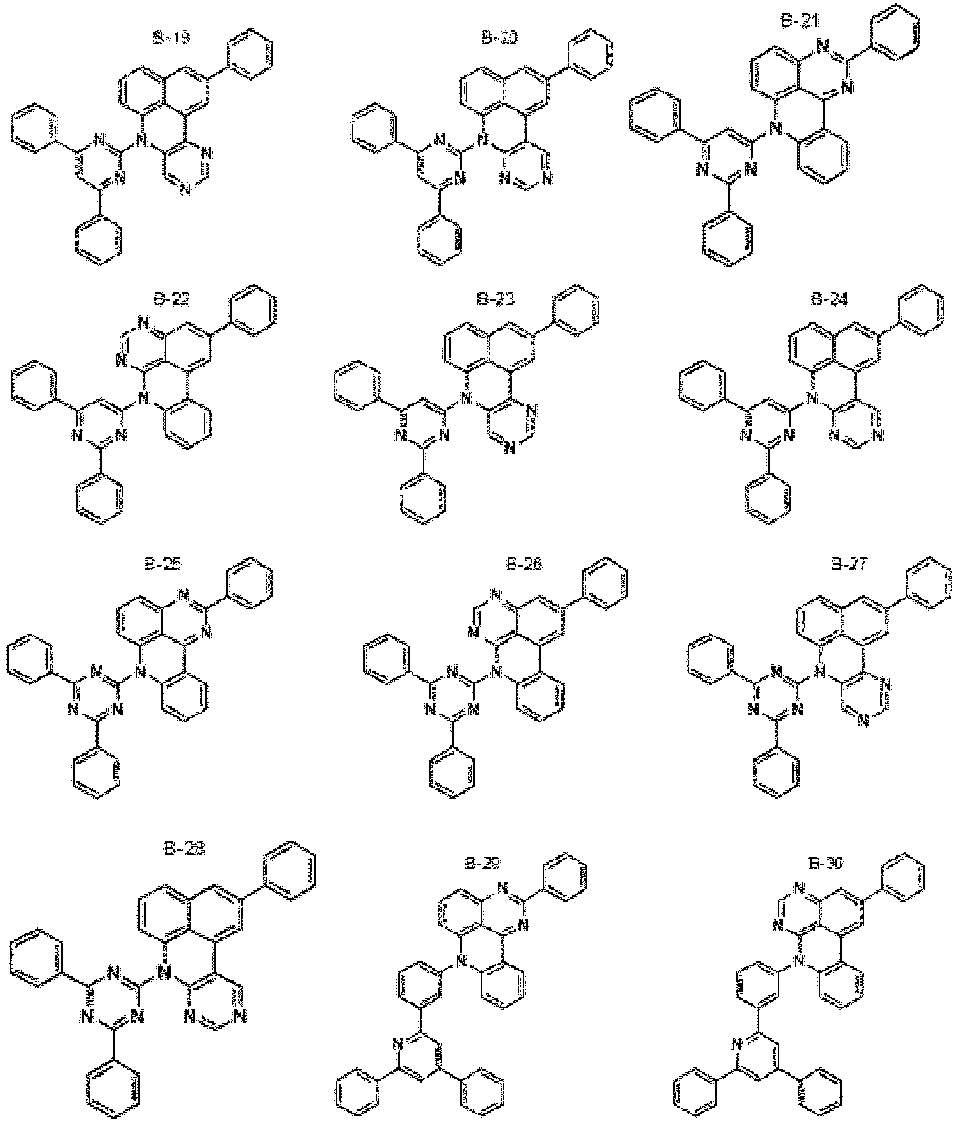

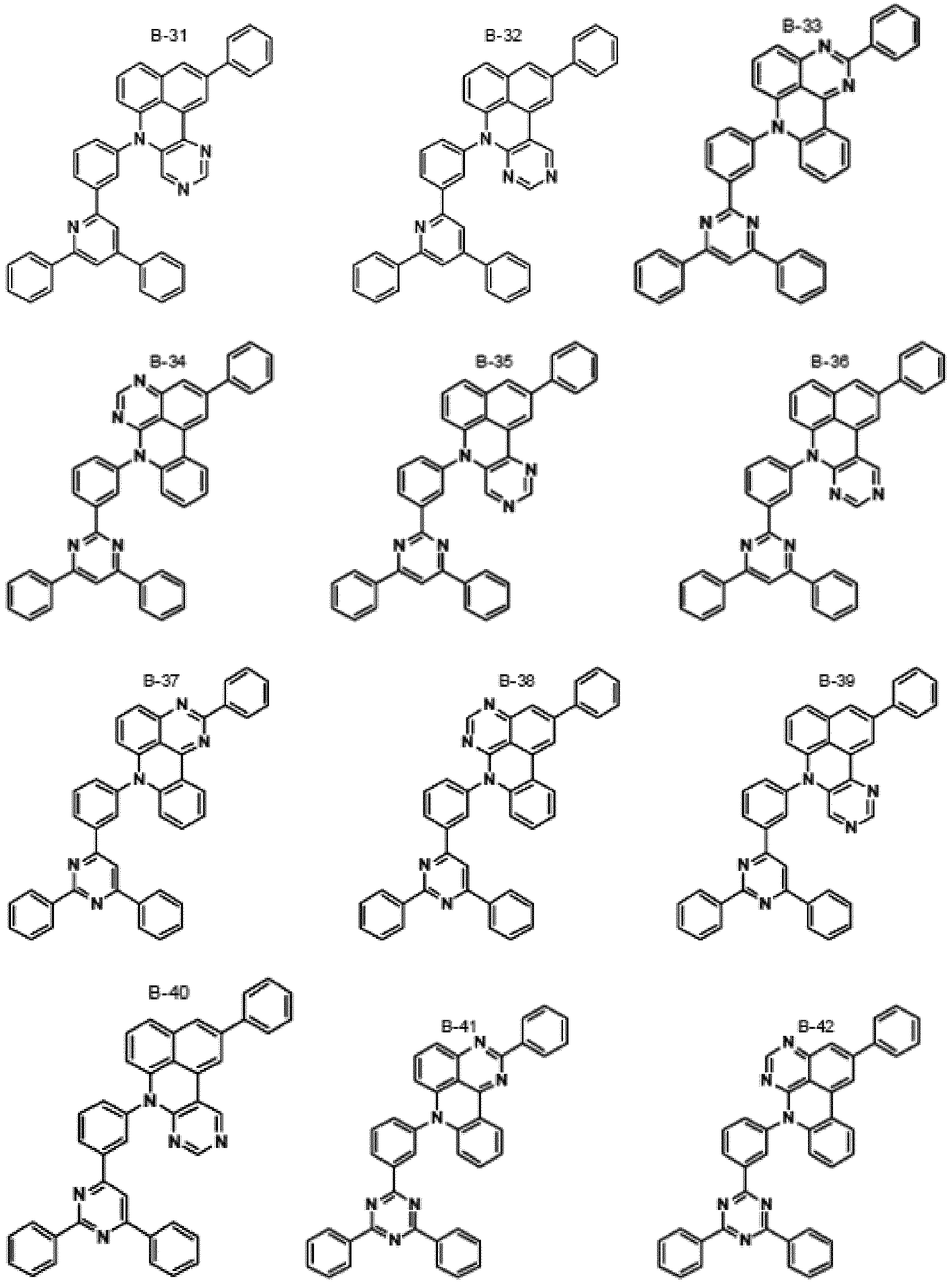

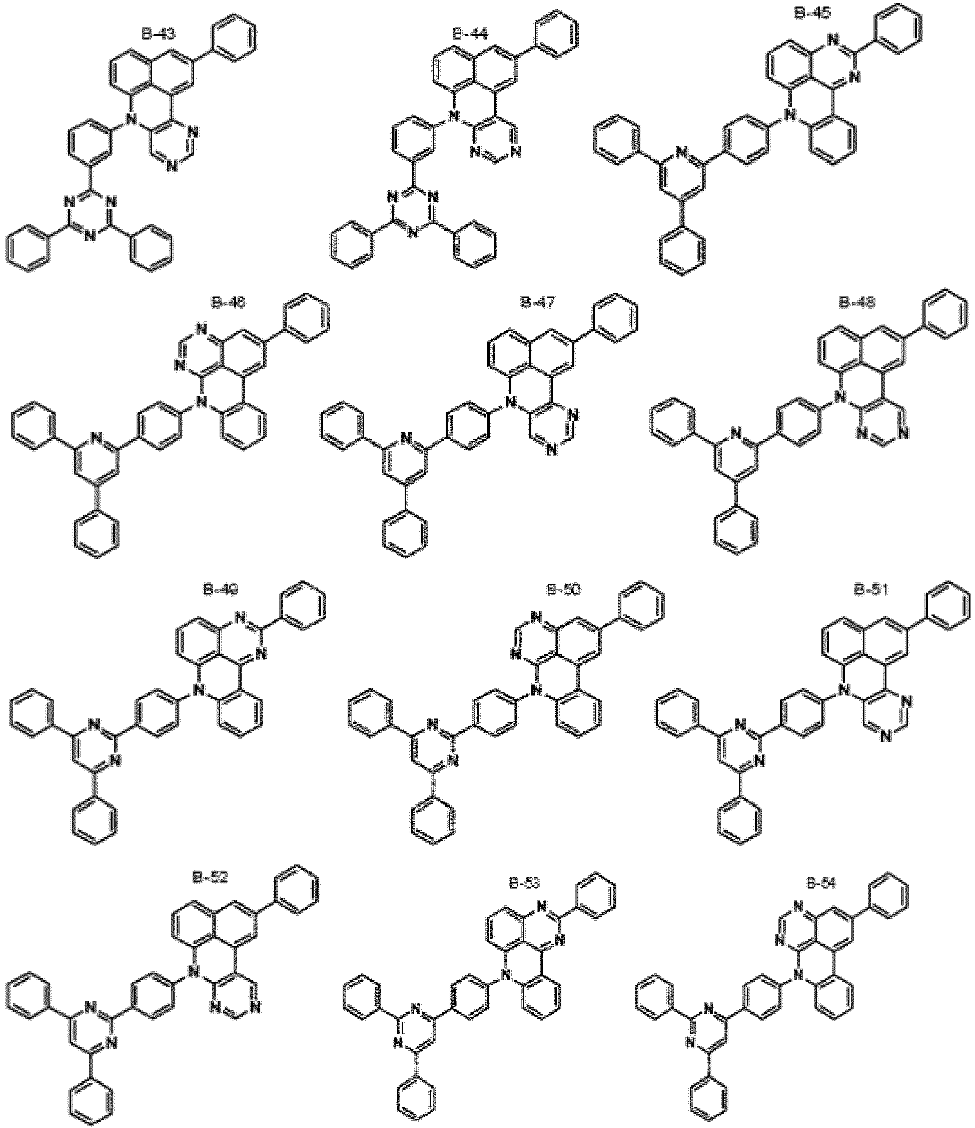

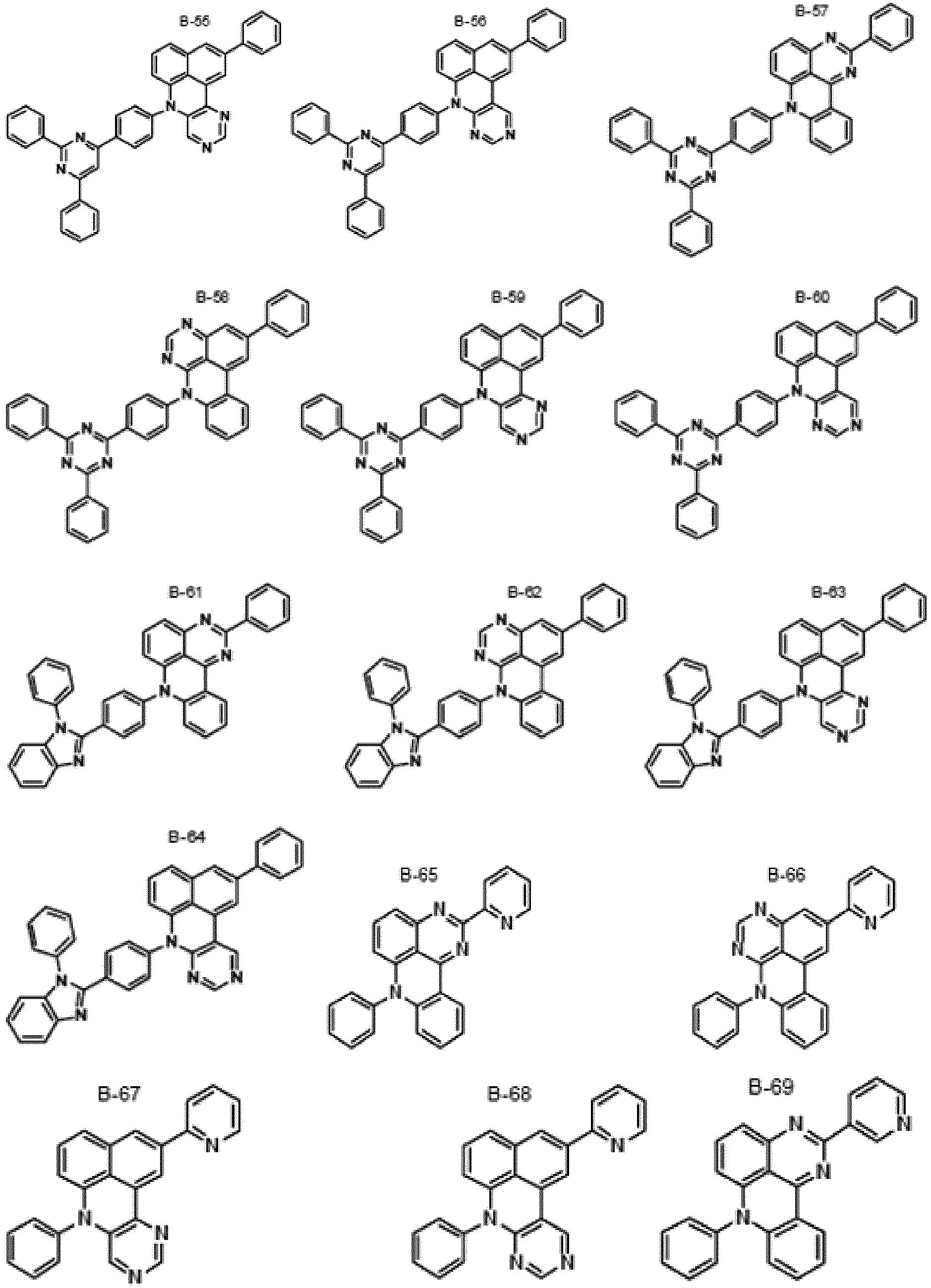

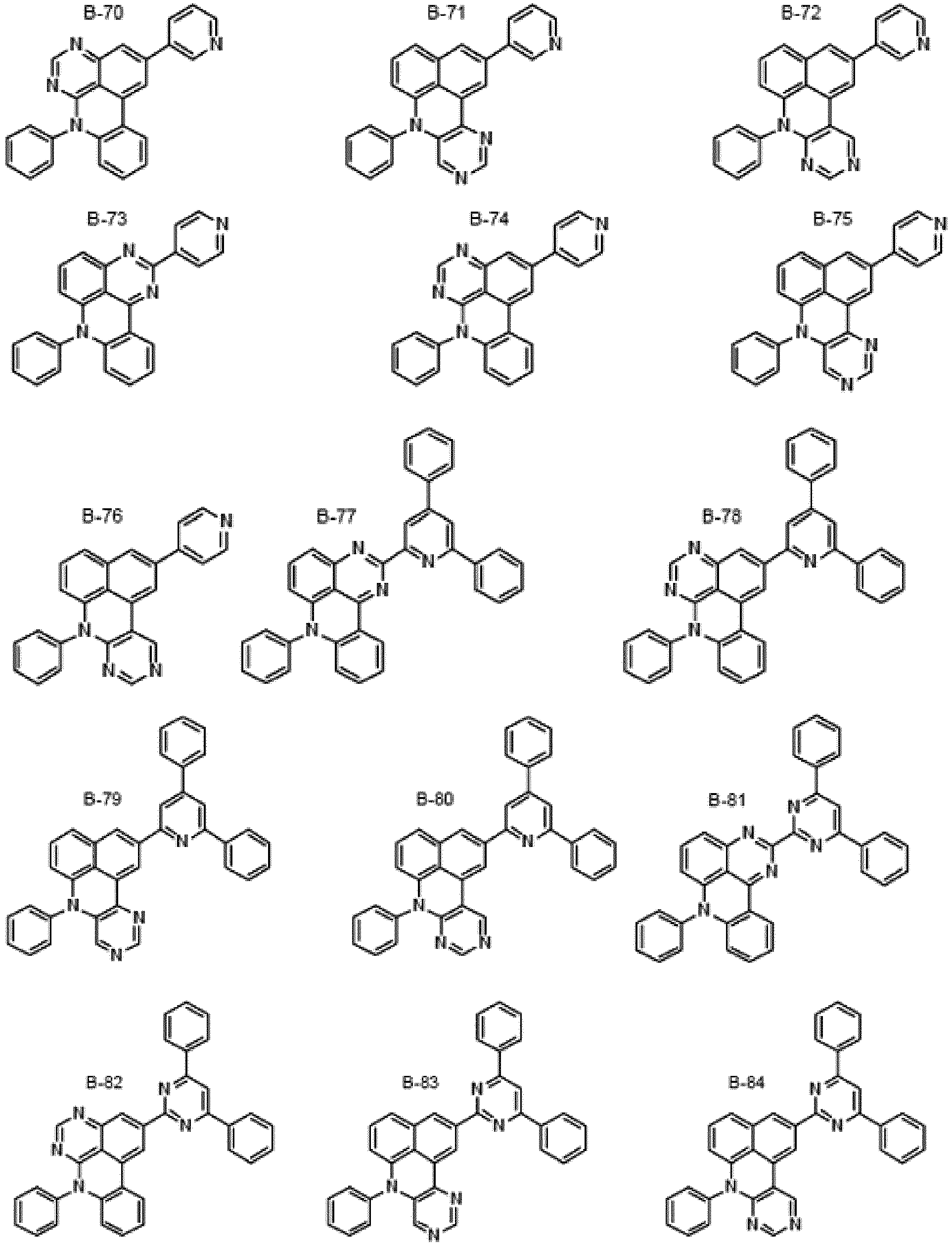

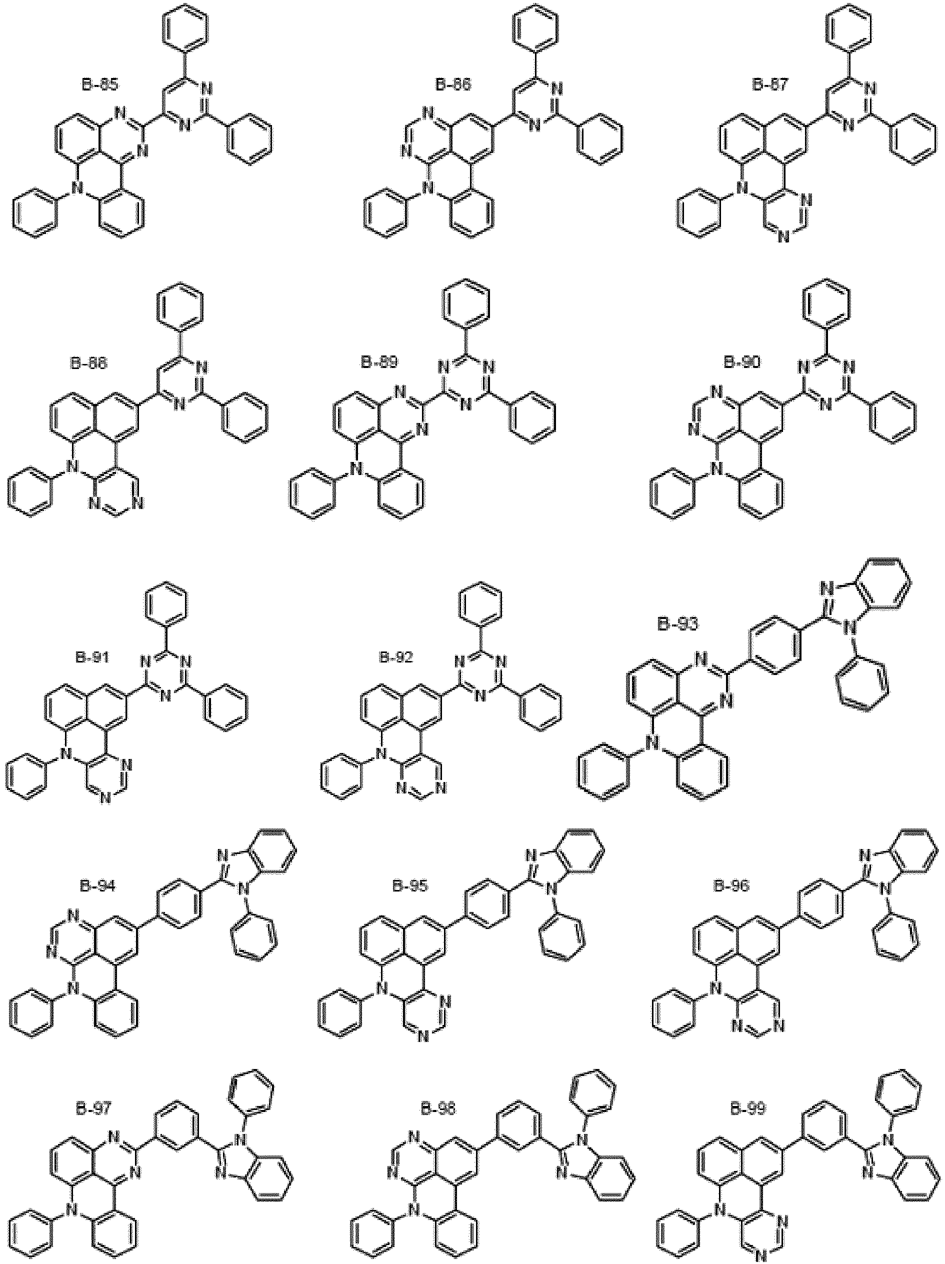

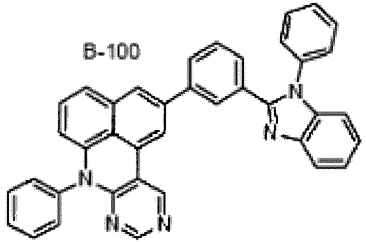

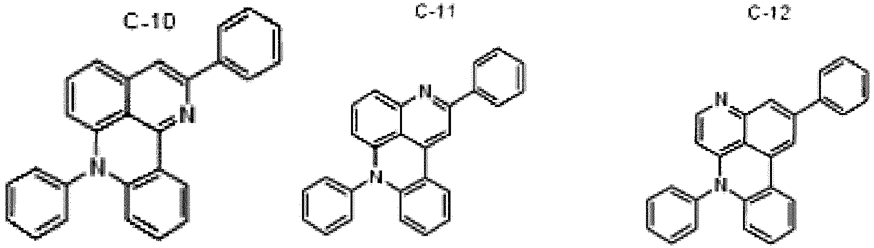

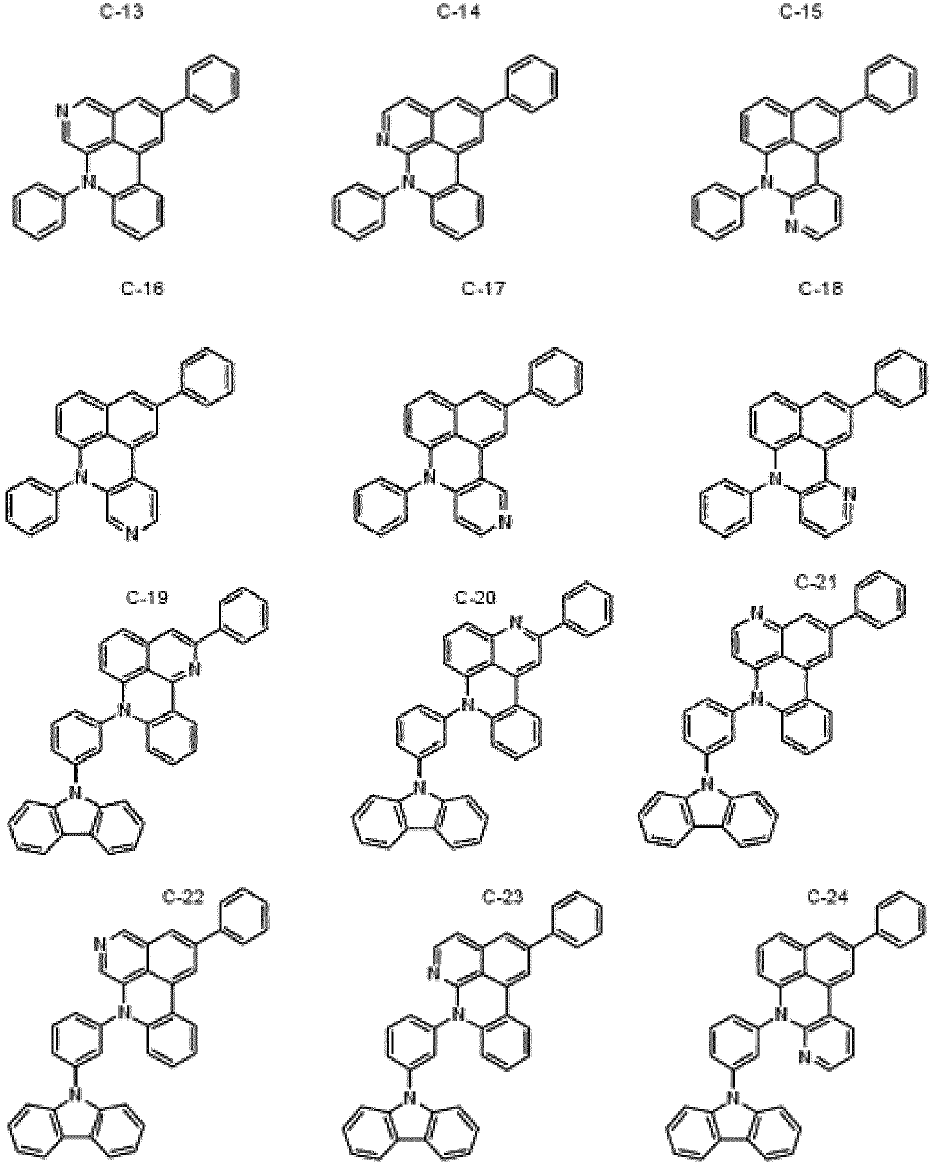

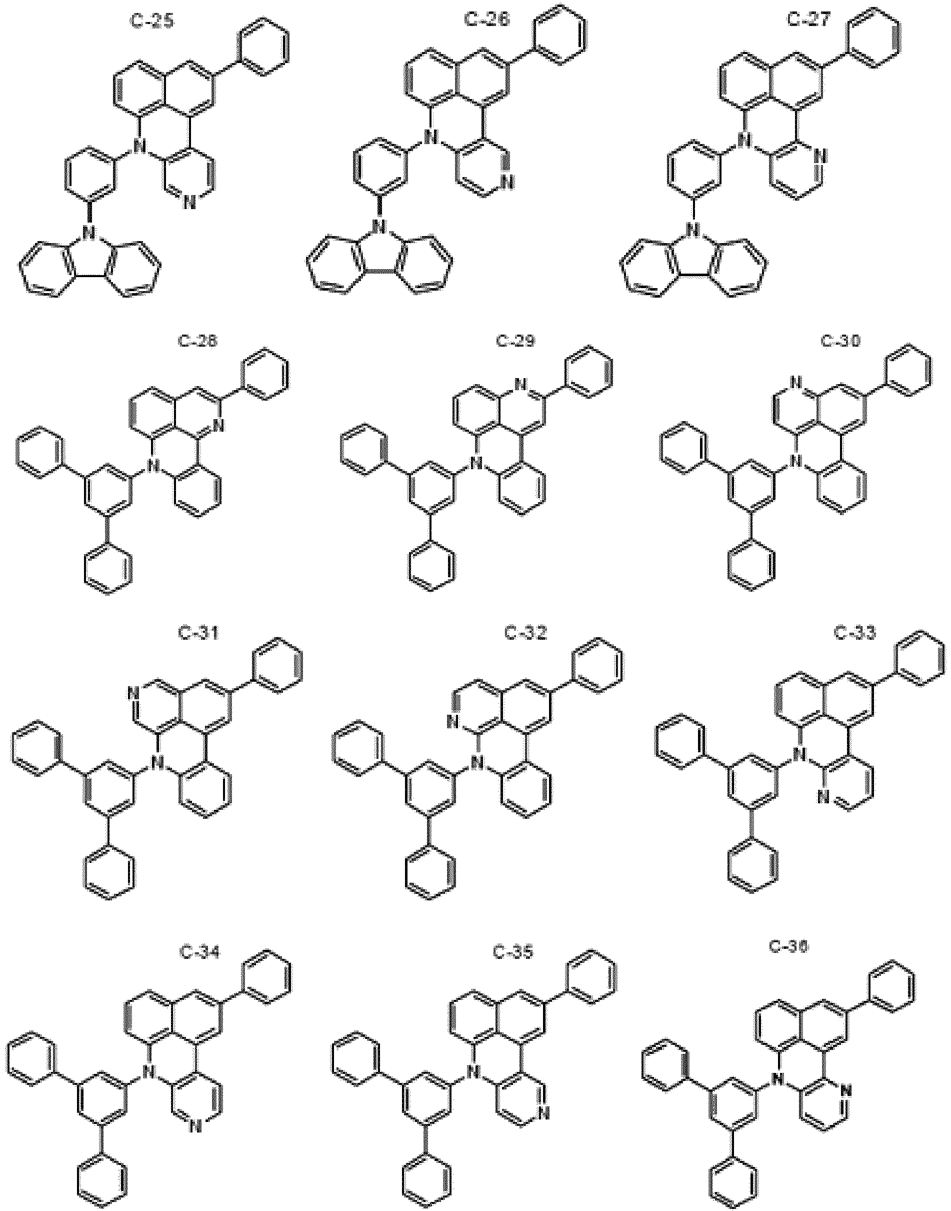

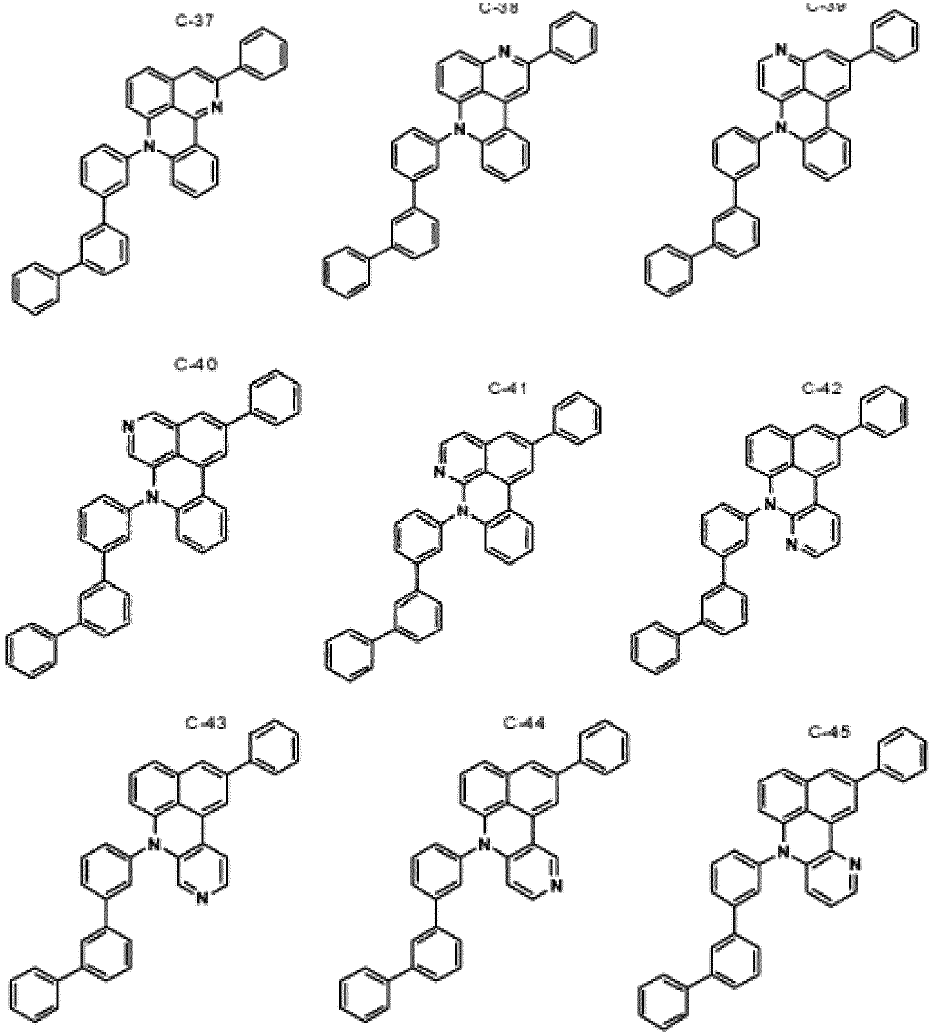

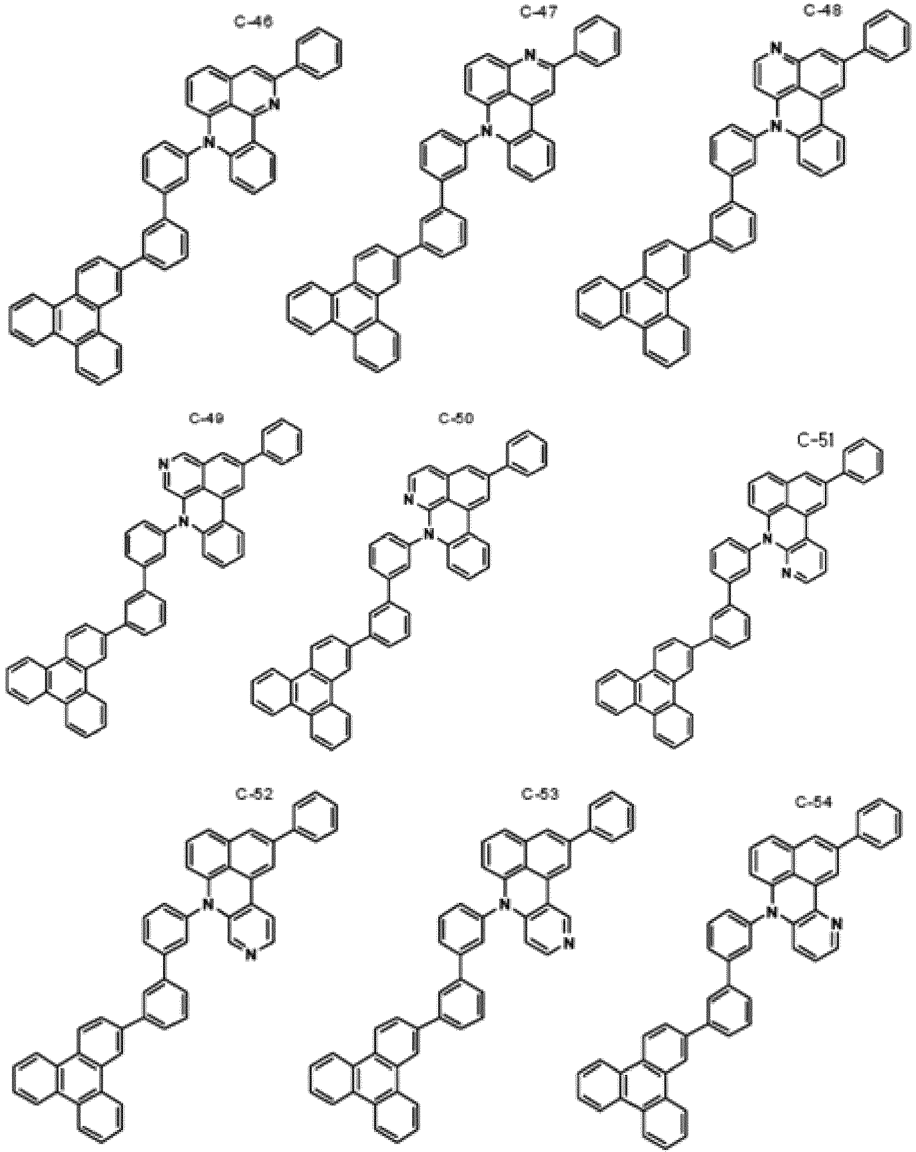

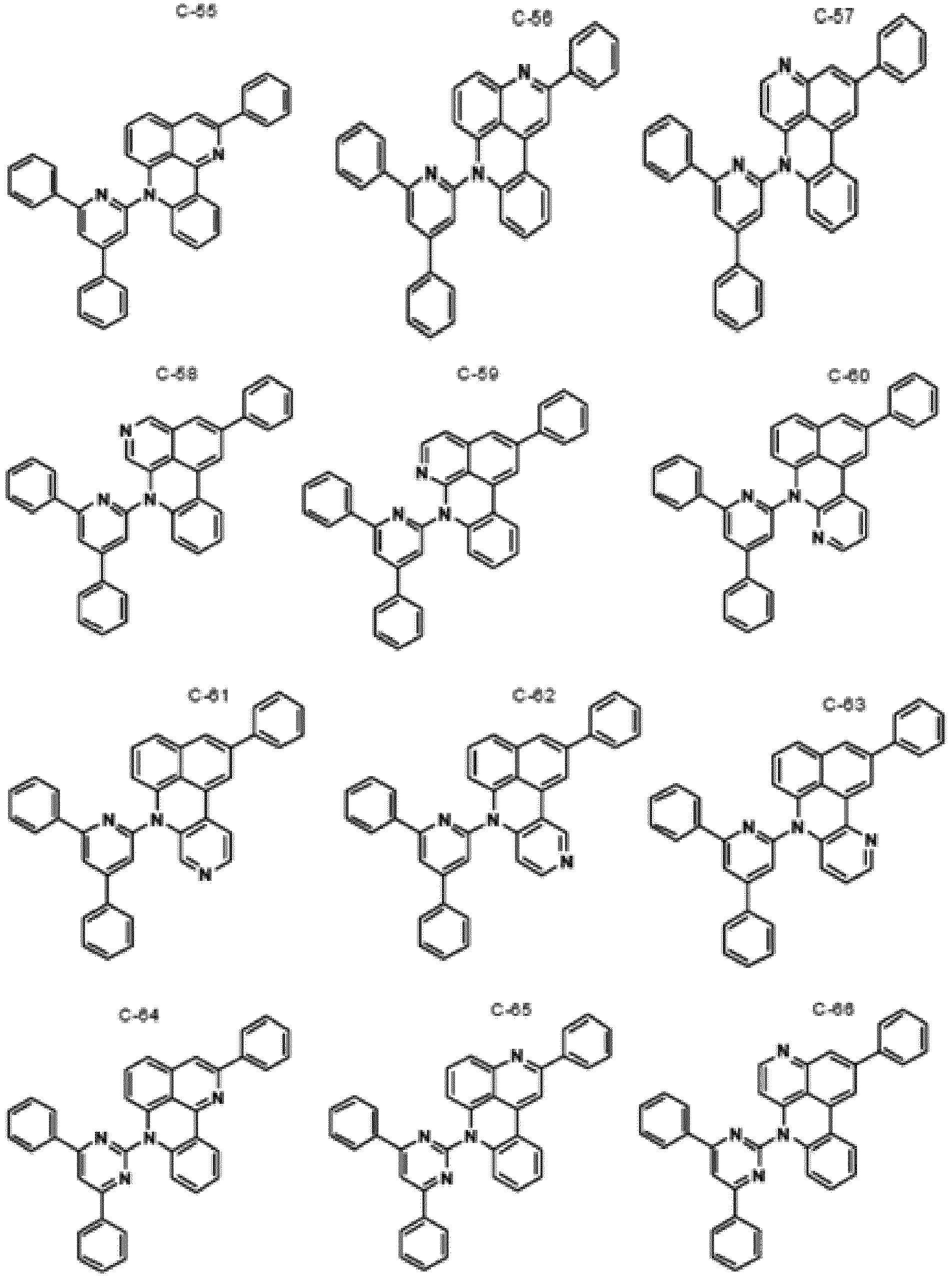

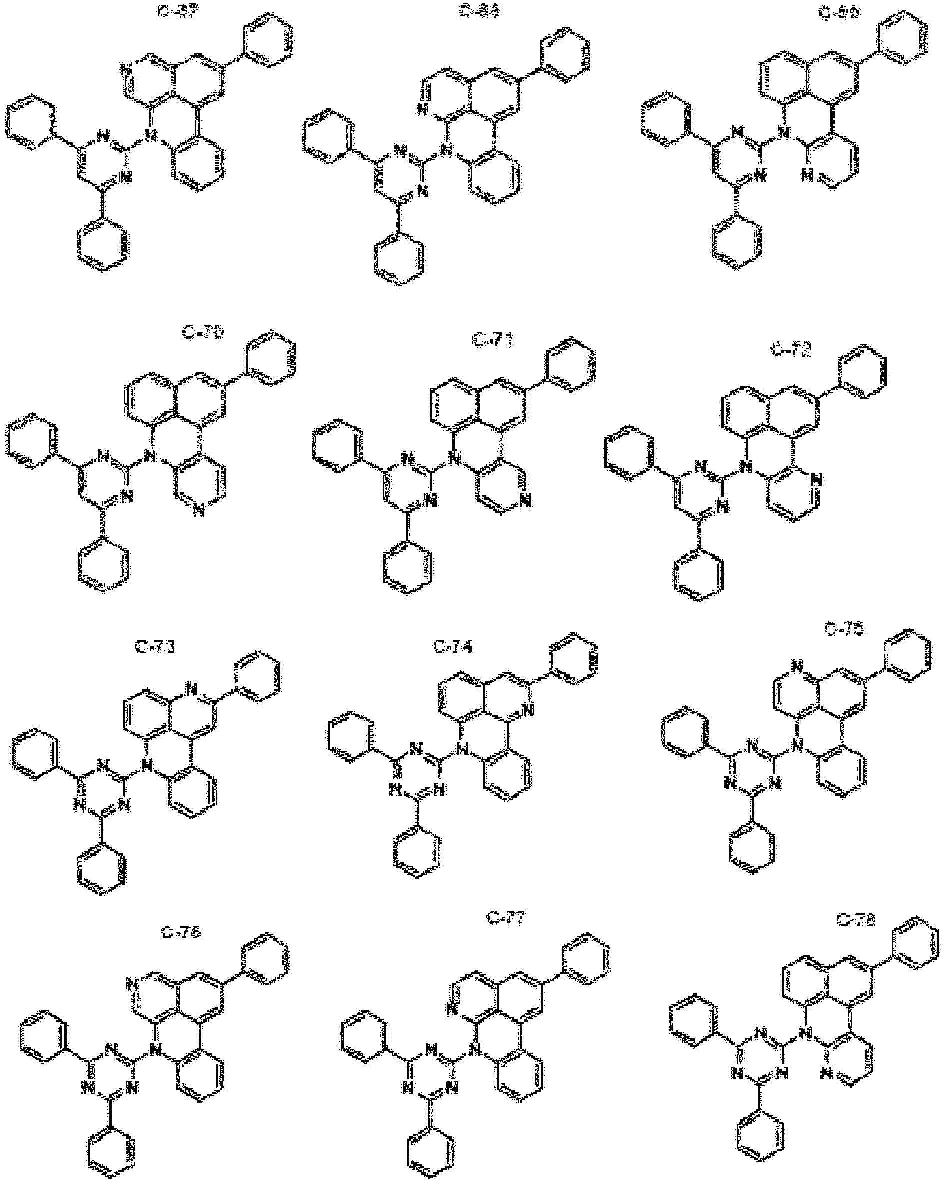

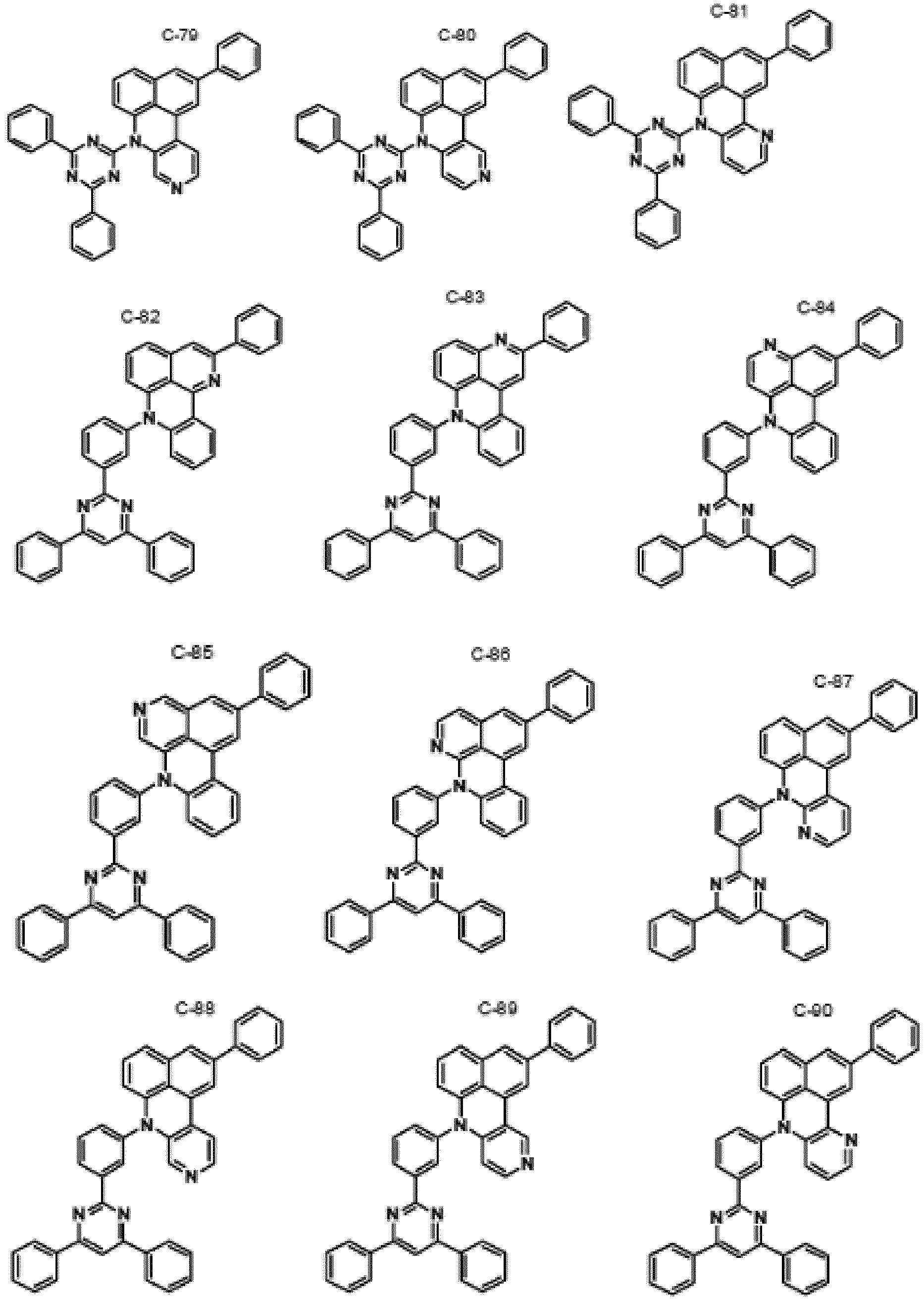

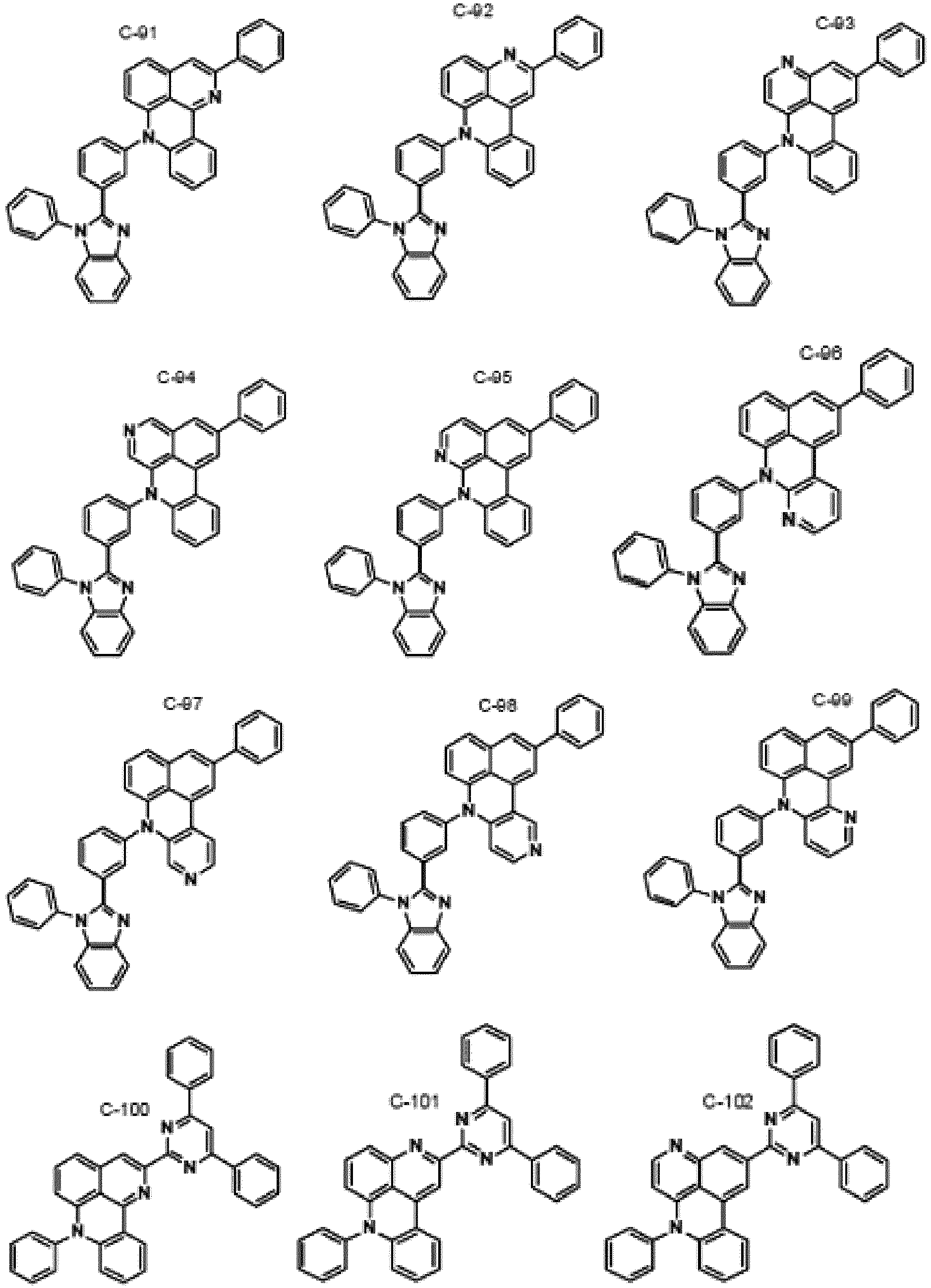

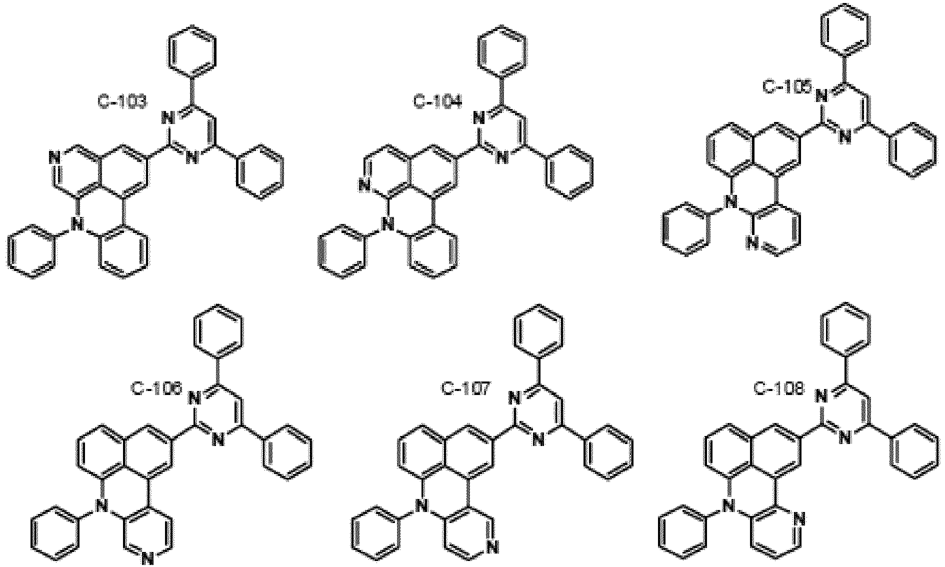

4. An organic optoelectronic device comprising
an anode (120) and a cathode (110) facing each other, and
at least one organic layer (130) positioned between the anode and the cathode
wherein, the organic layer comprises the compound of any one of claim 1 to claim 2.

5. The organic optoelectronic device of claim 4, wherein the organic layer comprises an emission layer,
wherein the emission layer comprises the compound.

6. The organic optoelectronic device of claim 5, wherein the compound is included as a host of the emission layer.

7. The organic optoelectronic device of claim 4, wherein the organic layer comprises at least one auxiliary layer selected from a hole injection layer, a hole transport layer, an electron blocking layer, an electron transport layer, an electron injection layer and a hole blocking layer,
wherein the auxiliary layer comprises the compound.

8. An organic optoelectronic device comprising
an anode (120) and a cathode (110) facing each other, and
at least one organic layer (130) positioned between the anode and the cathode wherein, the organic layer comprises the compound according to claim 3.

9. A display device comprising the organic optoelectronic device of claim 4.

10. A display device comprising the organic optoelectronic device of claim 8.

## Patentansprüche

1. Verbindung, die durch eine der folgenden chemischen Formeln 15 bis 27 dargestellt ist, wobei die Verbindung für eine organische optoelektronische Vorrichtung verwendet wird: 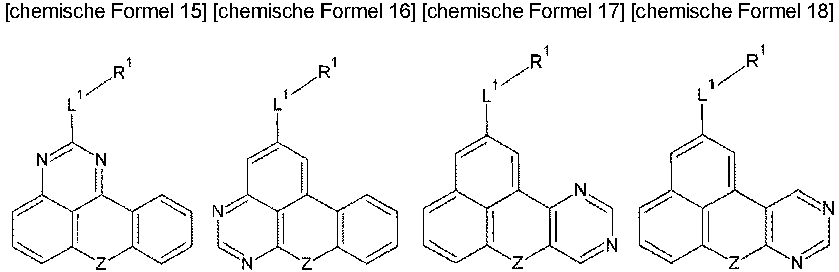 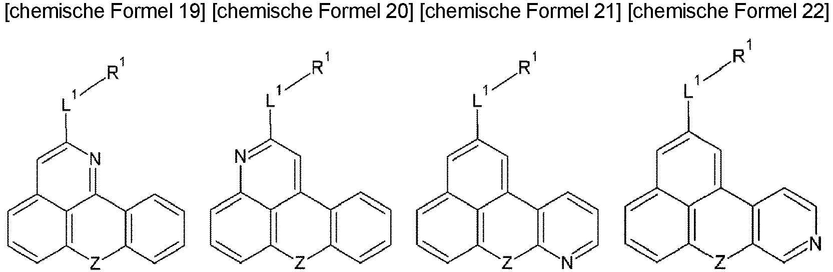 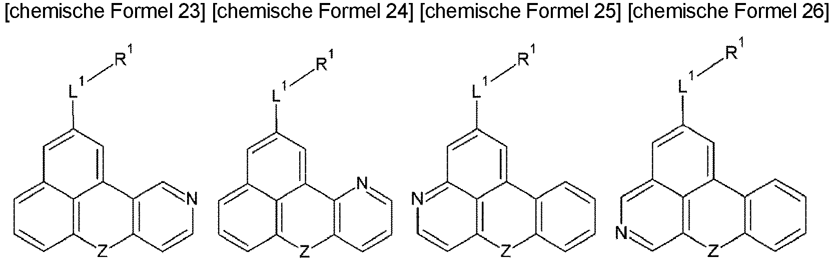 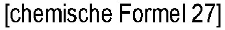 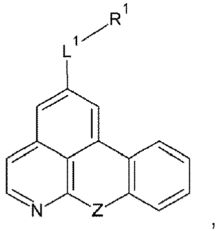 wobei in den chemischen Formeln 15 bis 27:
Z N-L⁴-R^{b} ist,
L¹ und L⁴ unabhängig voneinander eine Einfachbindung, eine substituierte oder unsubstituierte C6- bis C30-Arylengruppe, eine substituierte oder unsubstituierte heterocyclische C2- bis C30-Gruppe oder eine Kombination davon sind, und
wobei R¹ und R^{b} unabhängig voneinander eine Gruppe sind, die ausgewählt ist aus substituierten oder unsubstituierten Gruppen, die in der folgenden Gruppe II aufgeführt sind: 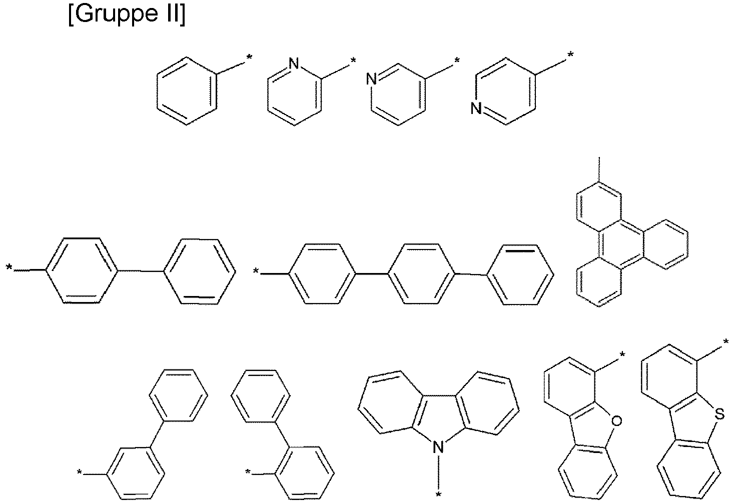 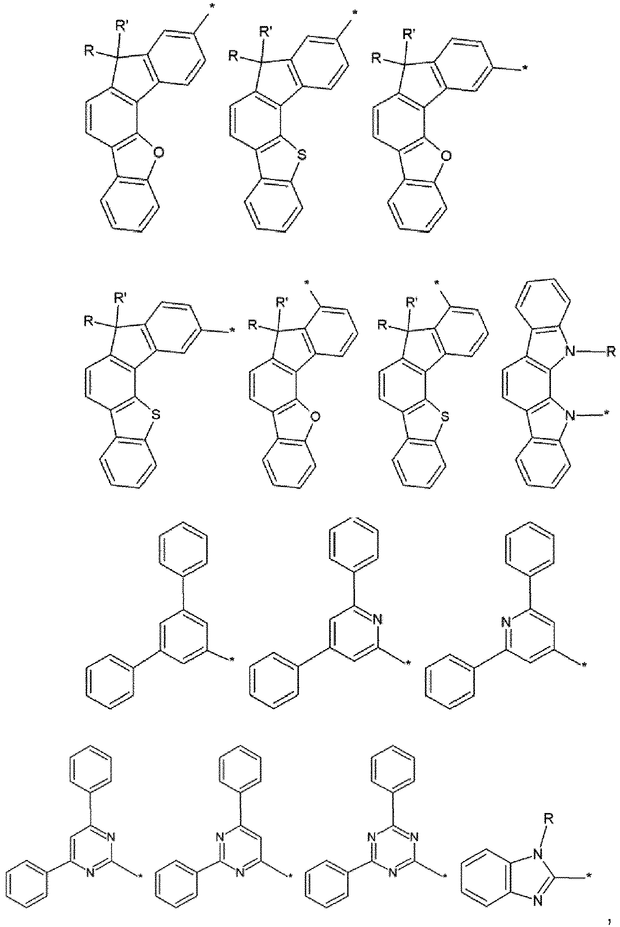 wobei in Gruppe II,
R und R' unabhängig voneinander Wasserstoff, Deuterium, eine substituierte oder unsubstituierte C1- bis C30-Alkylgruppe, eine substituierte oder unsubstituierte C6- bis C30-Arylgruppe, eine substituierte oder unsubstituierte C2- bis C30-Heteroarylgruppe oder eine Kombination davon sind, und
* ein Bindungspunkt ist.

2. Verbindung nach Anspruch 1, wobei L¹ und L⁴ unabhängig voneinander eine Einfachbindung oder eine Gruppe sind, die aus substituierten oder unsubstituierten Gruppen ausgewählt ist, die in der folgenden Gruppe I aufgeführt sind: 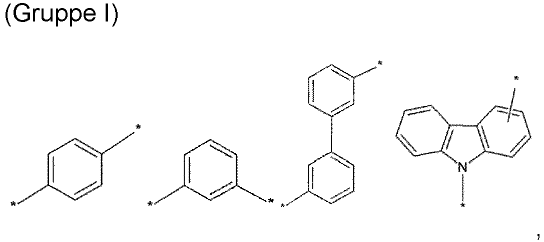
wobei in Gruppe I
* einen Bindungspunkt darstellt.

3. Verbindung, die durch eine der folgenden Verbindungen dargestellt ist, die in der folgenden Gruppe III aufgeführt sind:

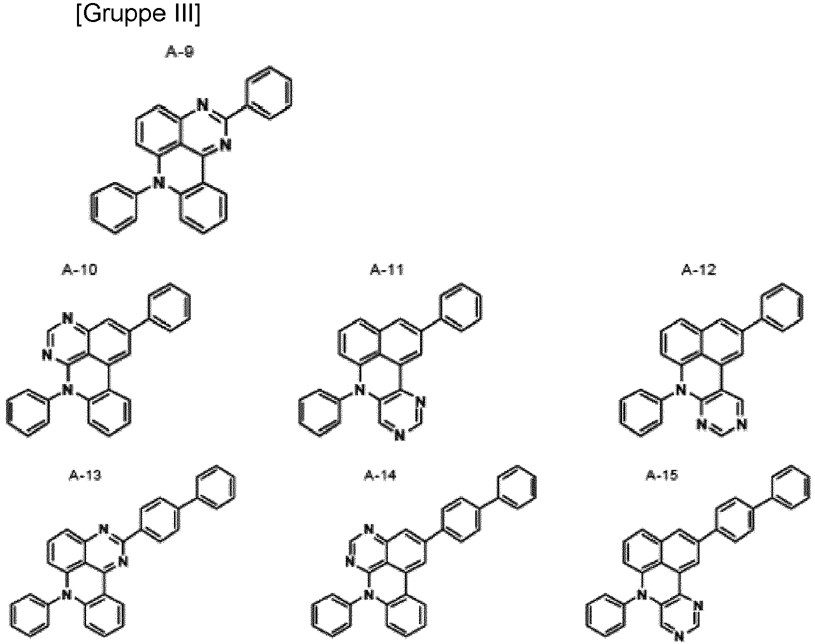

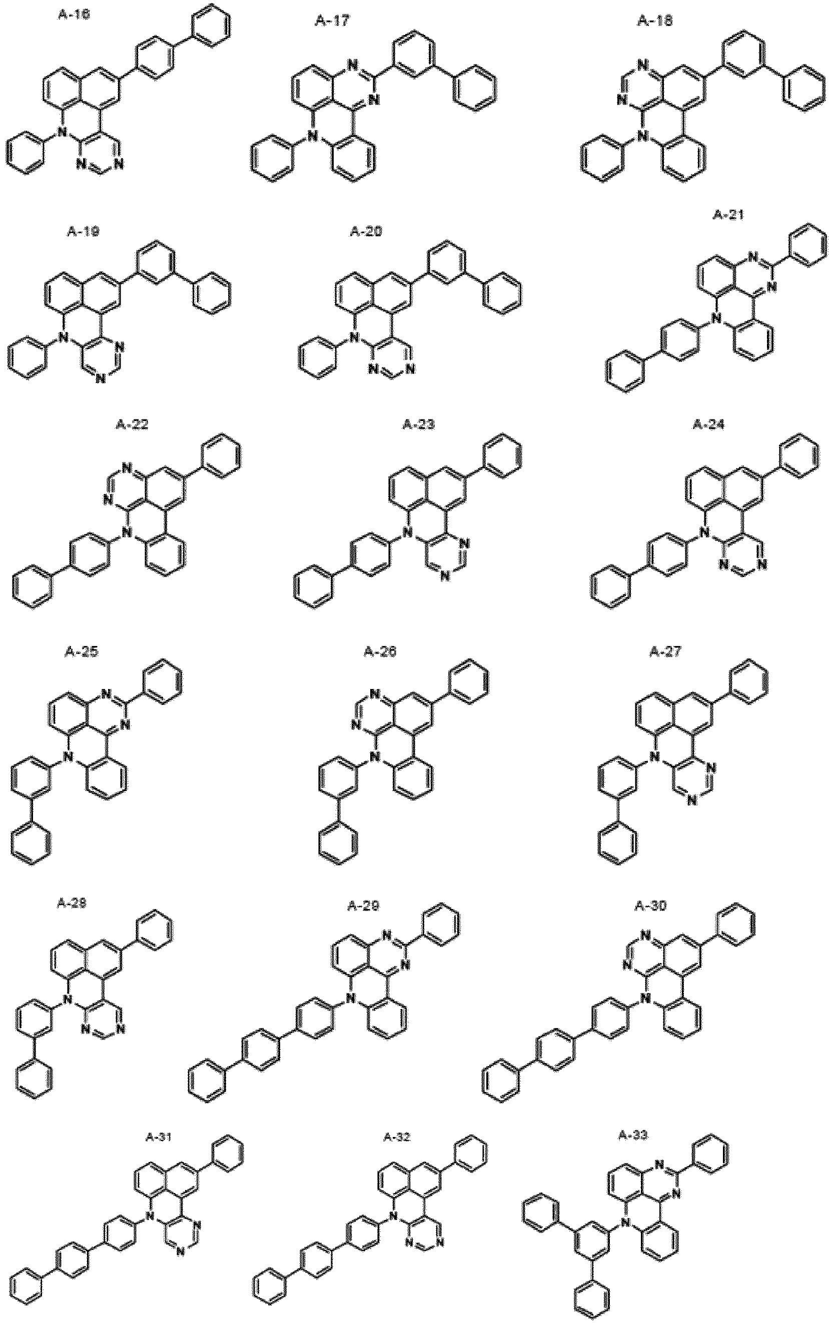

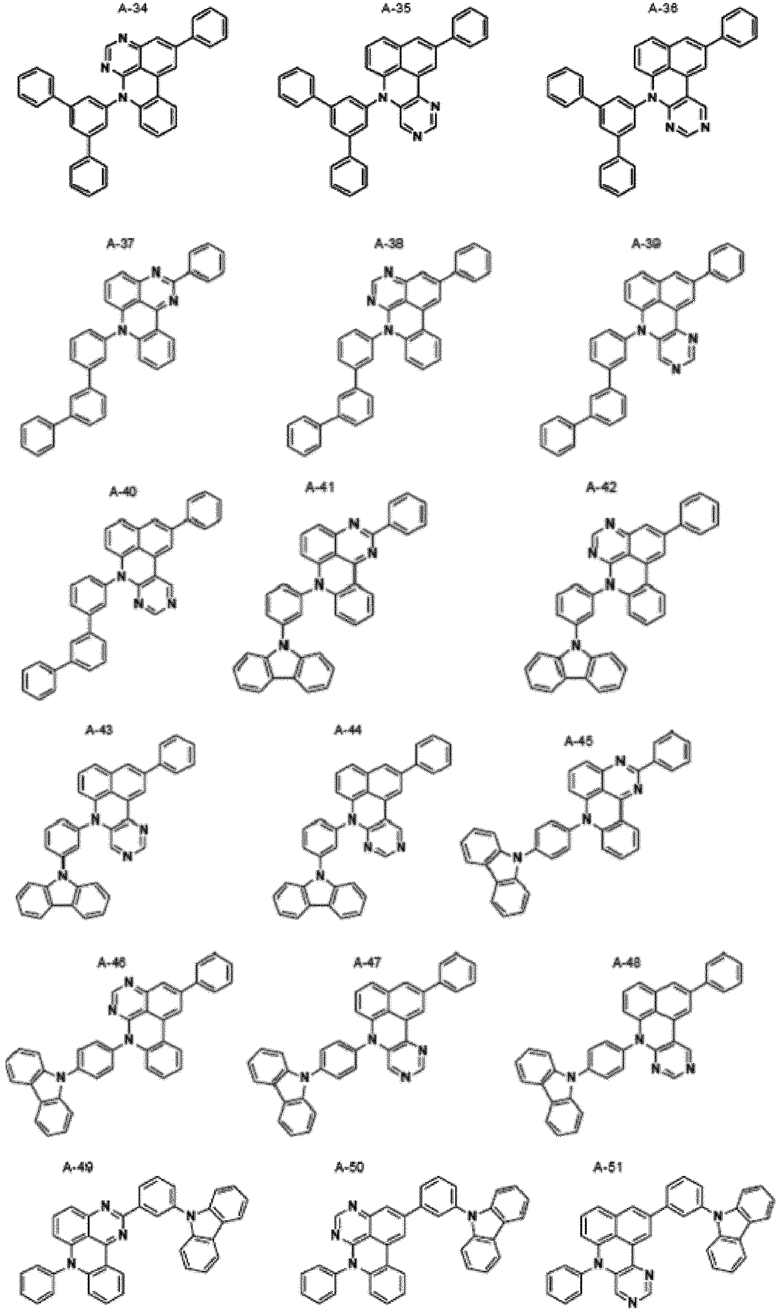

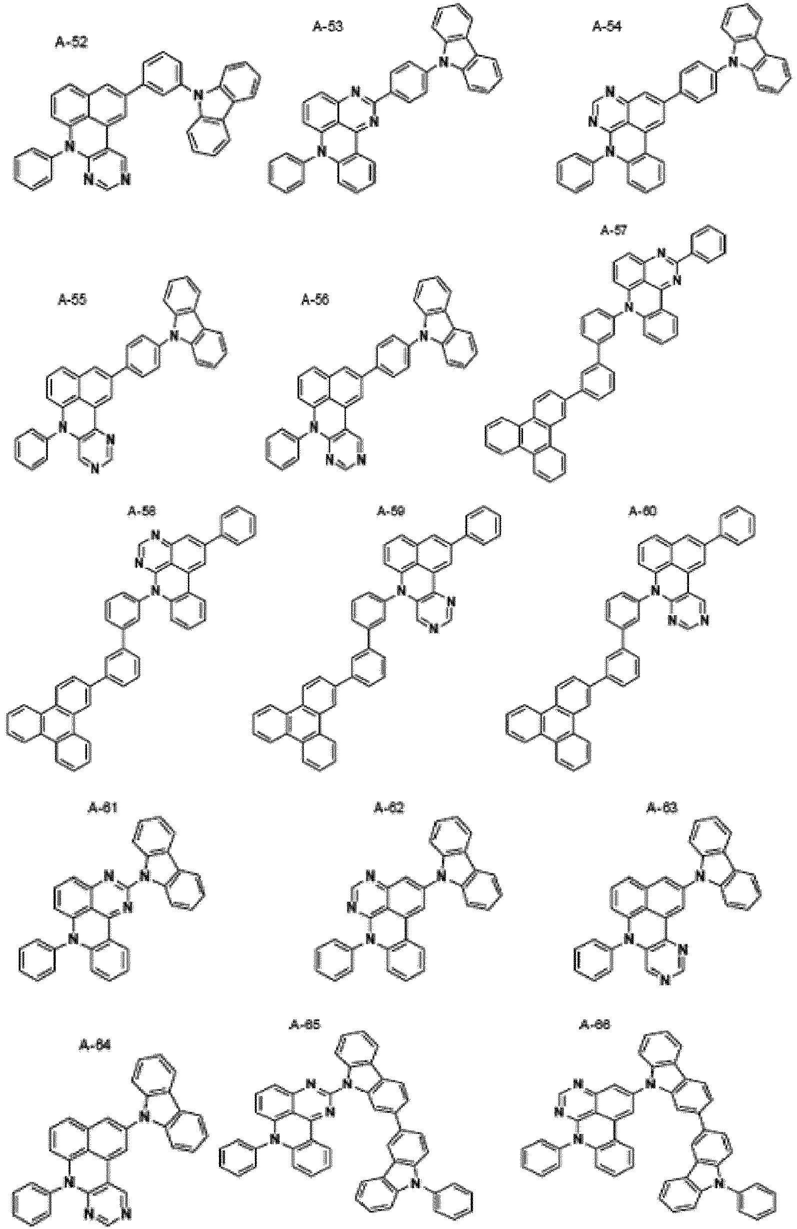

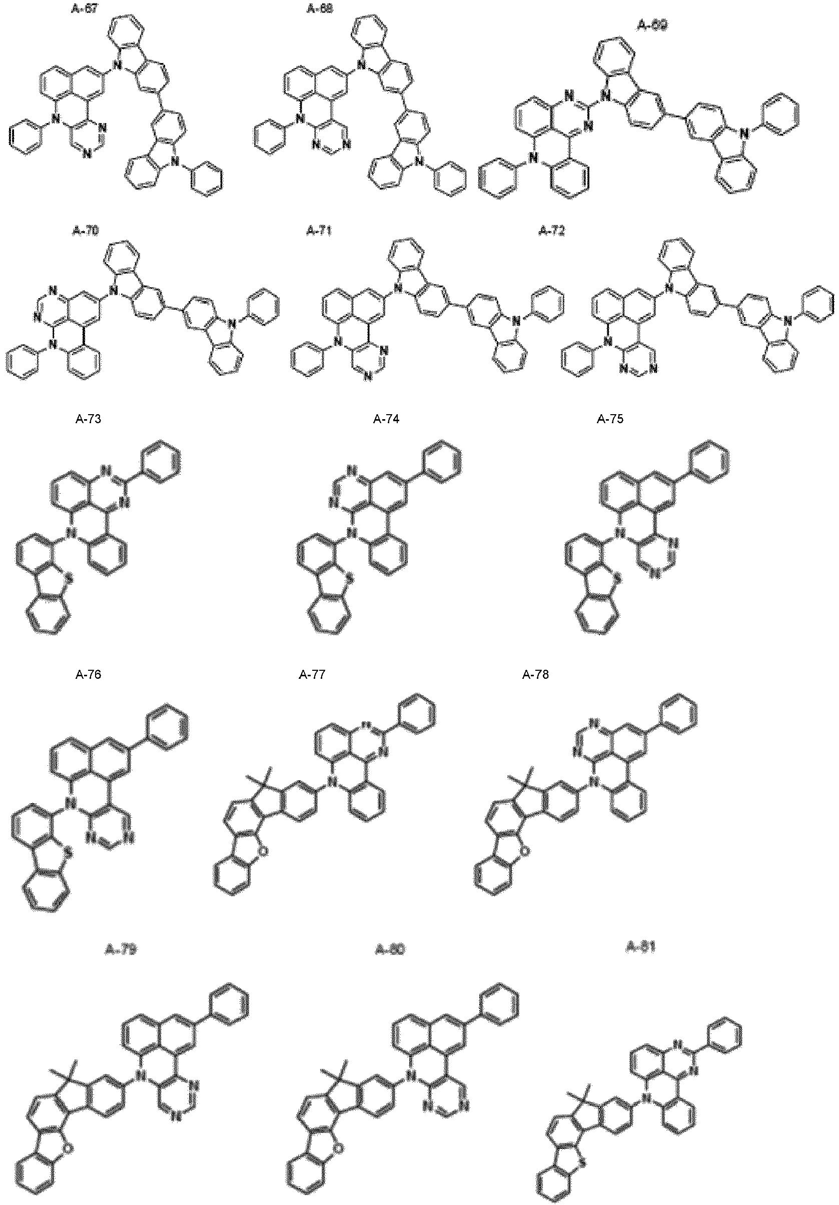

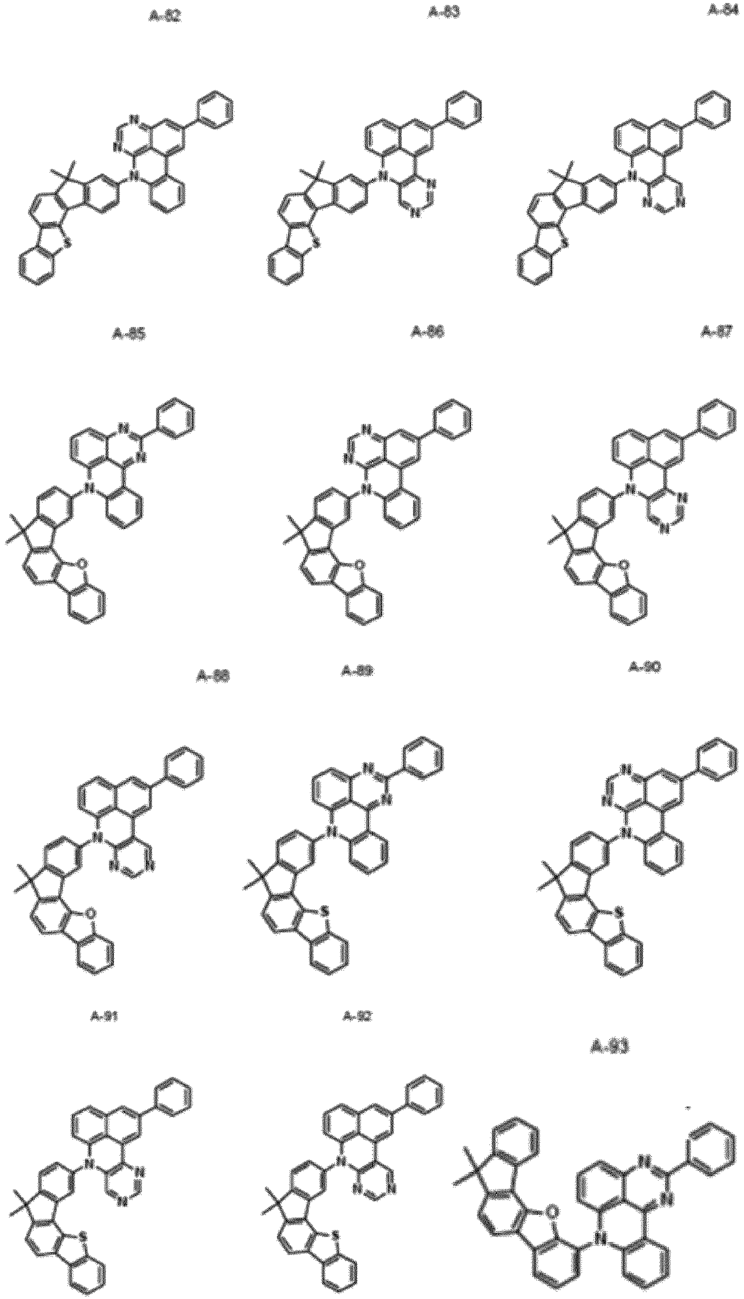

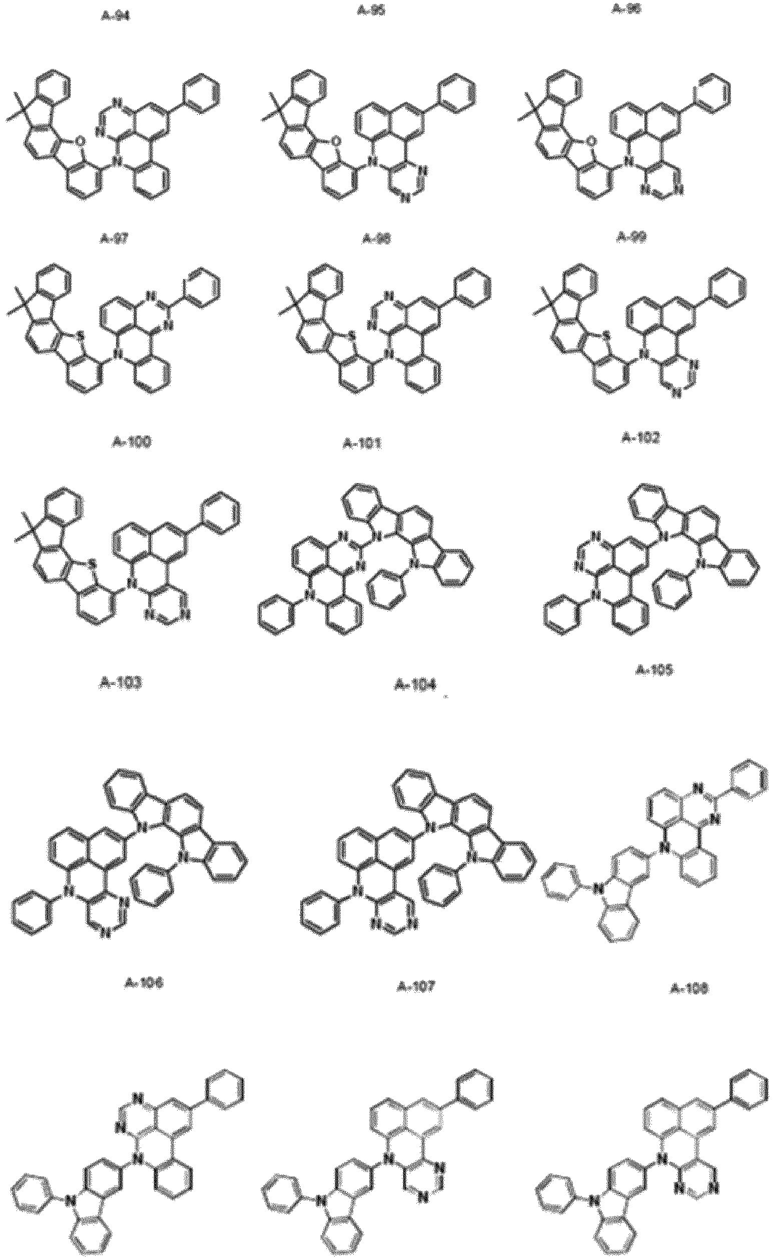

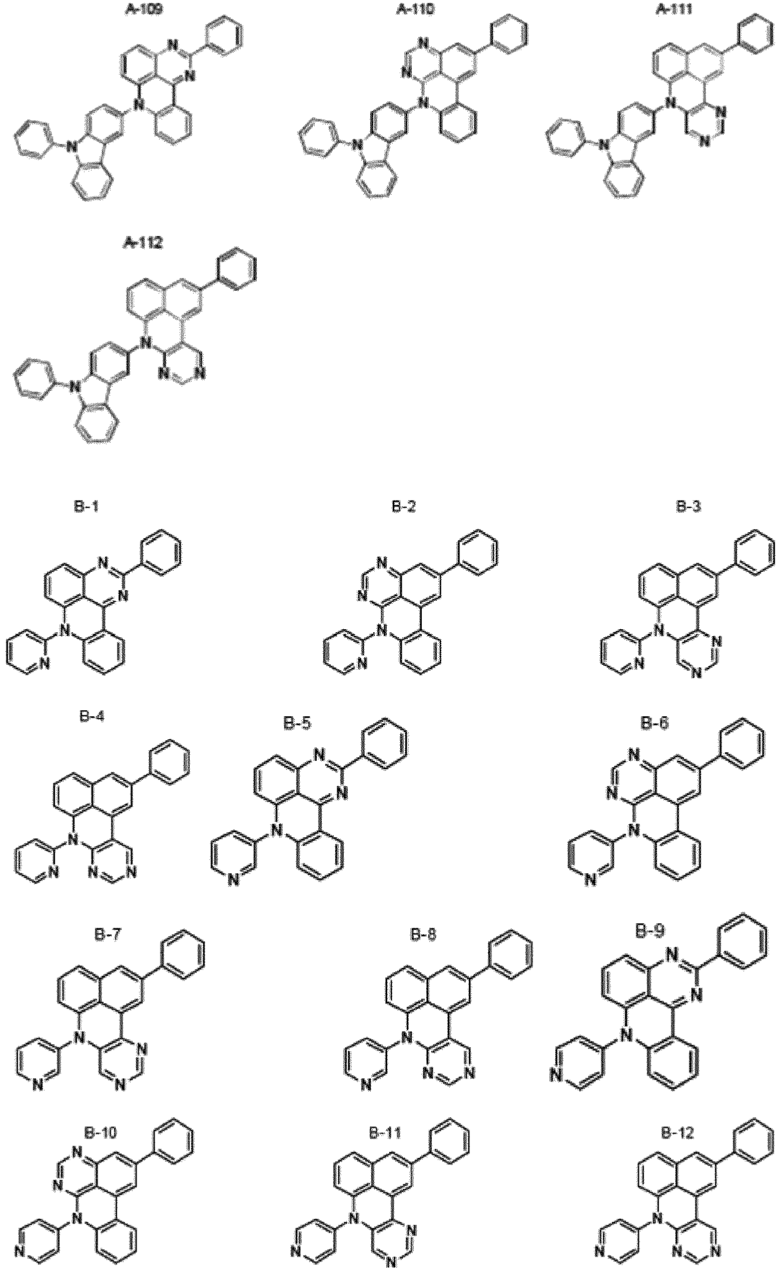

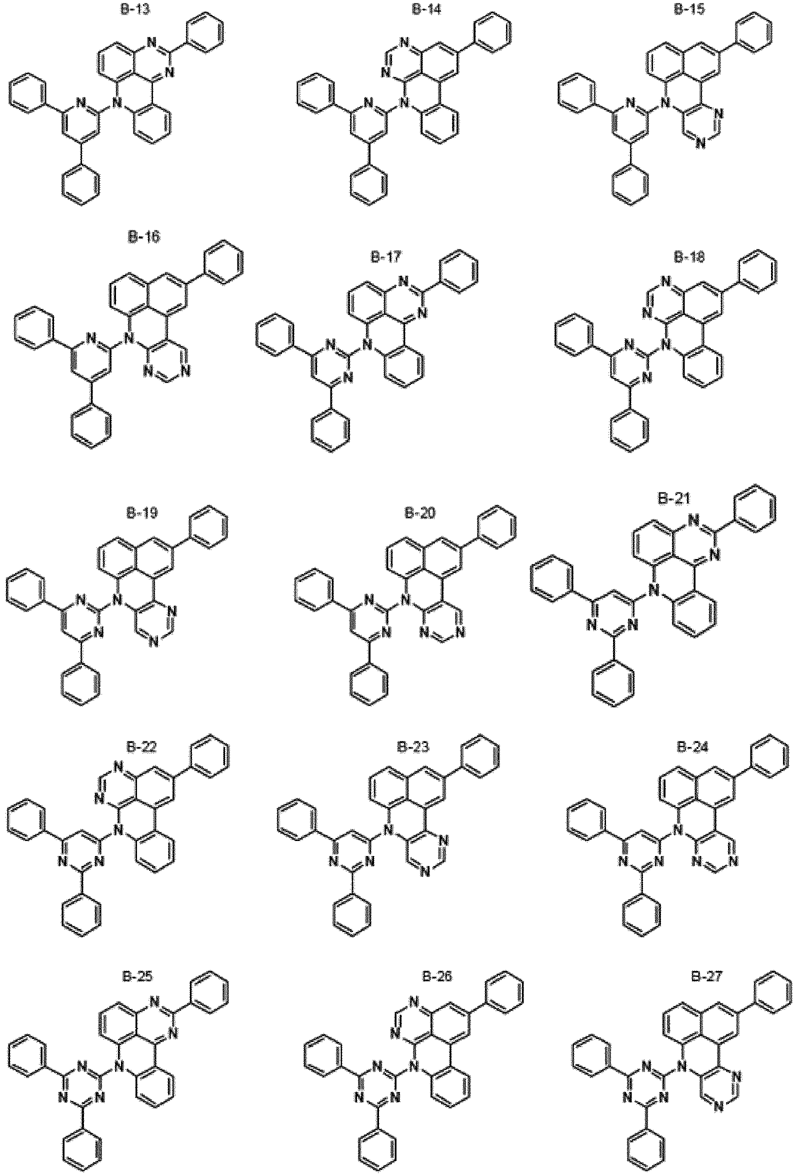

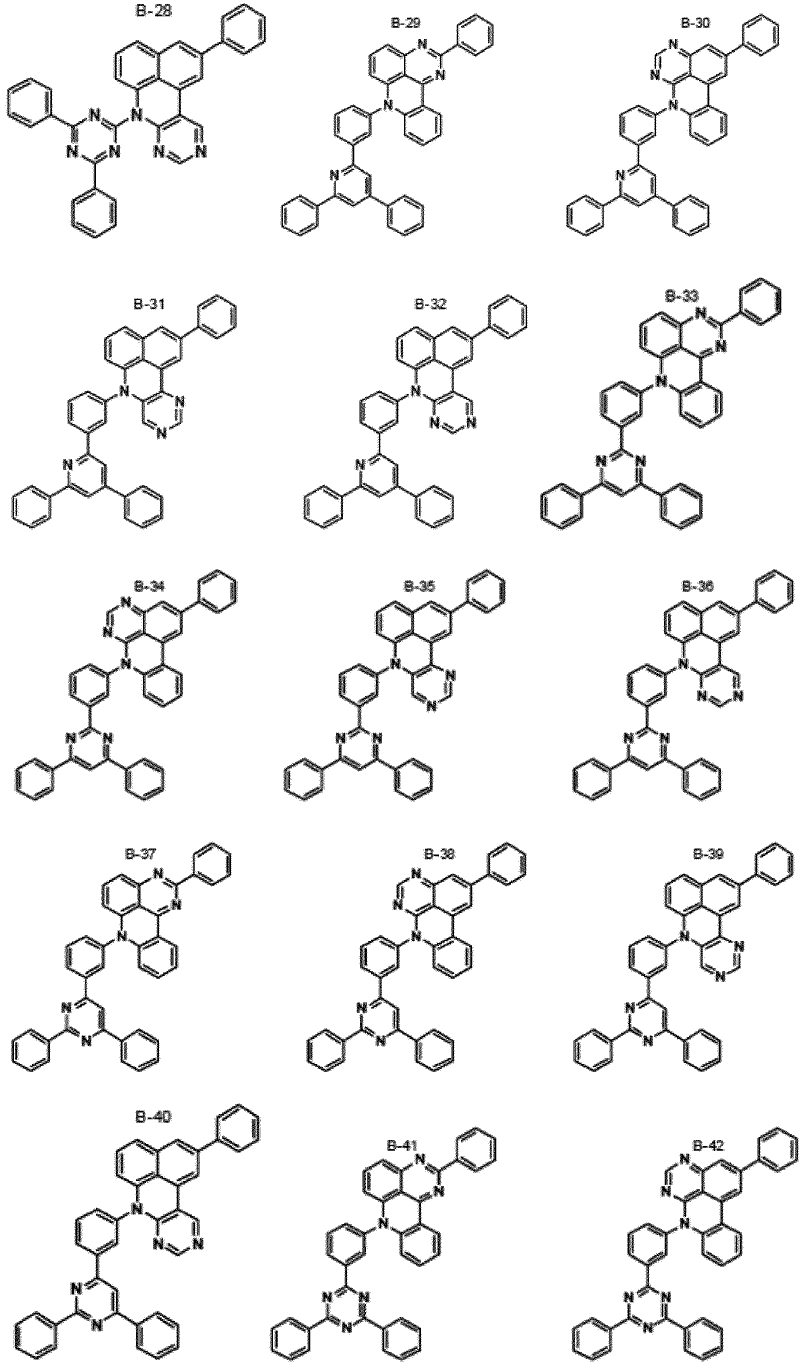

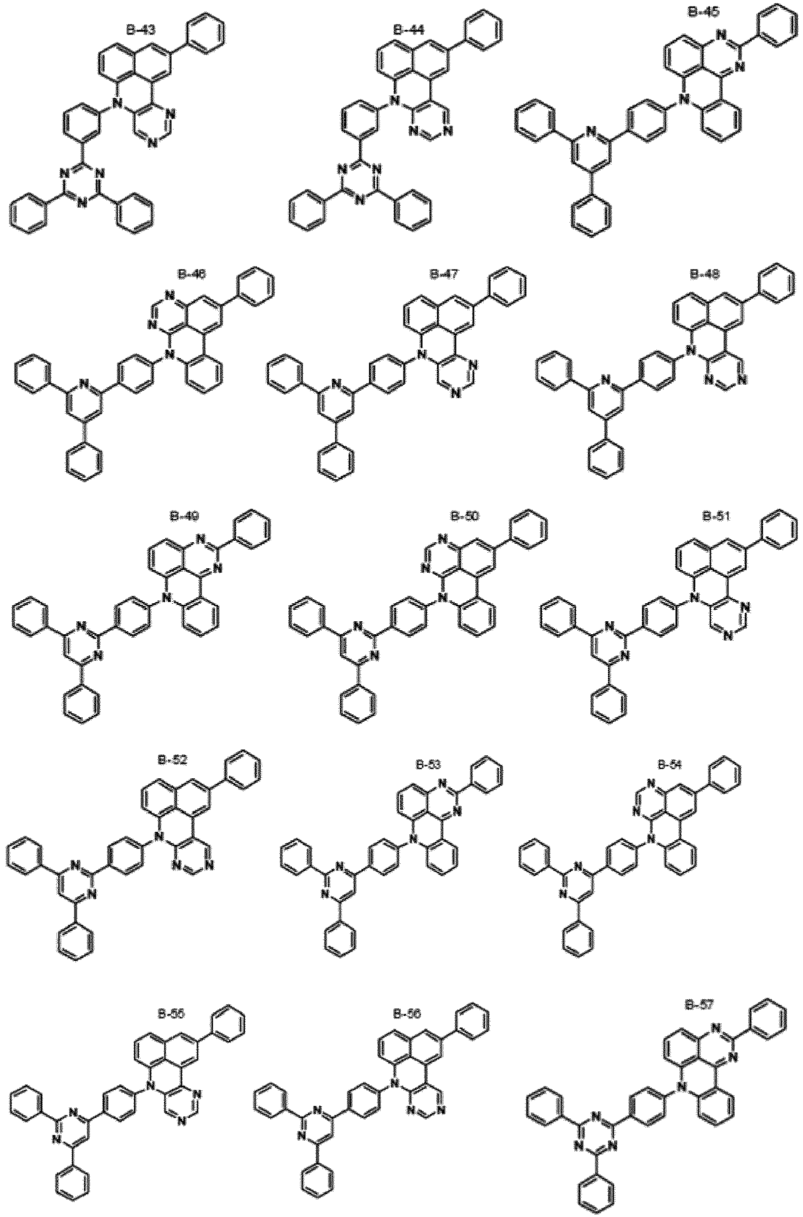

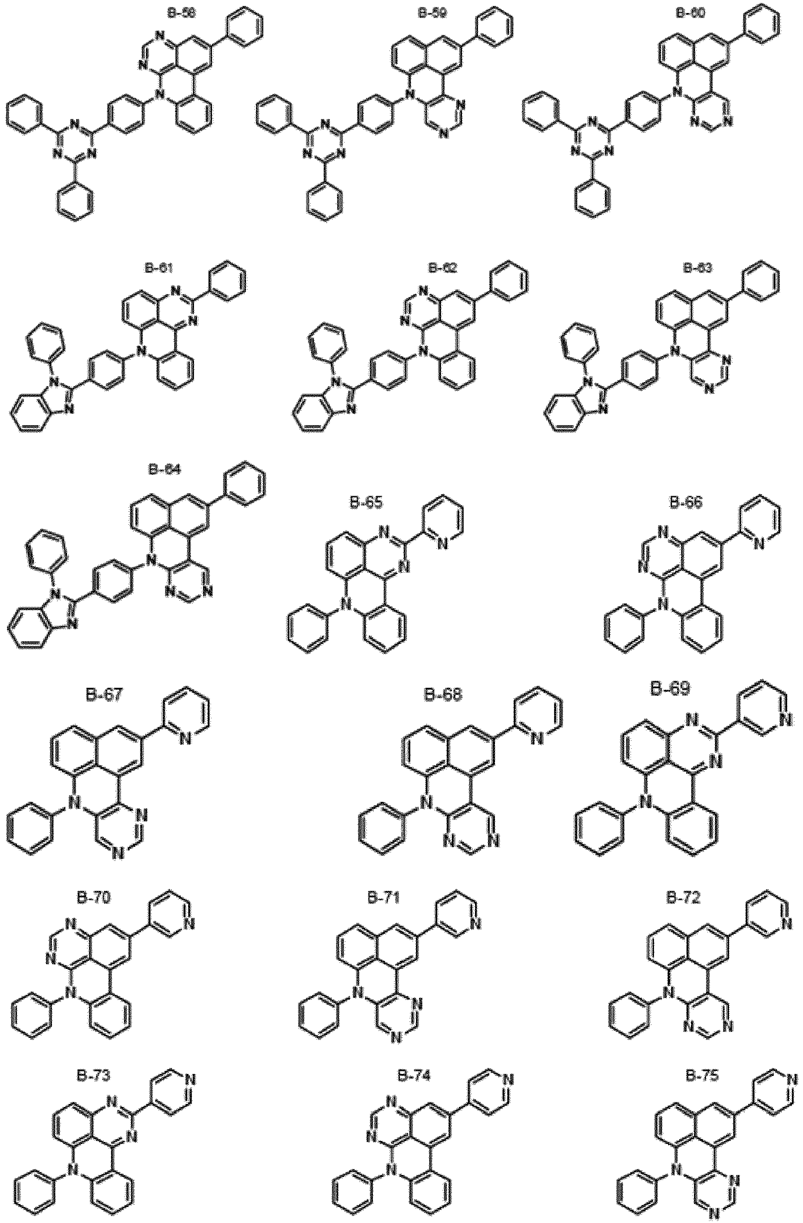

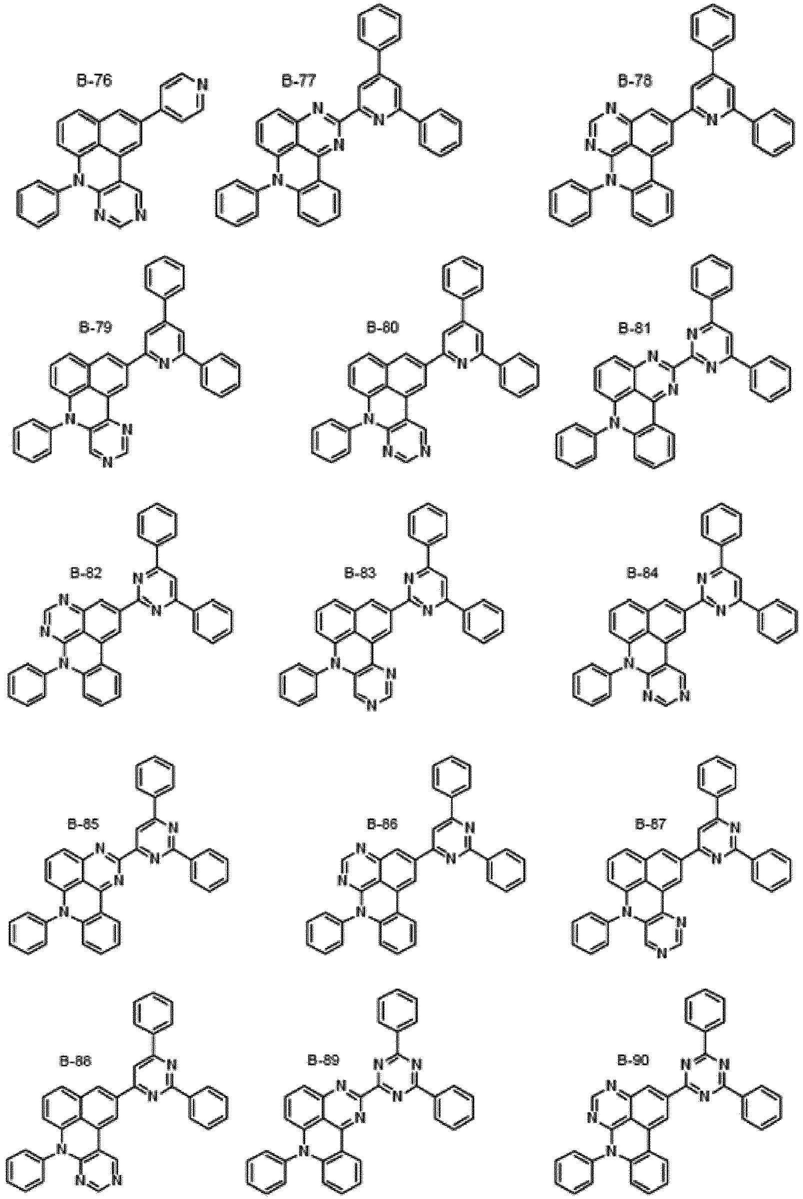

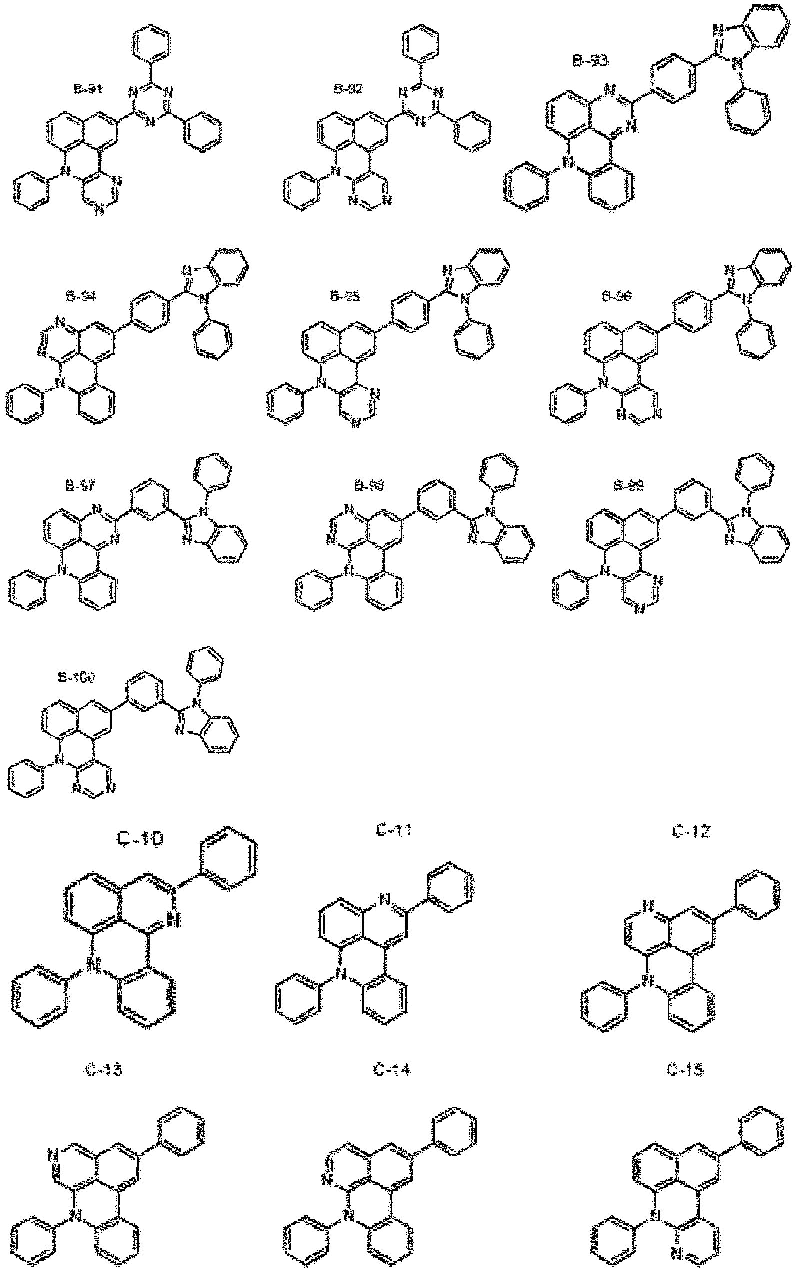

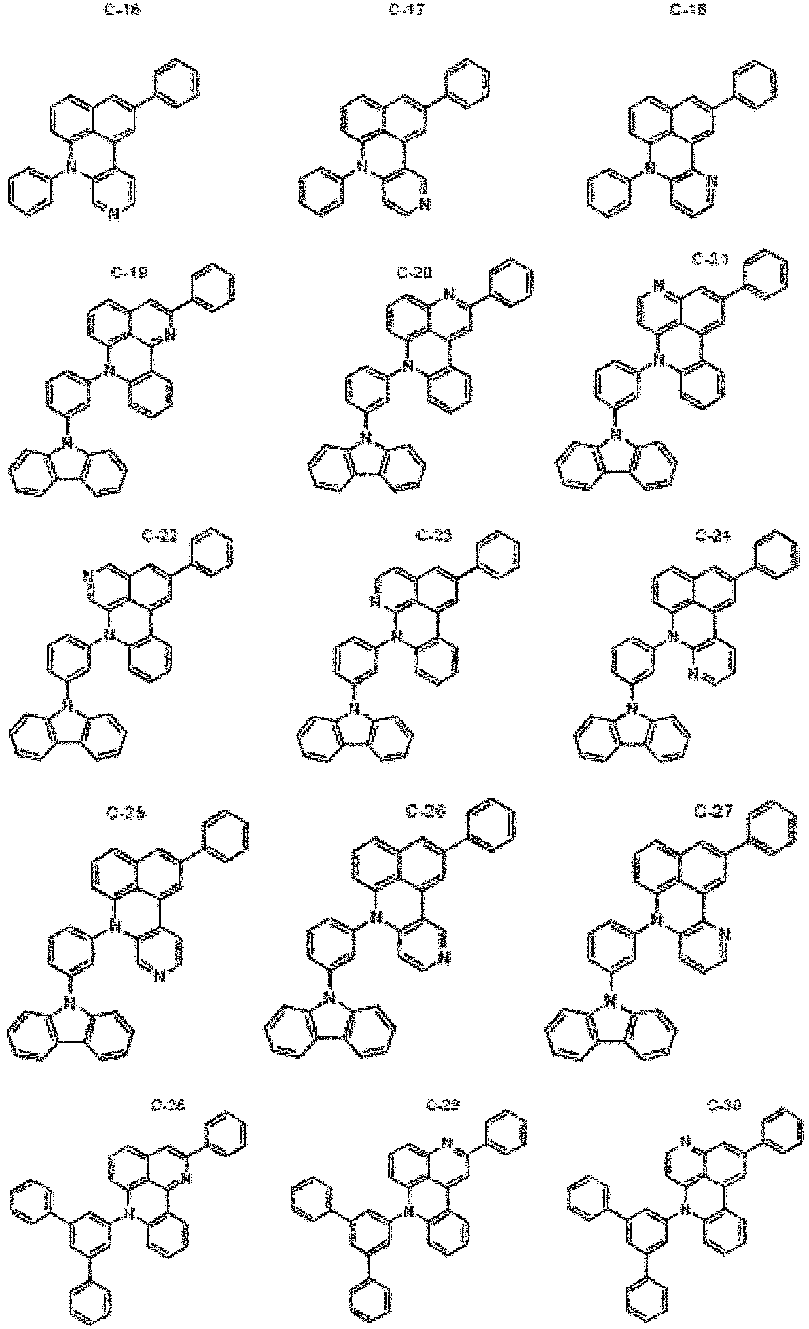

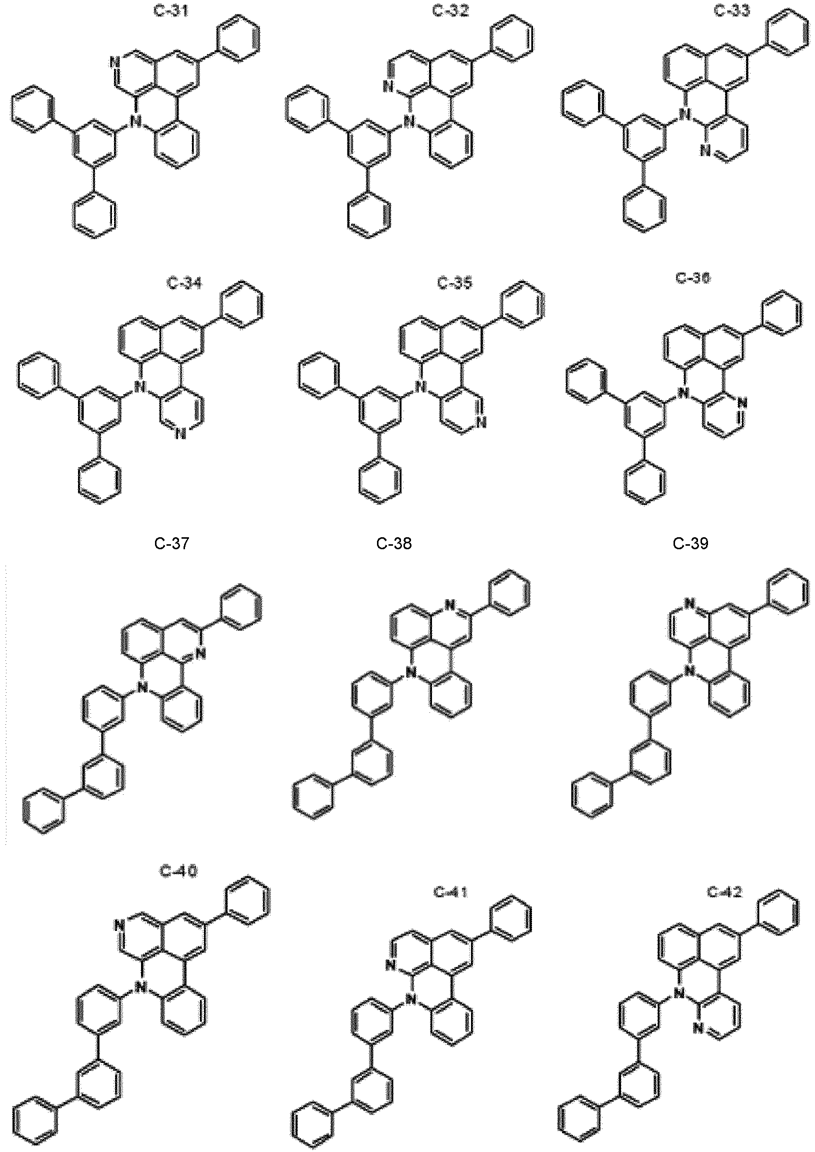

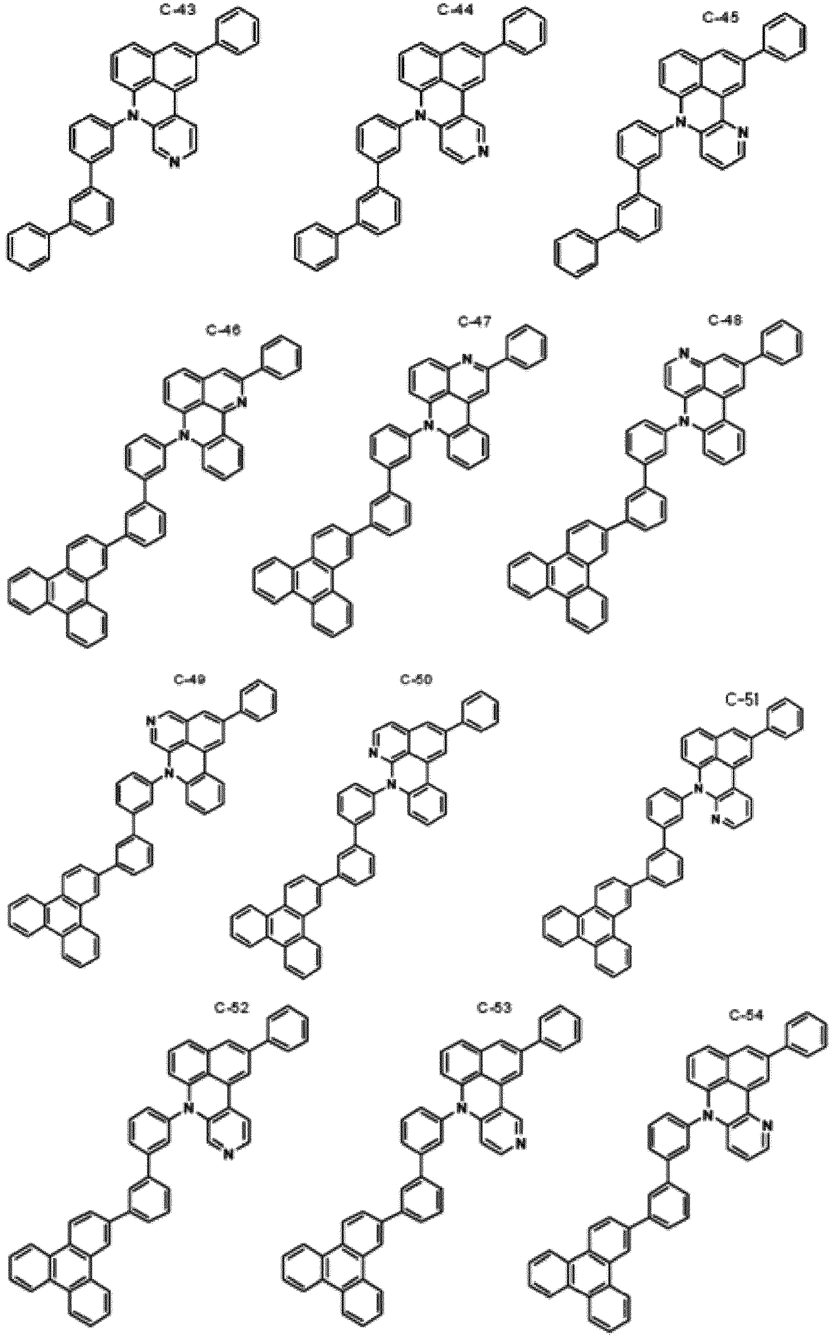

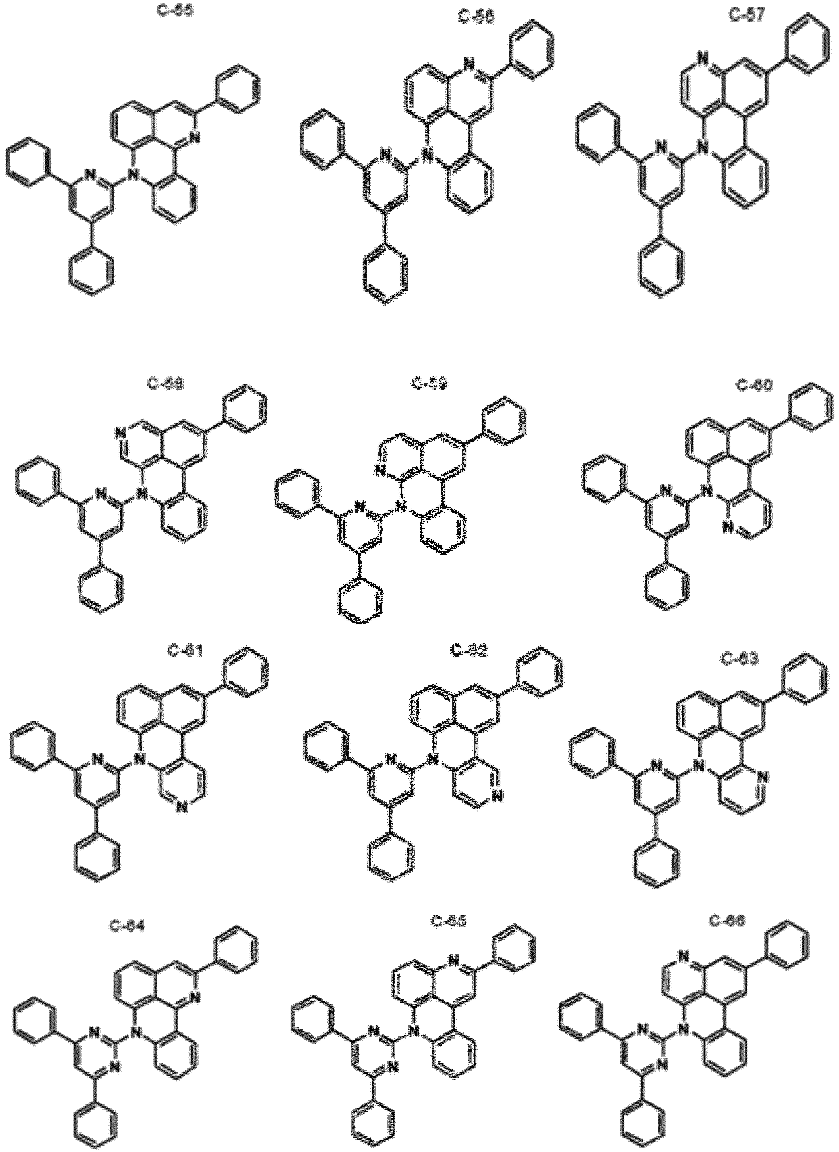

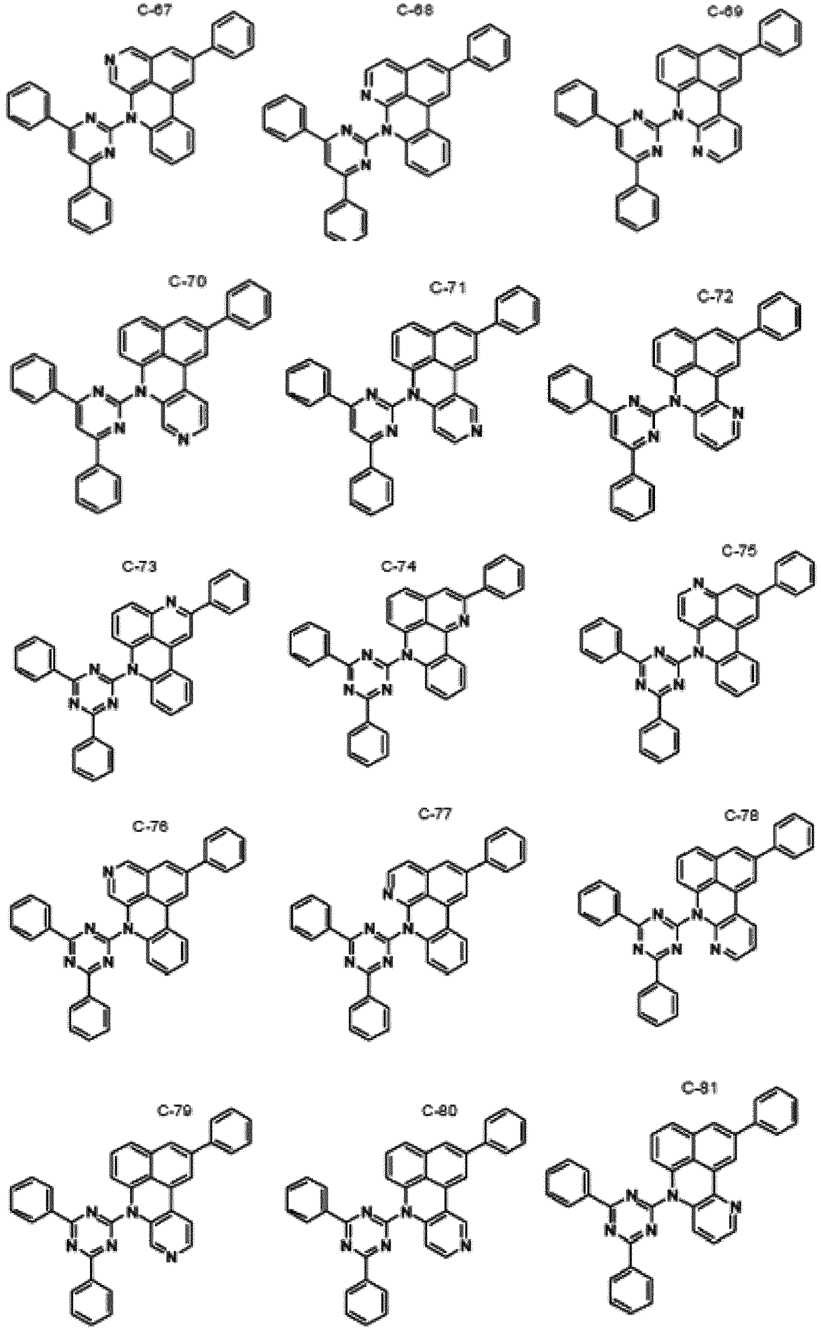

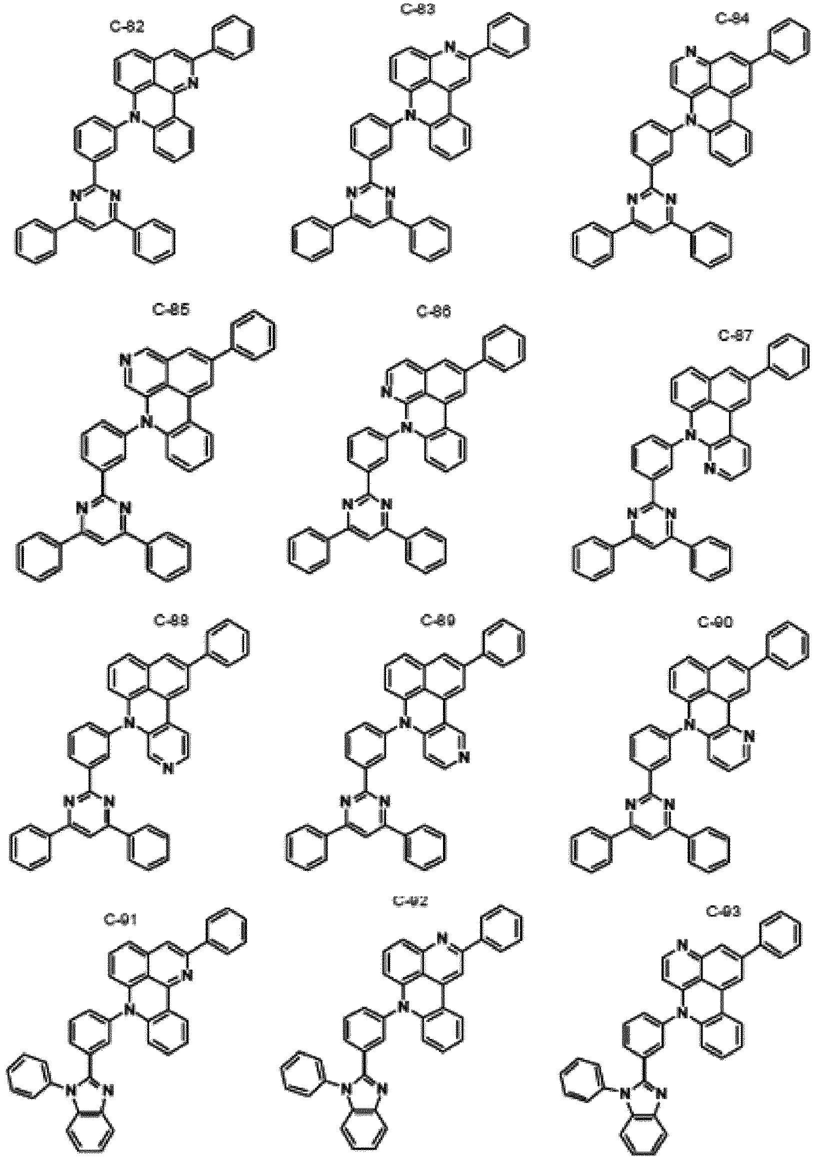

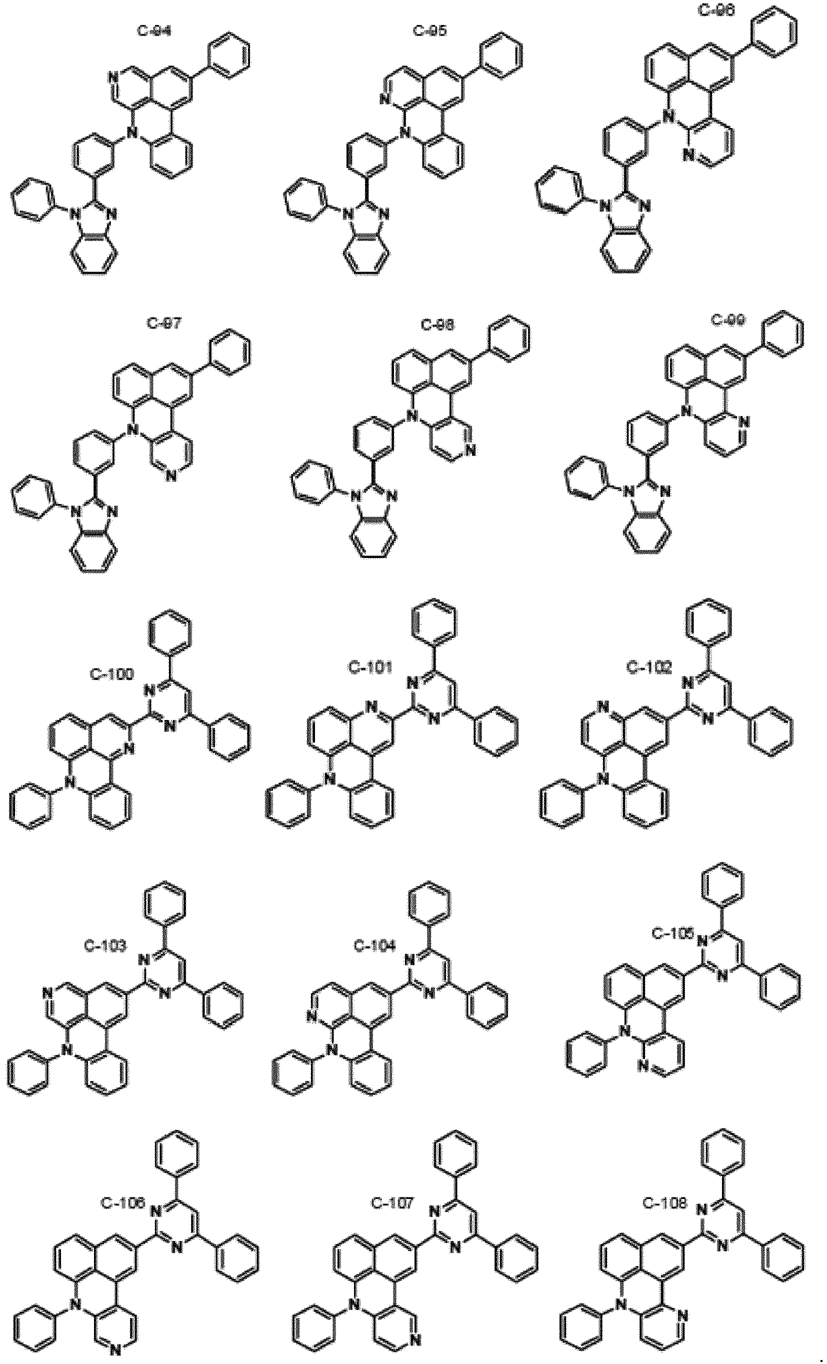

4. Organische optoelektronische Vorrichtung mit:
einer Anode (120) und einer Kathode (110), die einander zugewandt sind; und
mindestens einer zwischen der Anode und der Kathode angeordneten organischen Schicht (130), wobei die organische Schicht die Verbindung nach Anspruch 1 oder 2 aufweist.

5. Organische optoelektronische Vorrichtung nach Anspruch 4, wobei die organische Schicht eine Emissionsschicht aufweist, wobei die Emissionsschicht die Verbindung enthält.

6. Organische optoelektronische Vorrichtung nach Anspruch 5, wobei die Verbindung als ein Wirtsmaterial der Emissionsschicht enthalten ist.

7. Organische optoelektronische Vorrichtung nach Anspruch 4, wobei die organische Schicht mindestens eine Zusatzschicht aufweist, die ausgewählt ist aus einer Lochinjektionsschicht, einer Lochtransportschicht, einer Elektronensperrschicht, einer Elektronentransportschicht, einer Elektroneninjektionsschicht und einer Lochsperrschicht,
wobei die Zusatzschicht die Verbindung aufweist.

8. Organische optoelektronische Vorrichtung mit:
einer Anode (120) und einer Kathode (110), die einander zugewandt sind; und
mindestens einer zwischen der Anode und der Kathode angeordneten organischen Schicht (130), wobei die organische Schicht die Verbindung nach Anspruch 3 aufweist.

9. Anzeigevorrichtung mit der organischen optoelektronischen Vorrichtung nach Anspruch 4.

10. Anzeigevorrichtung mit der organischen optoelektronischen Vorrichtung nach Anspruch 8.

## Revendications

1. Composé représenté par
une des Formules Chimiques 15 à 27, dans lequel le composé est utilisé pour un dispositif optoélectronique organique : 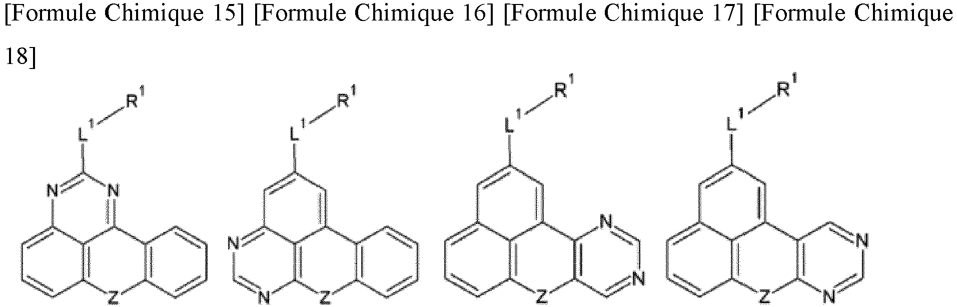 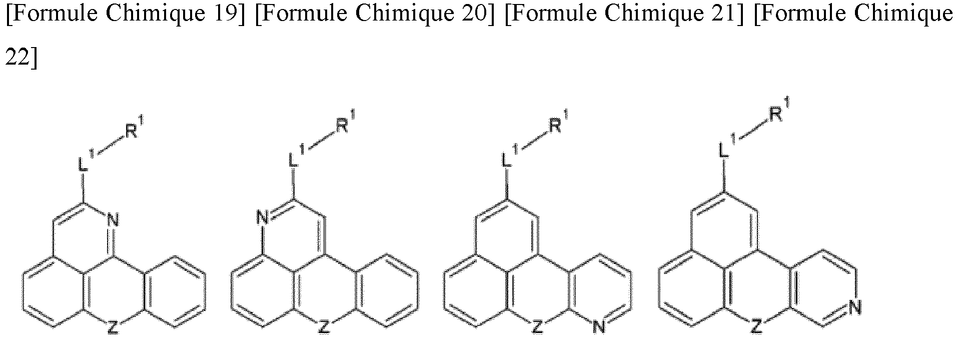 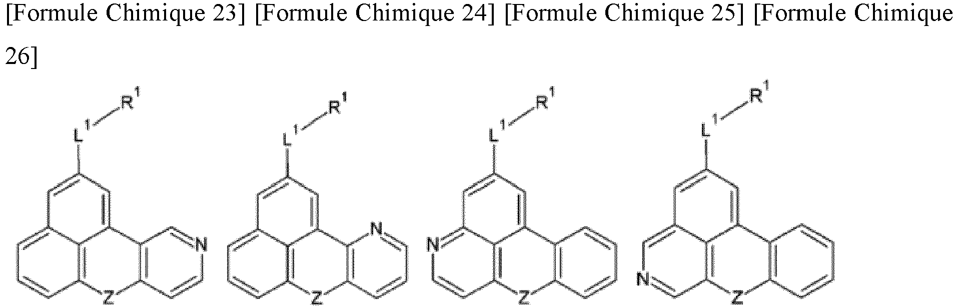 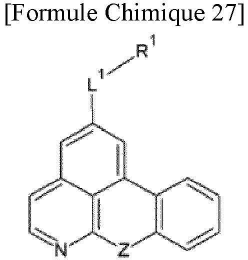
dans lequel, dans les Formules Chimiques 15 à 27,
Z est N-L⁴-R^{b},
L¹ et L⁴ sont indépendamment, une simple liaison, un groupement arylène en C6 à C30 substitué ou non substitué, un groupement hétérocyclique en C2 à C30 substitué ou non substitué, ou une combinaison de ceux-ci, et
dans lequel les R¹ et R^{b} sont indépendamment un groupement sélectionné parmi des groupements lités dans le Groupe II suivant : 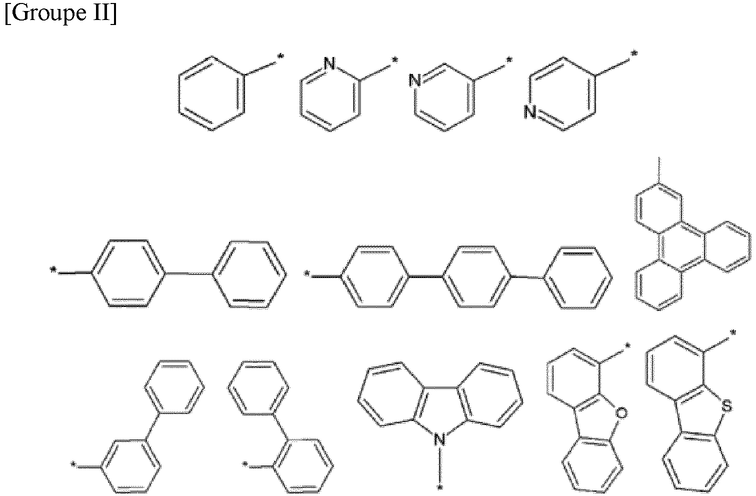 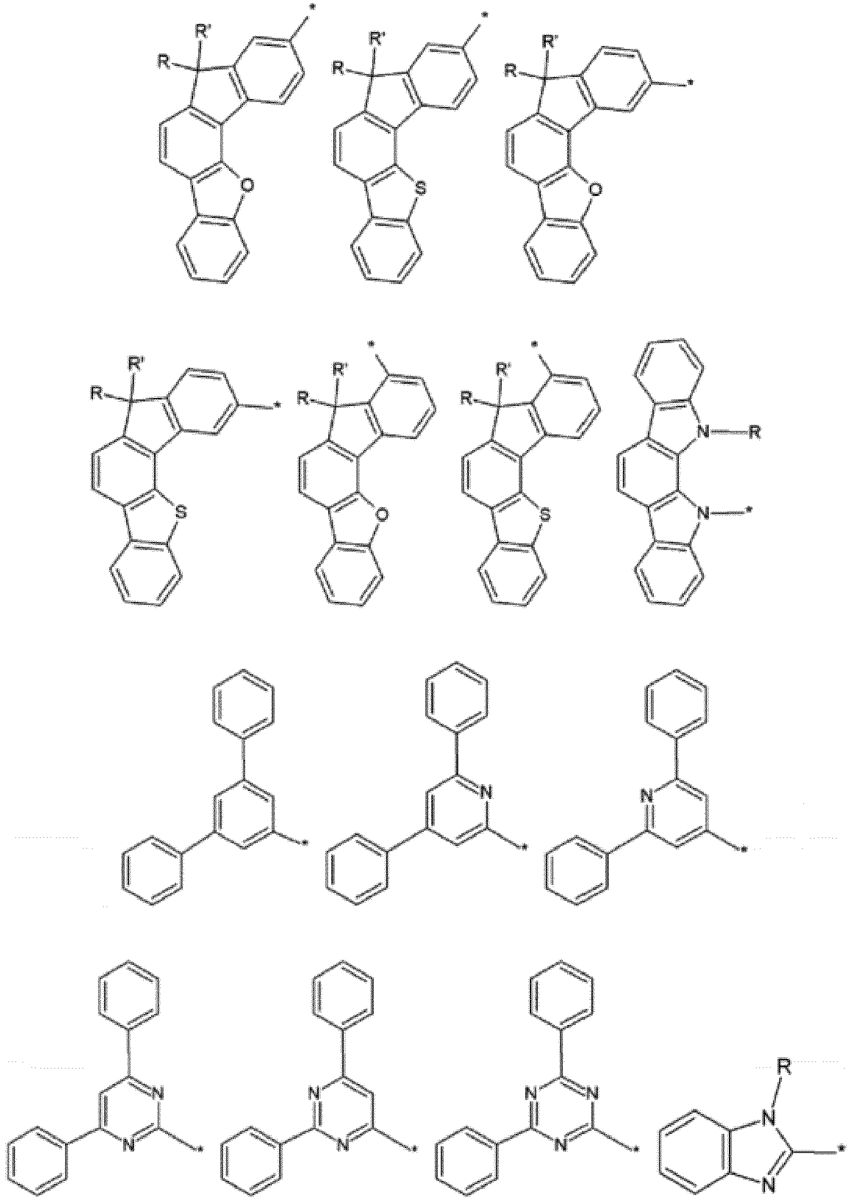
dans lequel, dans le Groupe II,
R et R' sont indépendamment un hydrogène, un deutérium, un groupement alkyle en C1 à C30 substitué ou non substitué, un groupement aryle en C6 à C30 substitué ou non substitué, un groupement hétéroaryle en C2 à C30 substitué ou non substitué, ou une combinaison de ceux-ci, et
* est un point de liaison.

2. Composé selon la revendication 1, dans lequel les L¹ et L⁴ sont indépendamment une simple liaison ou un groupement sélectionné parmi les groupements substitués ou non substitués listés dans le Groupe I suivant : 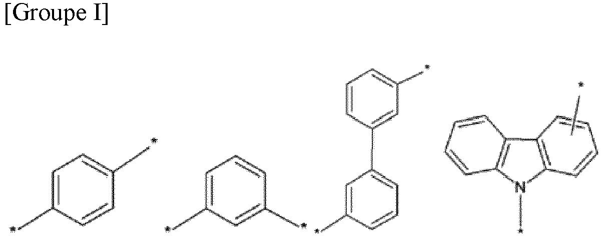
dans lequel, dans le Groupe I,
* indique un point de liaison.

3. Composé représenté par l'une des composés suivants listés dans le Groupe III suivant :

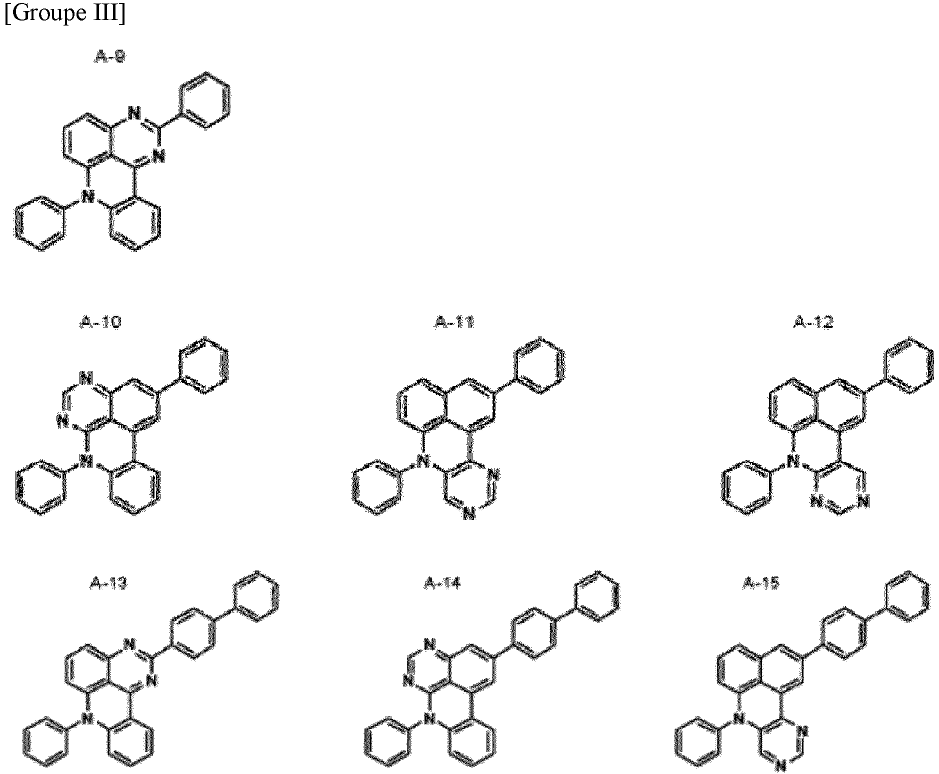

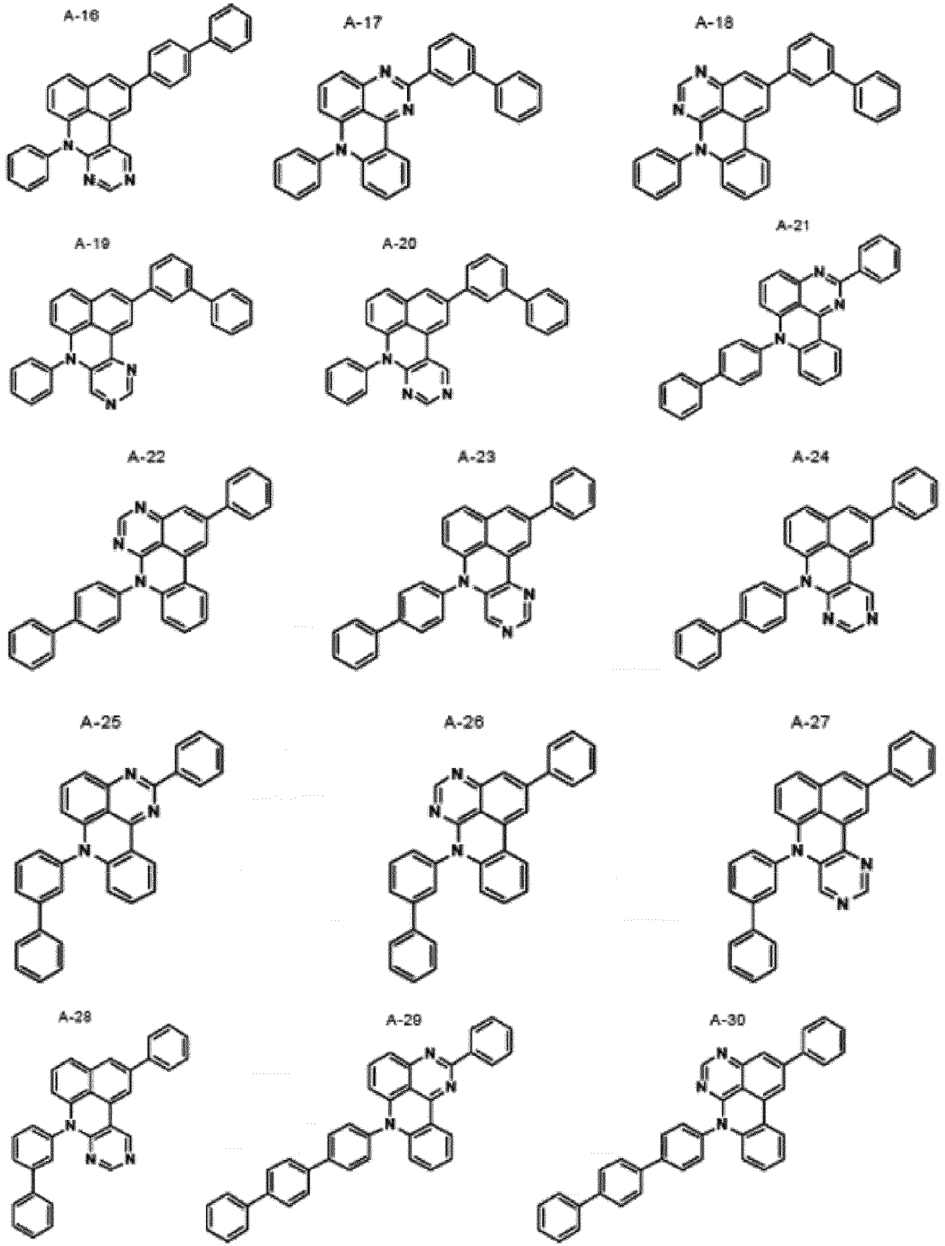

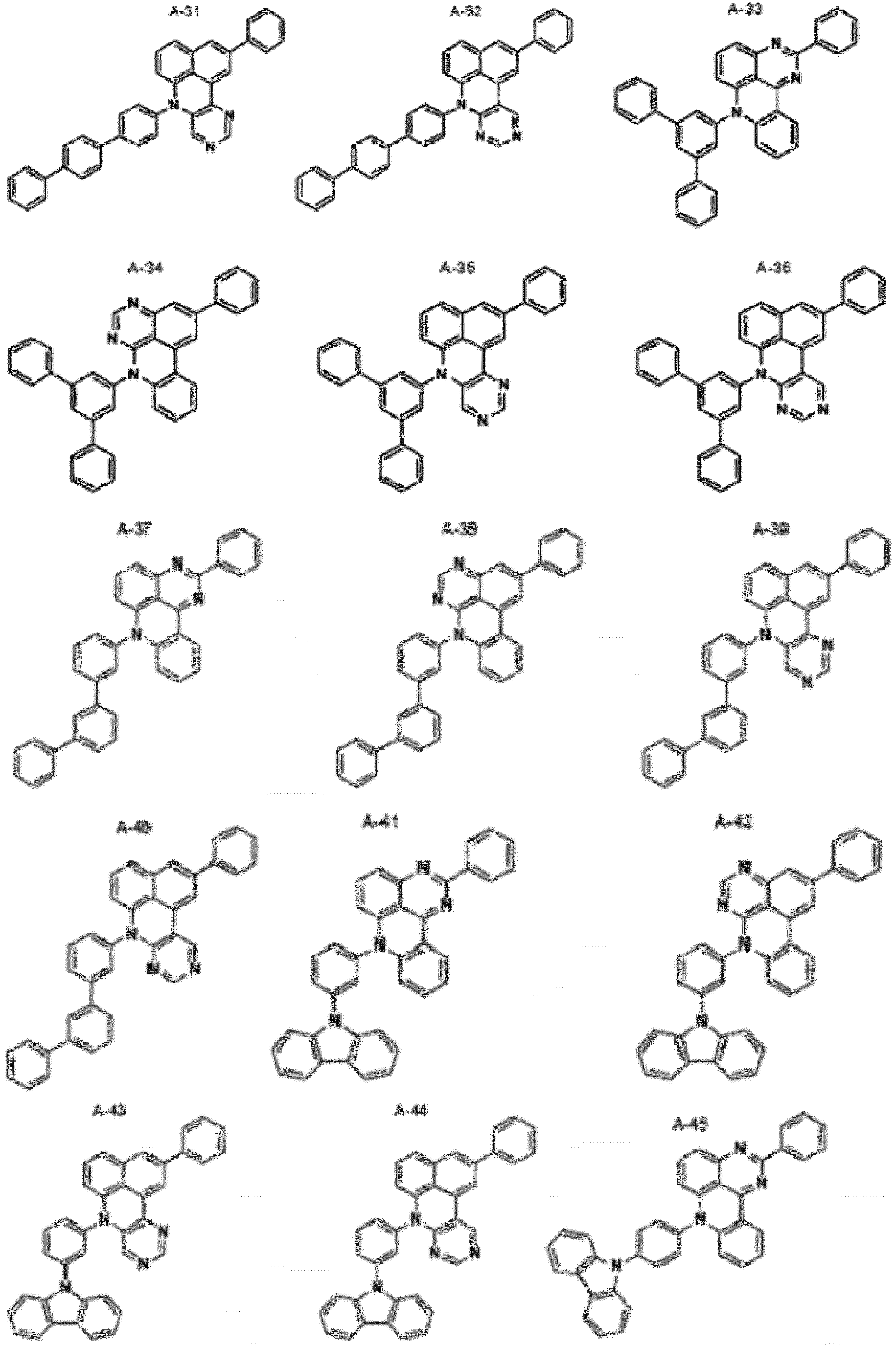

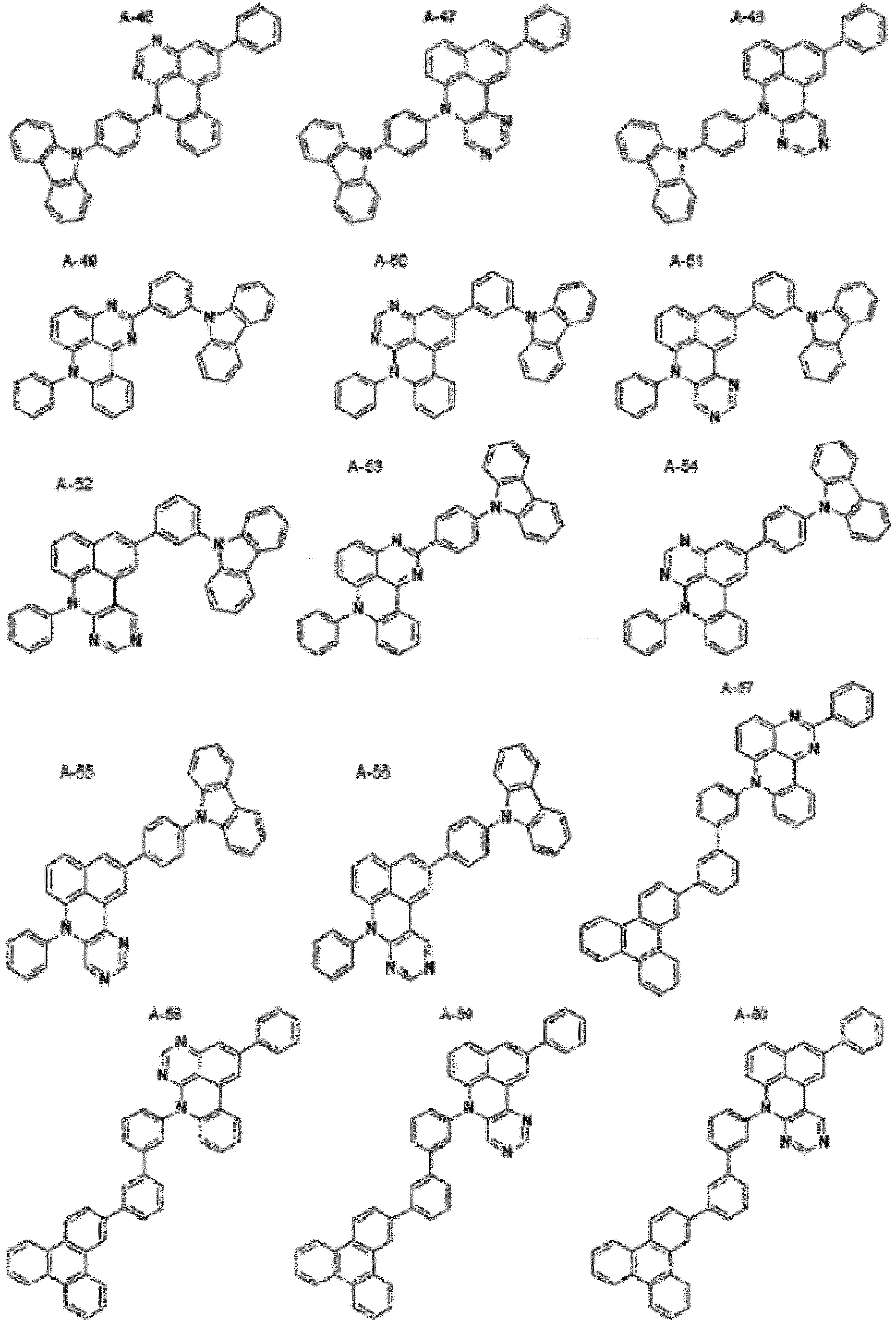

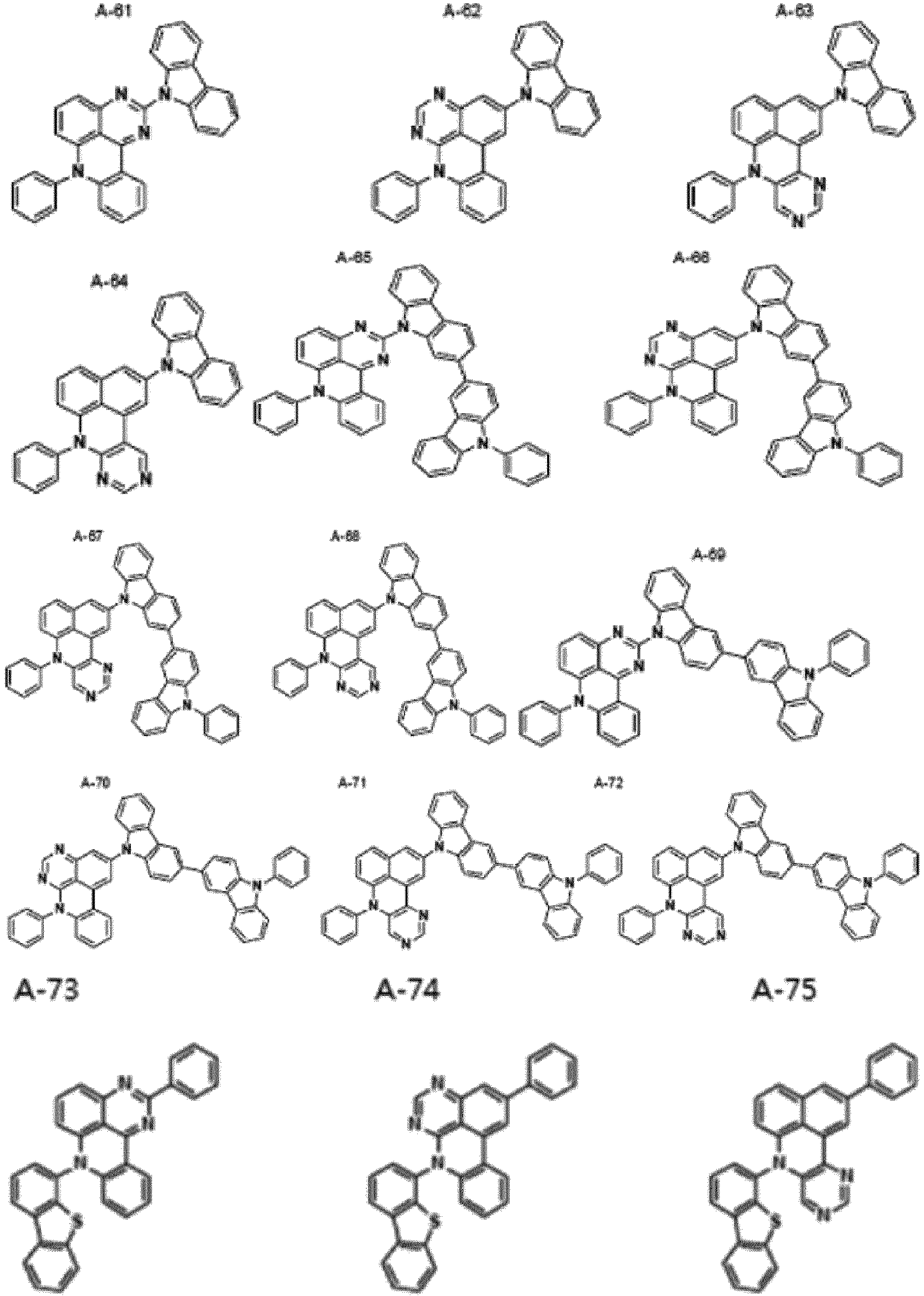

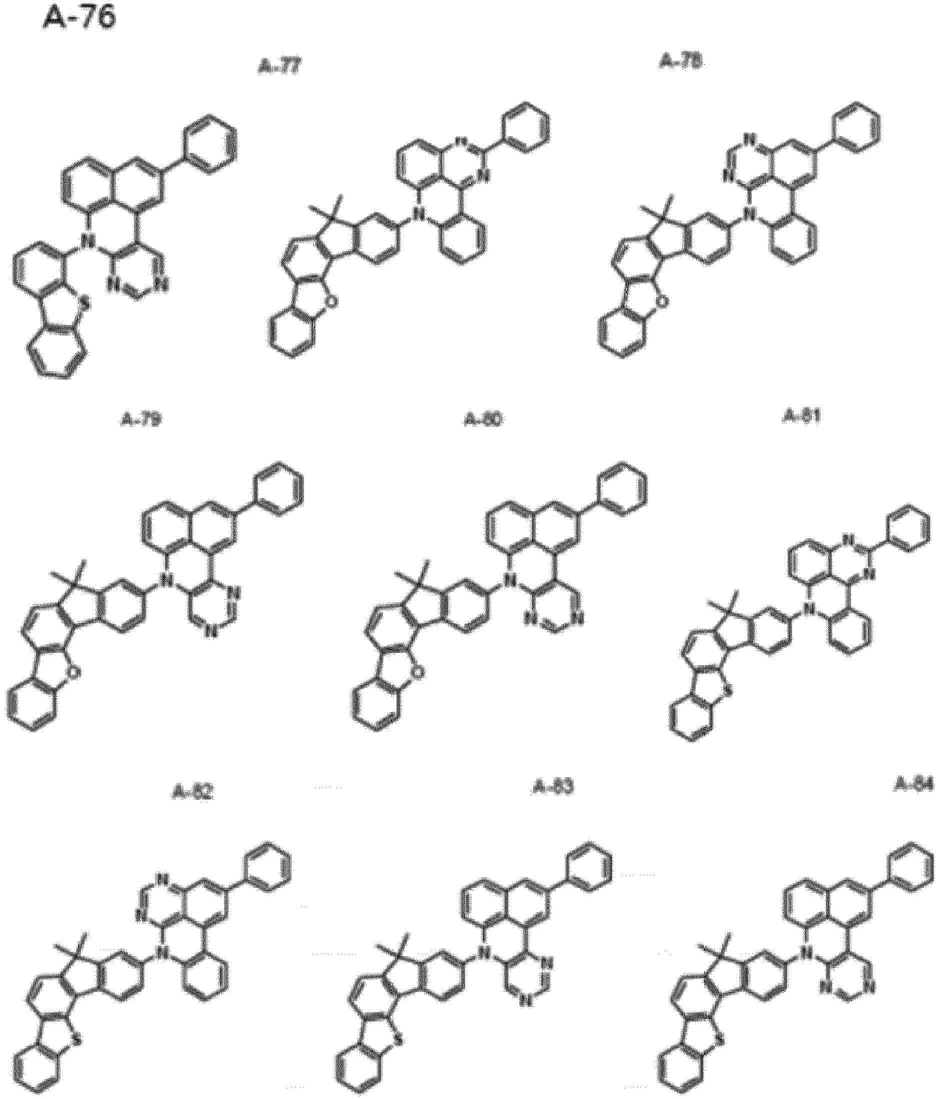

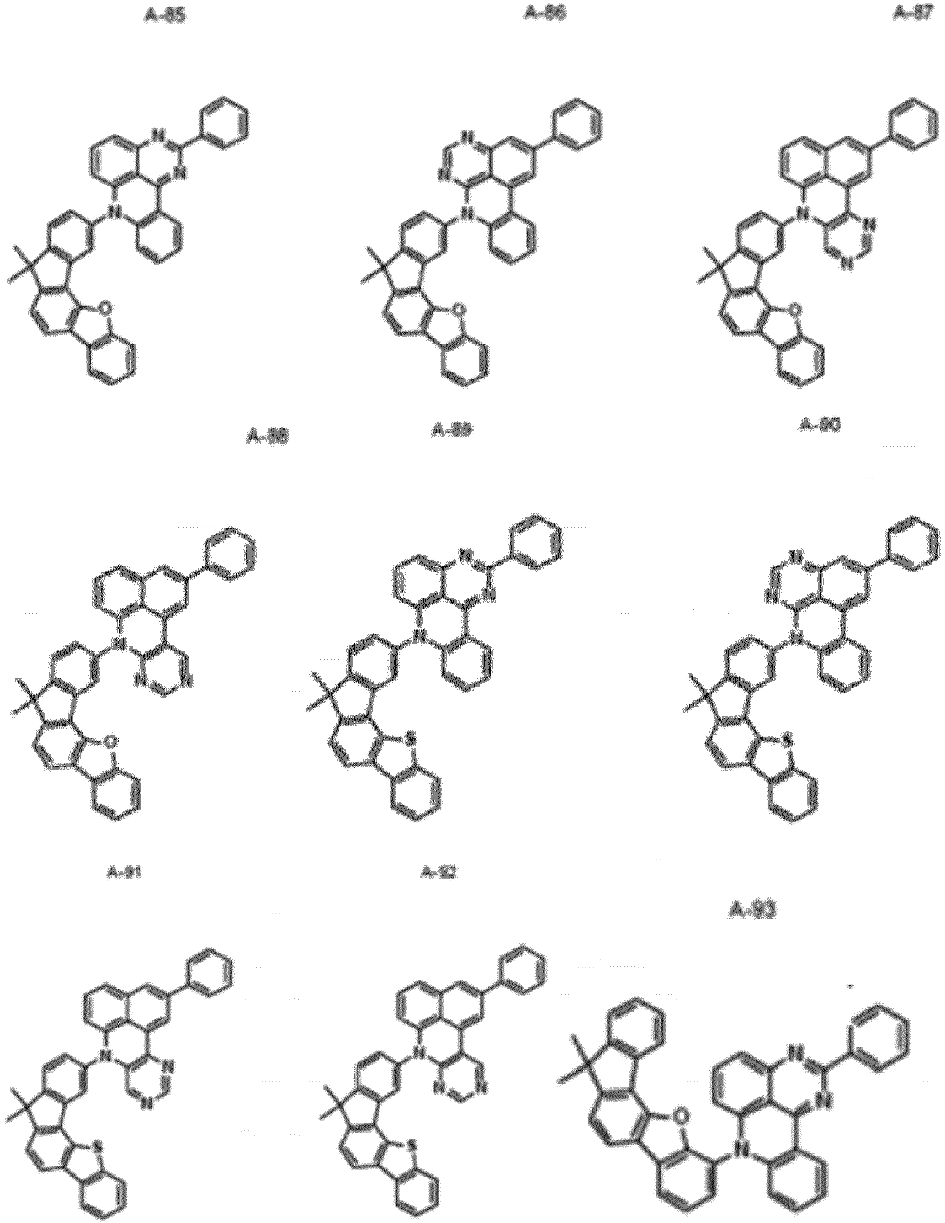

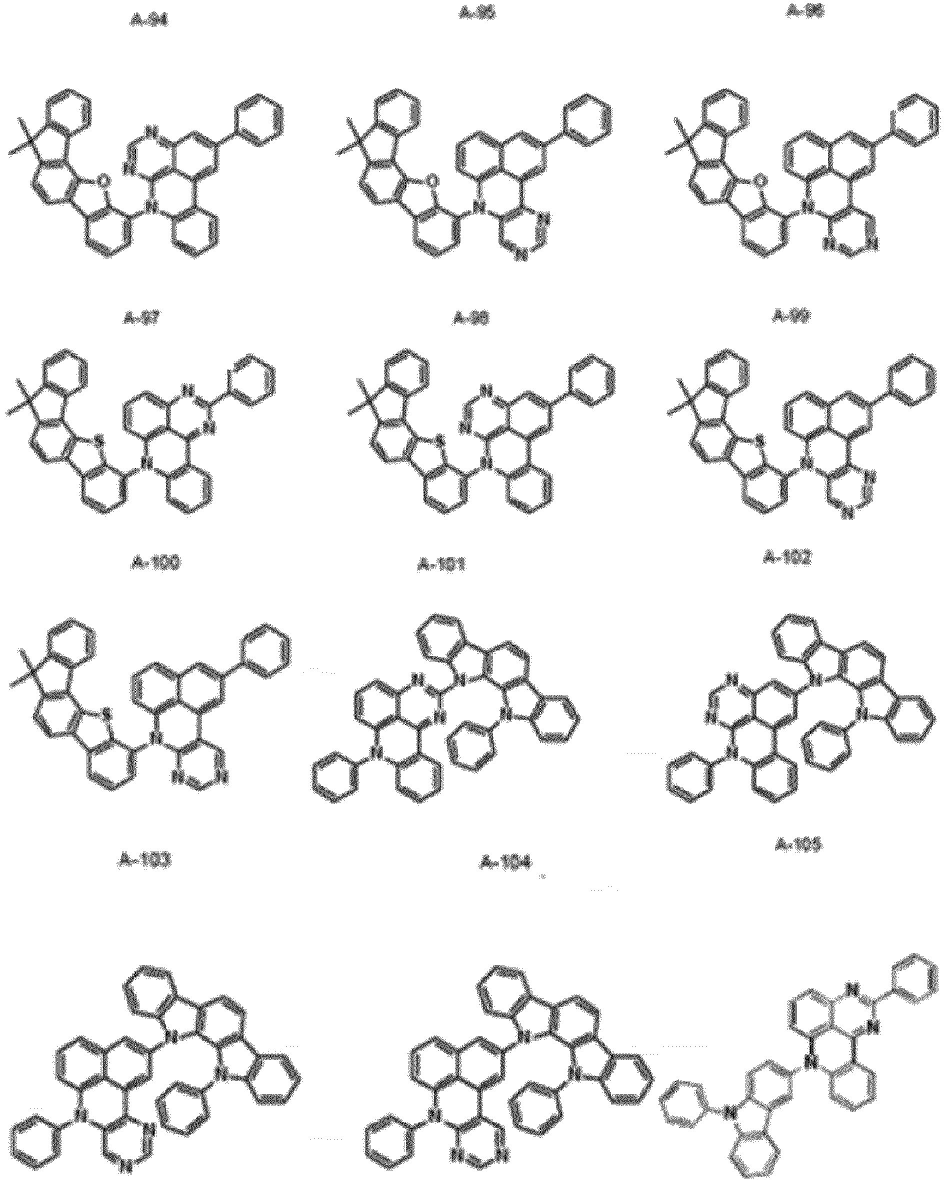

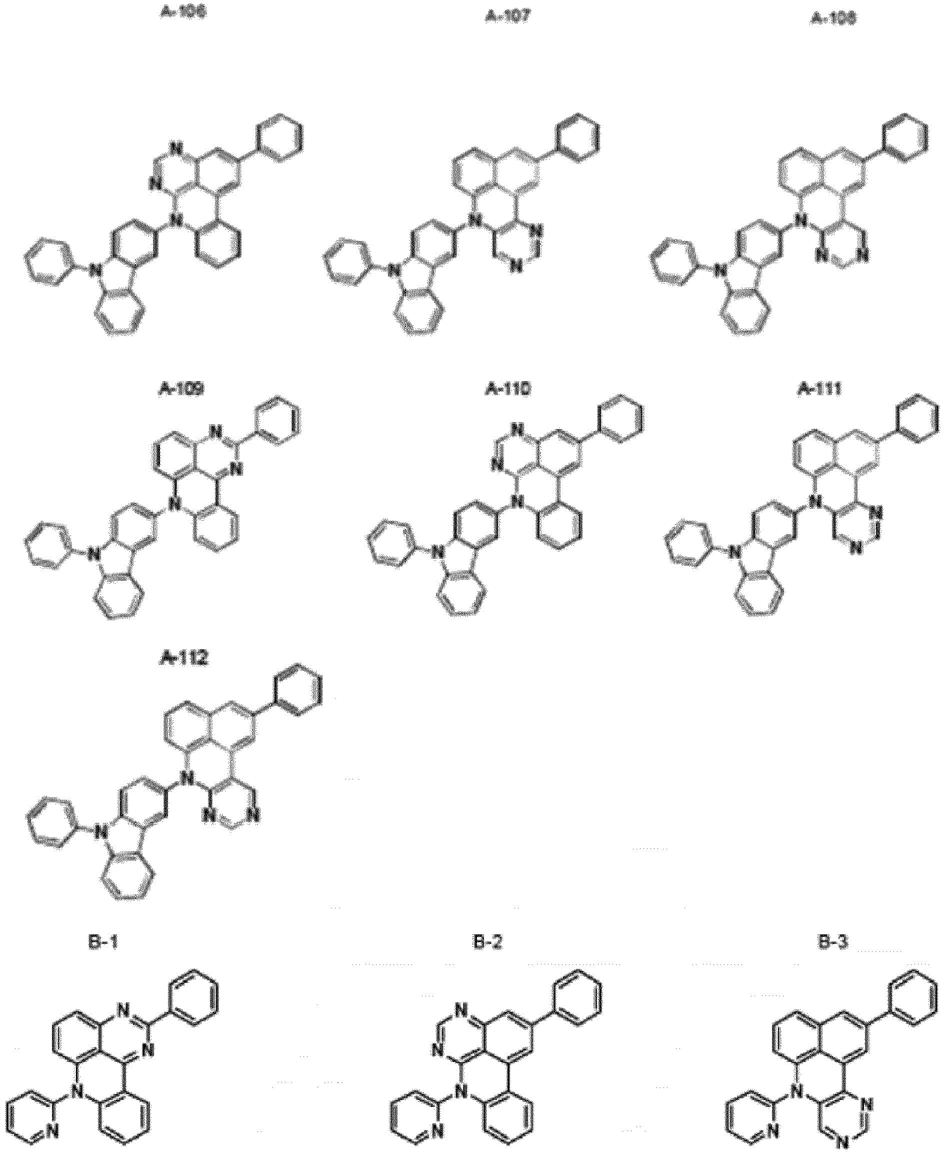

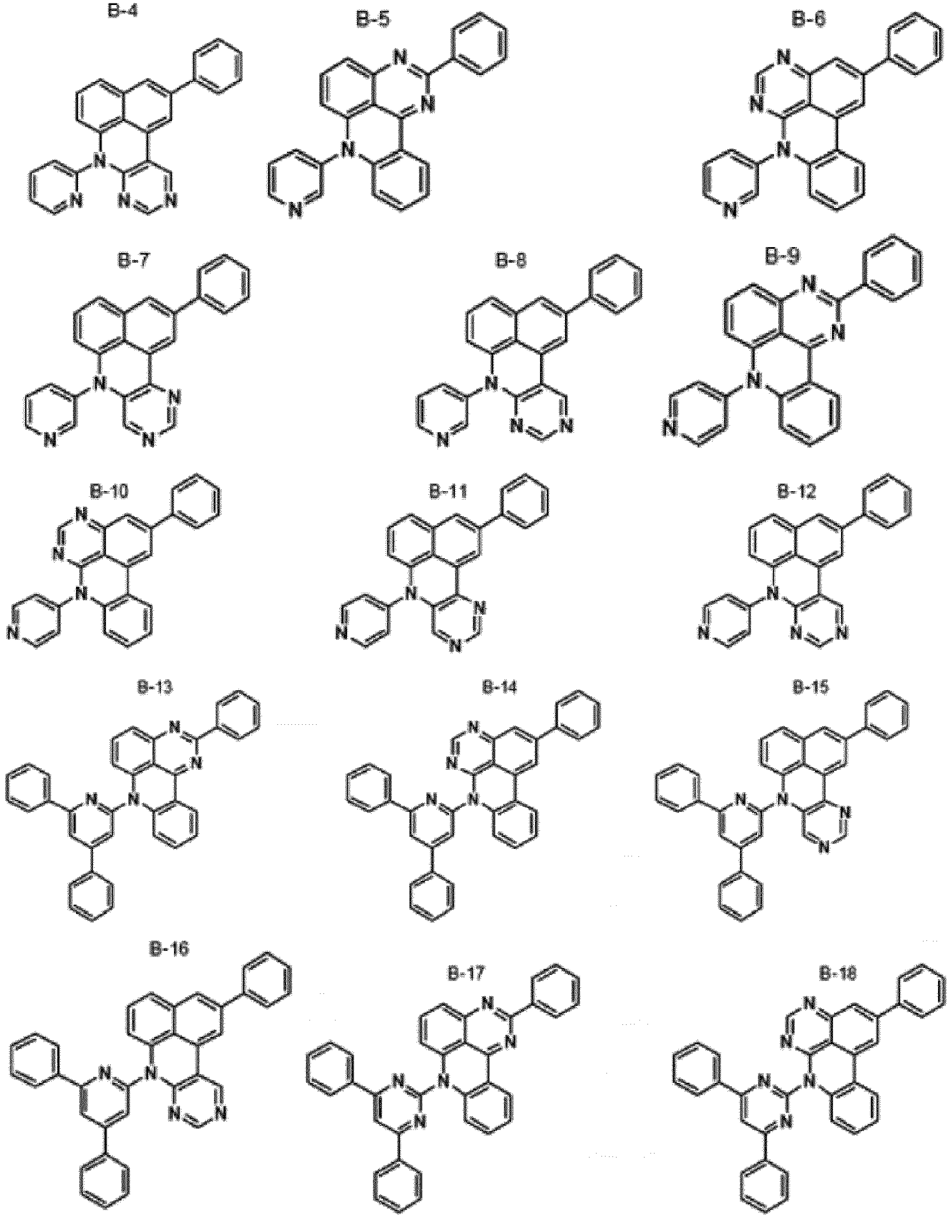

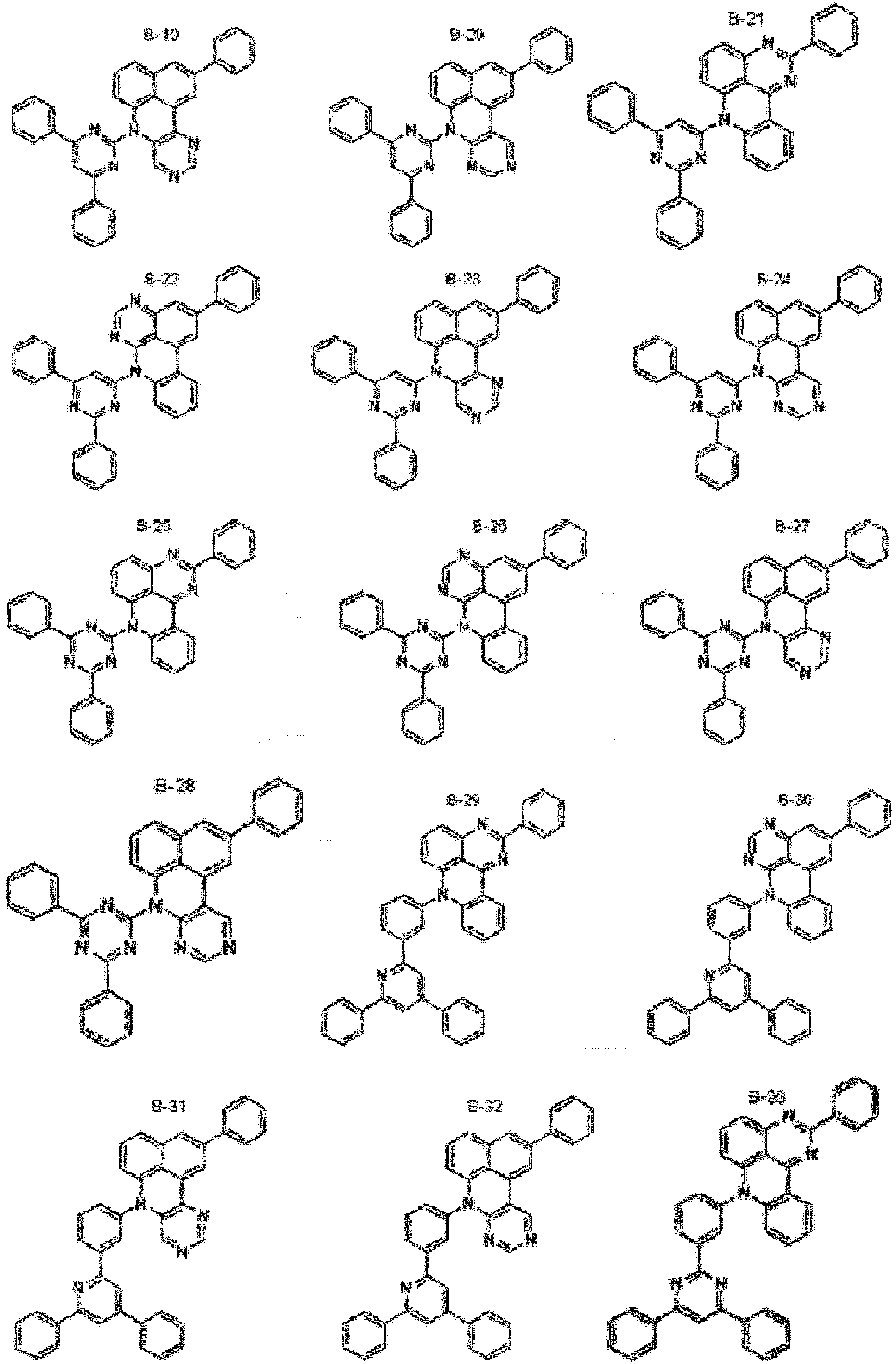

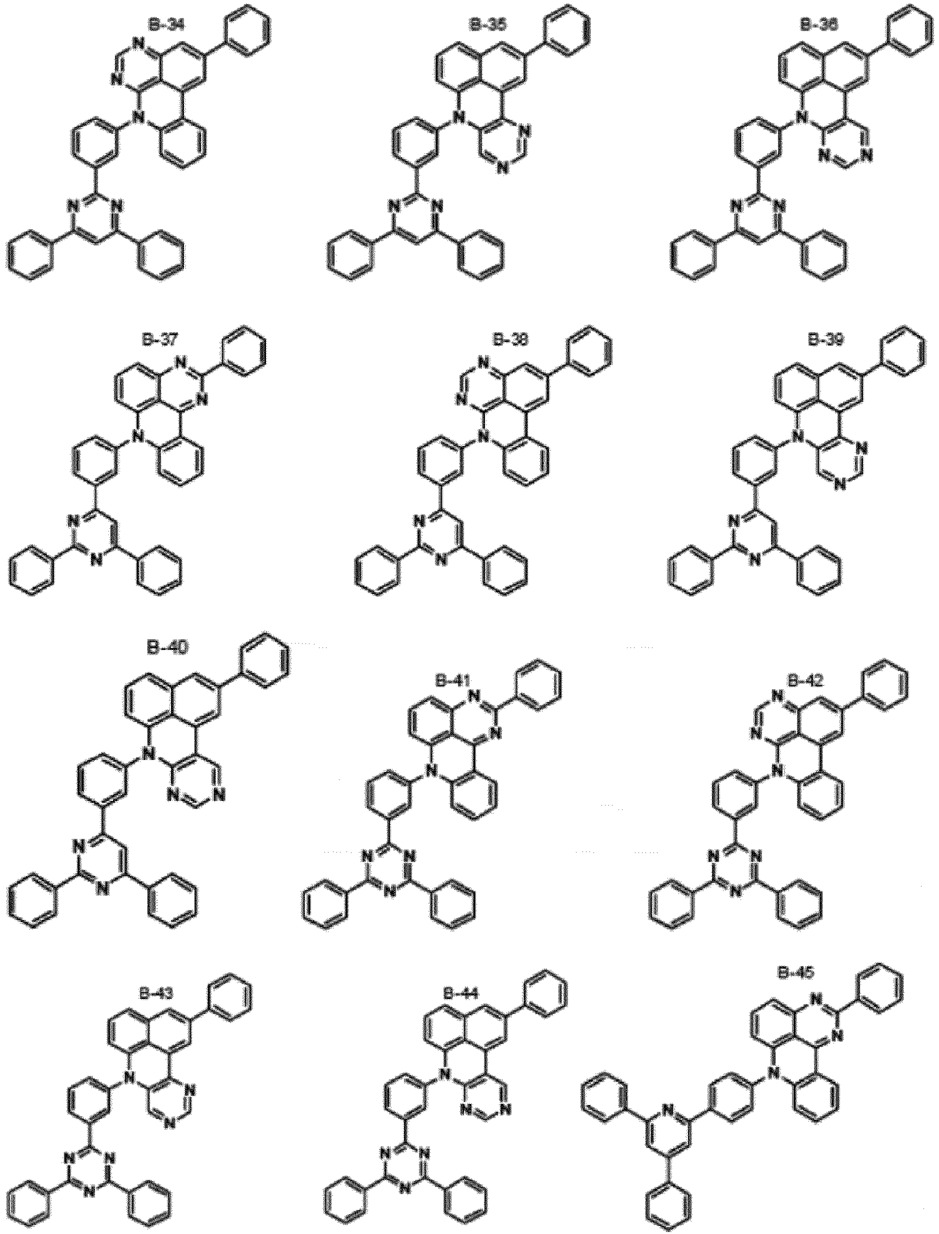

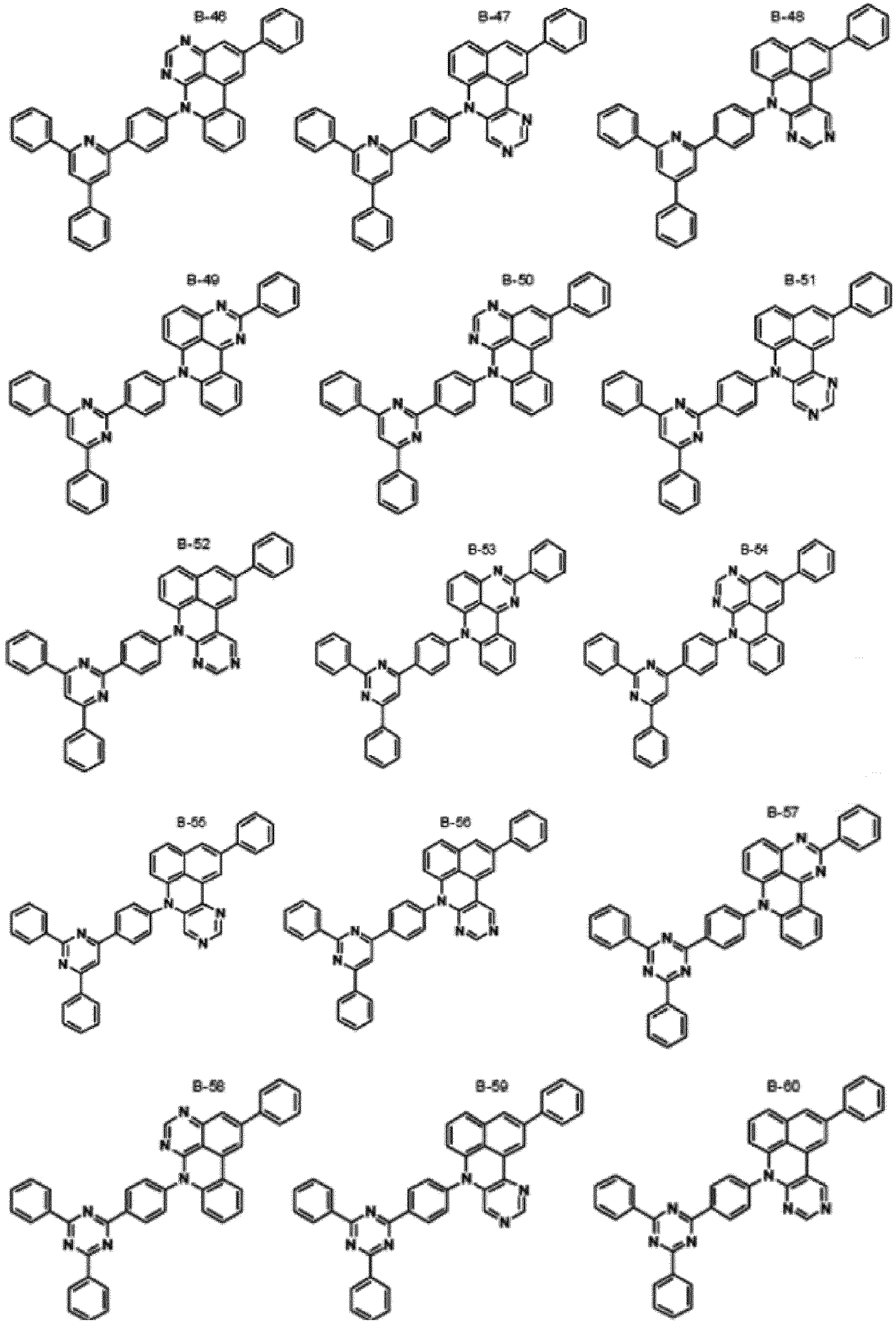

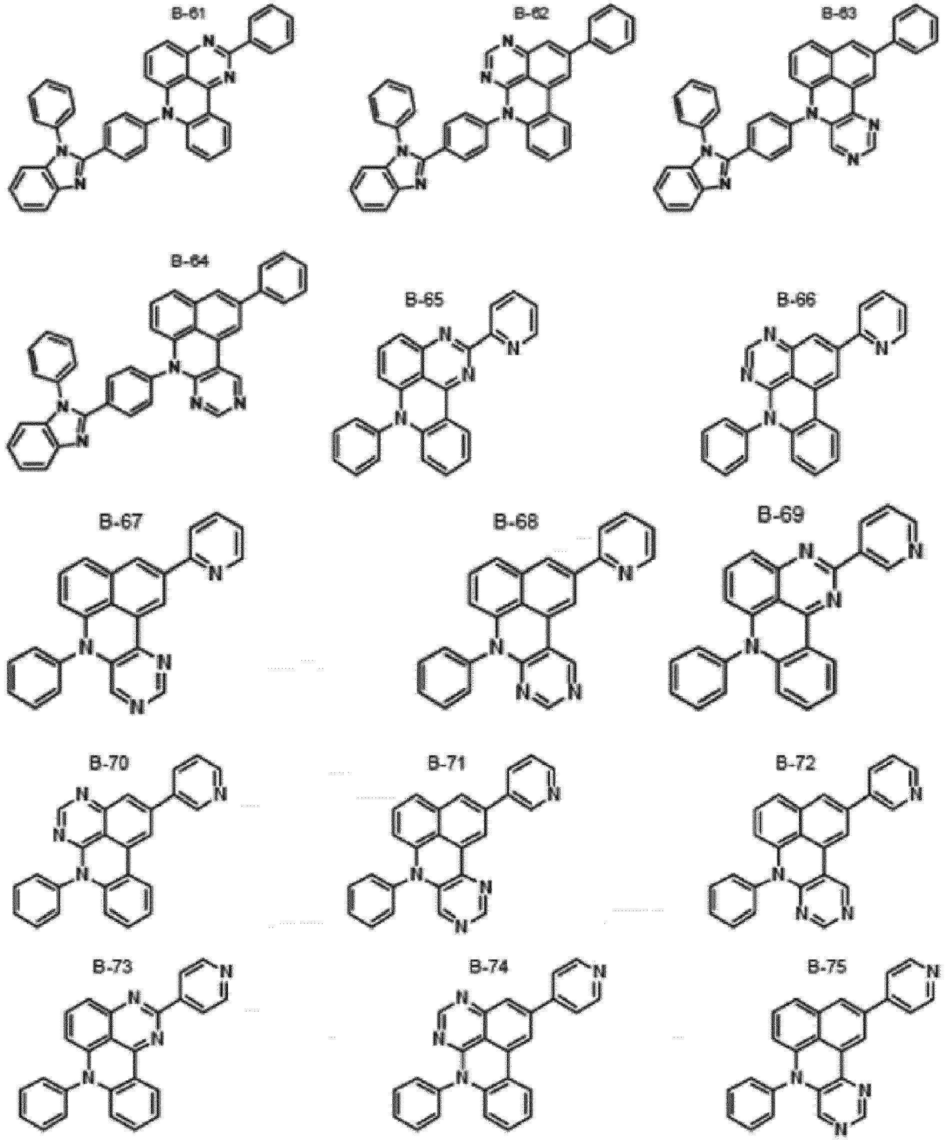

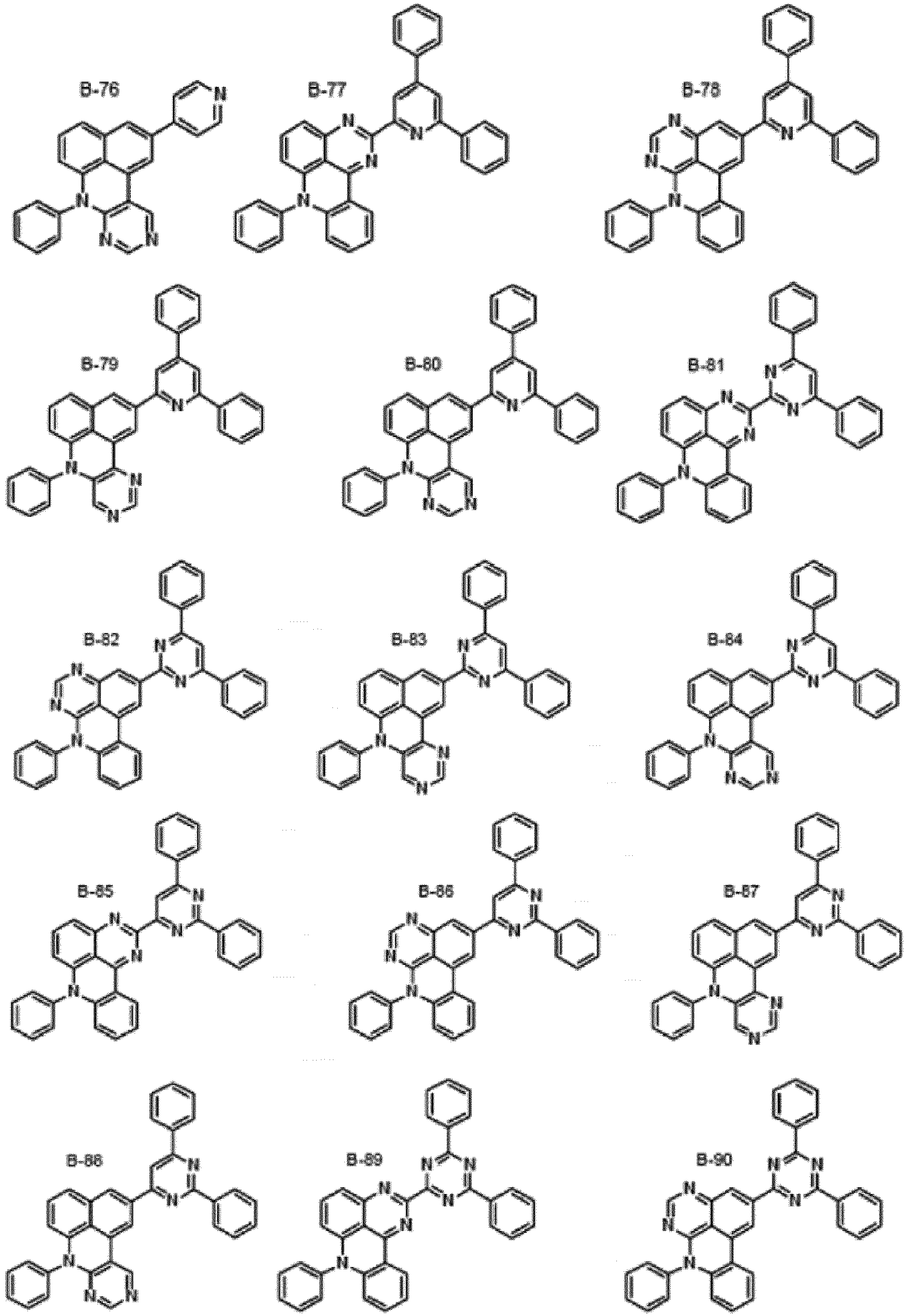

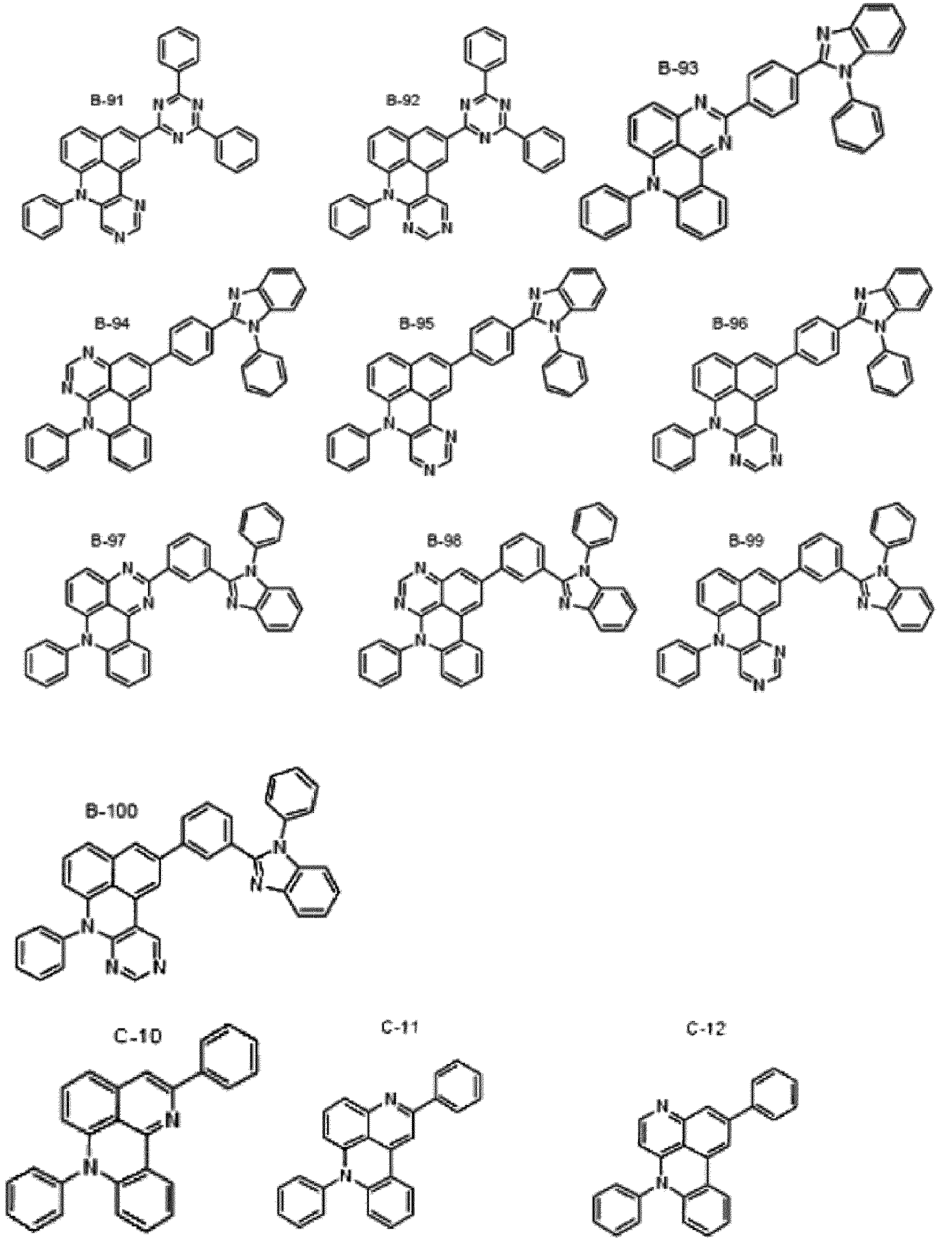

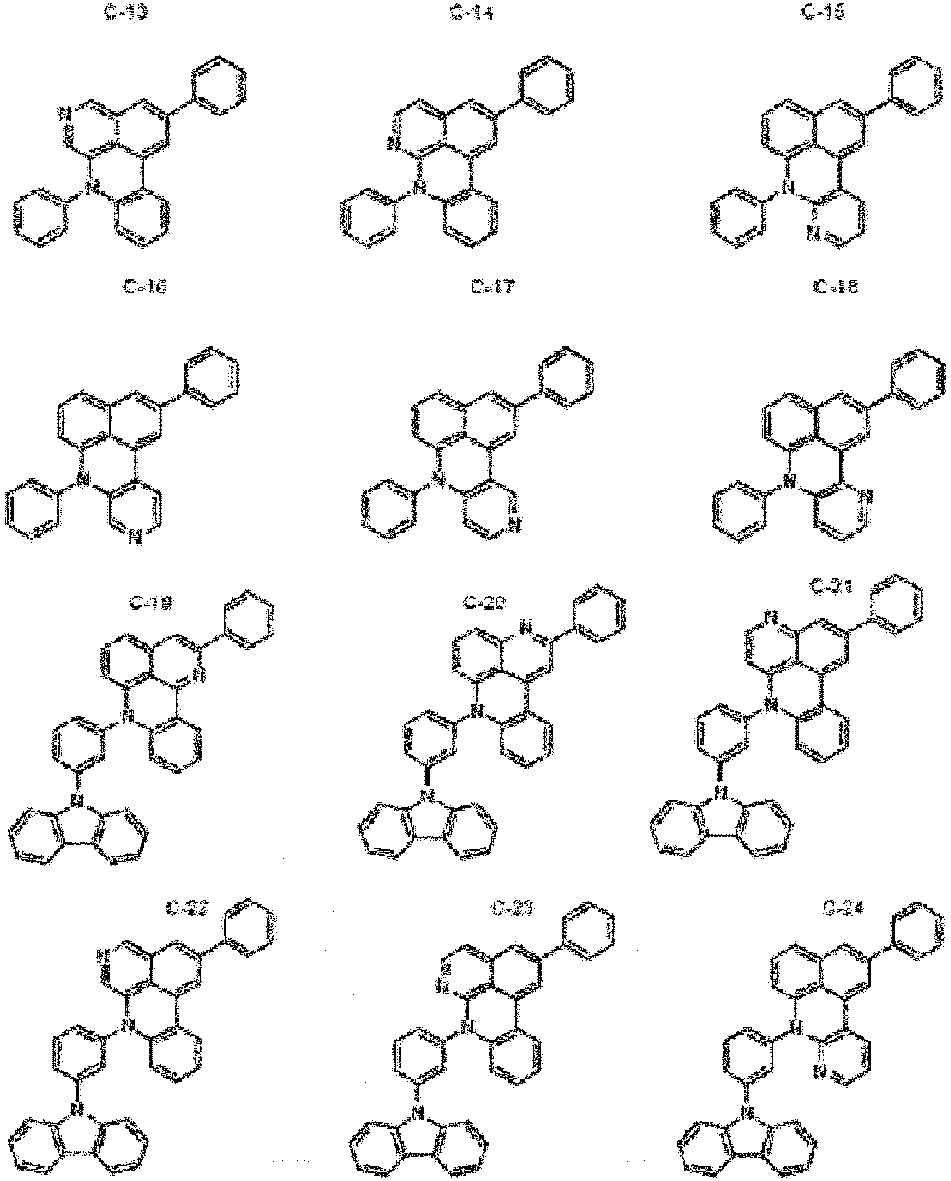

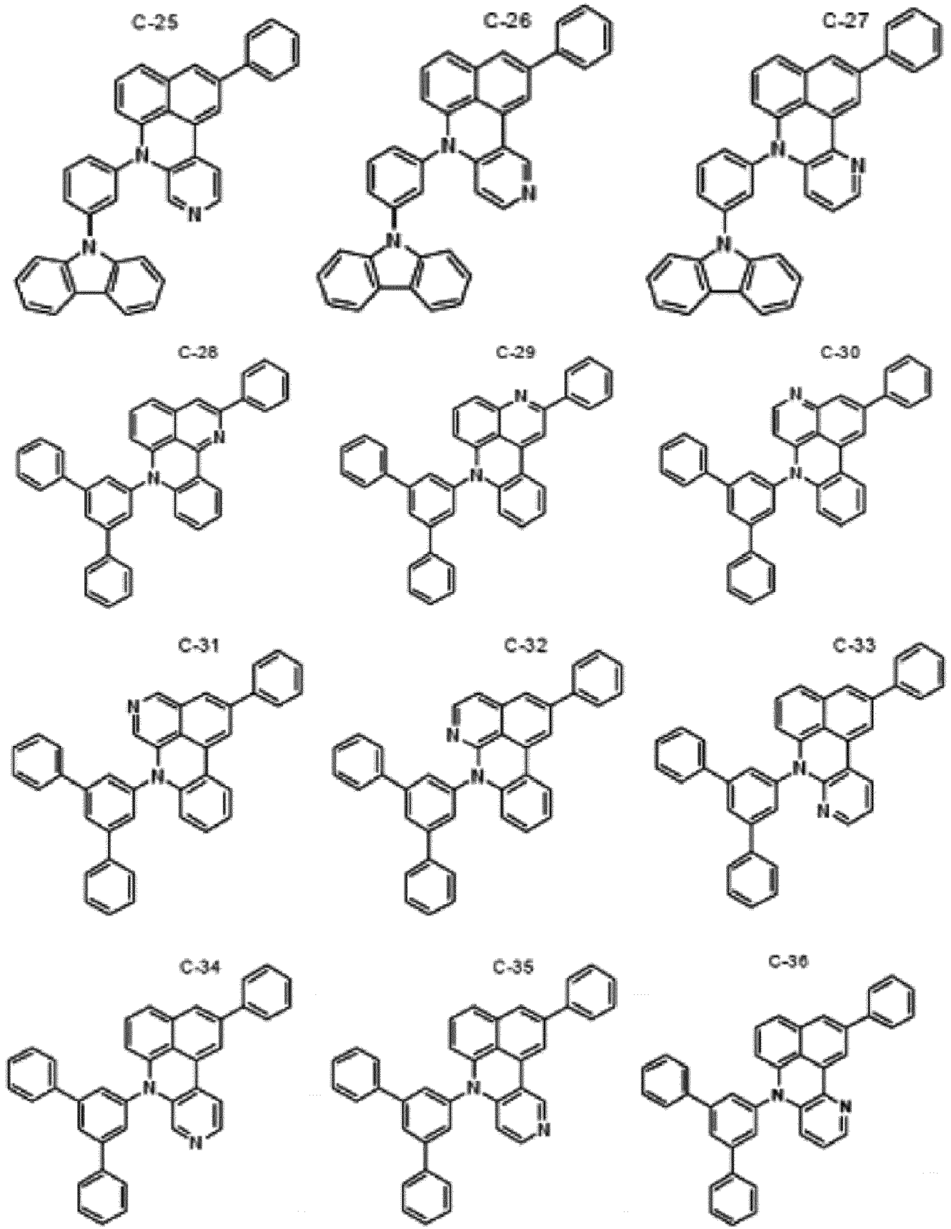

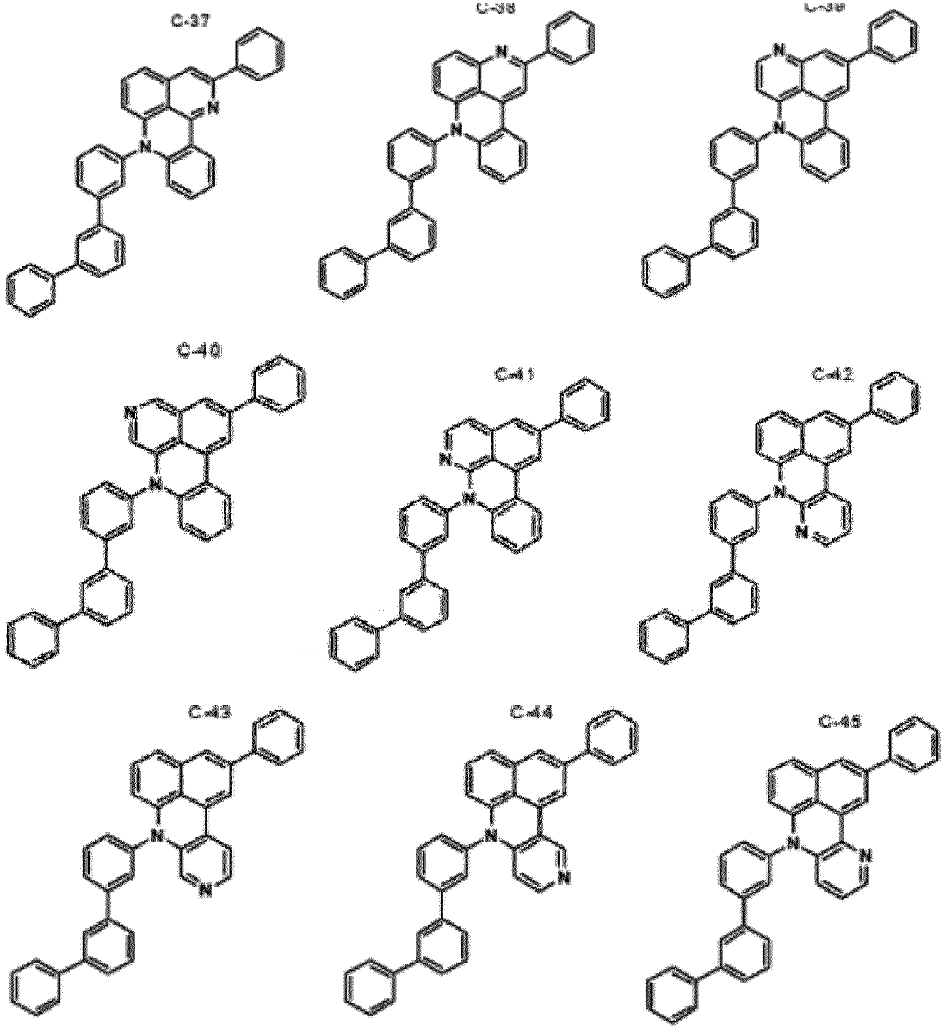

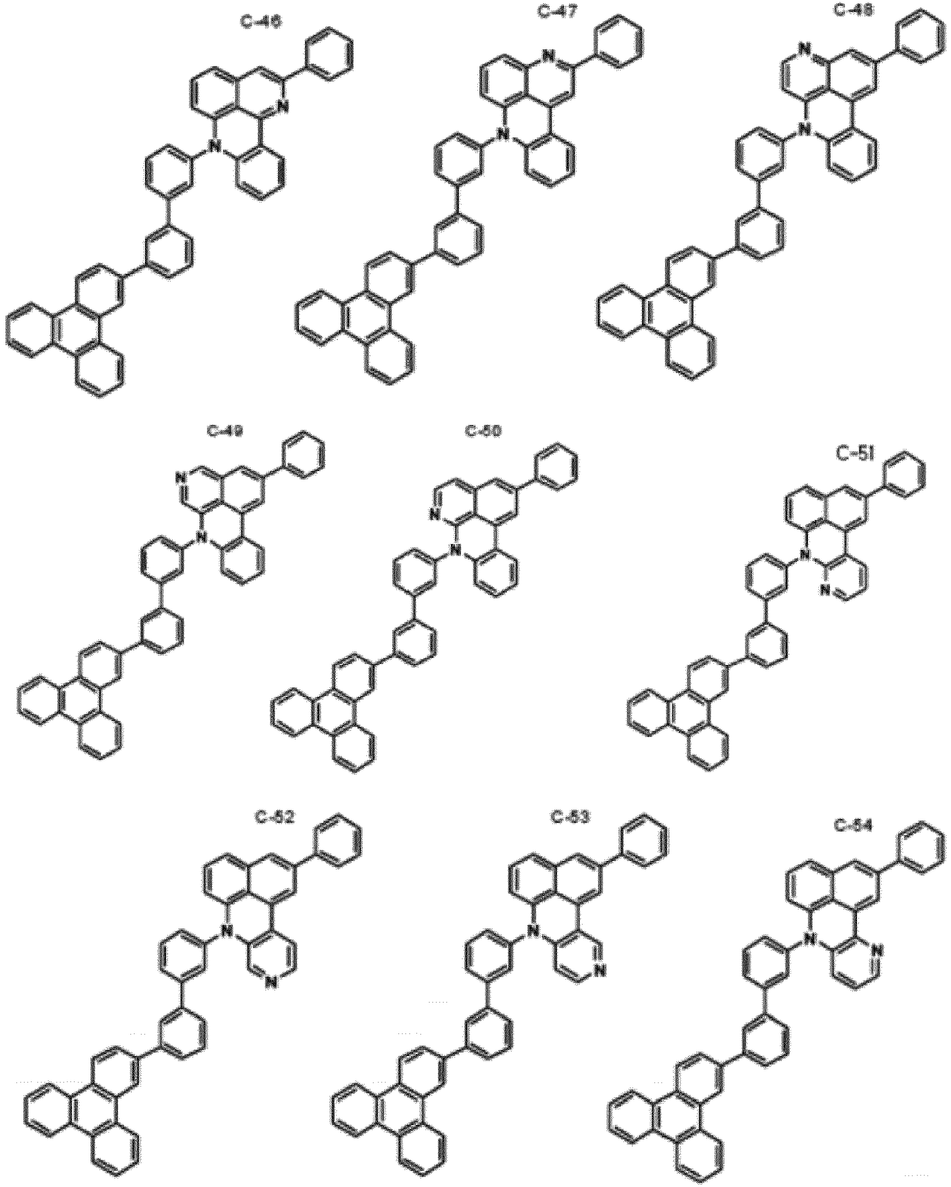

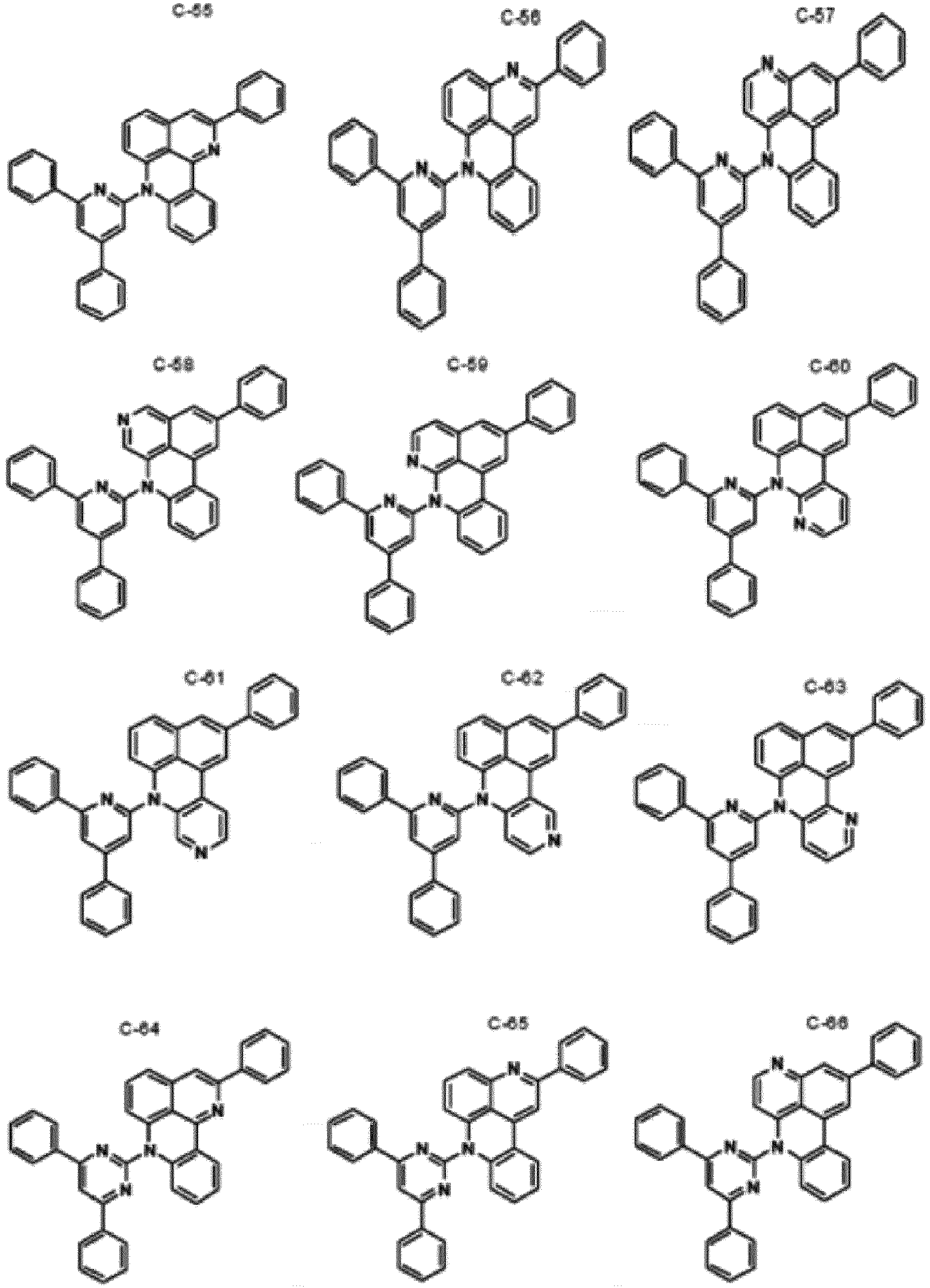

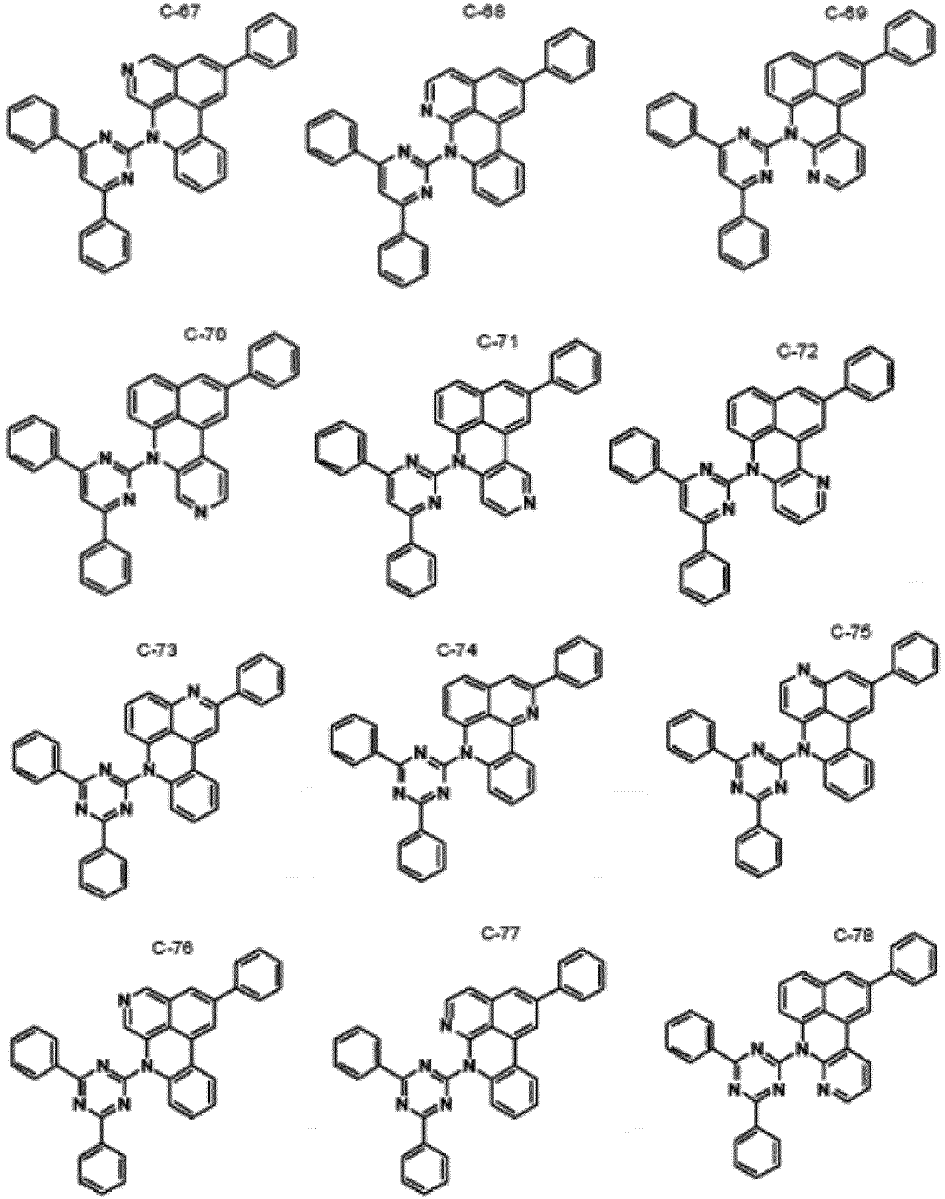

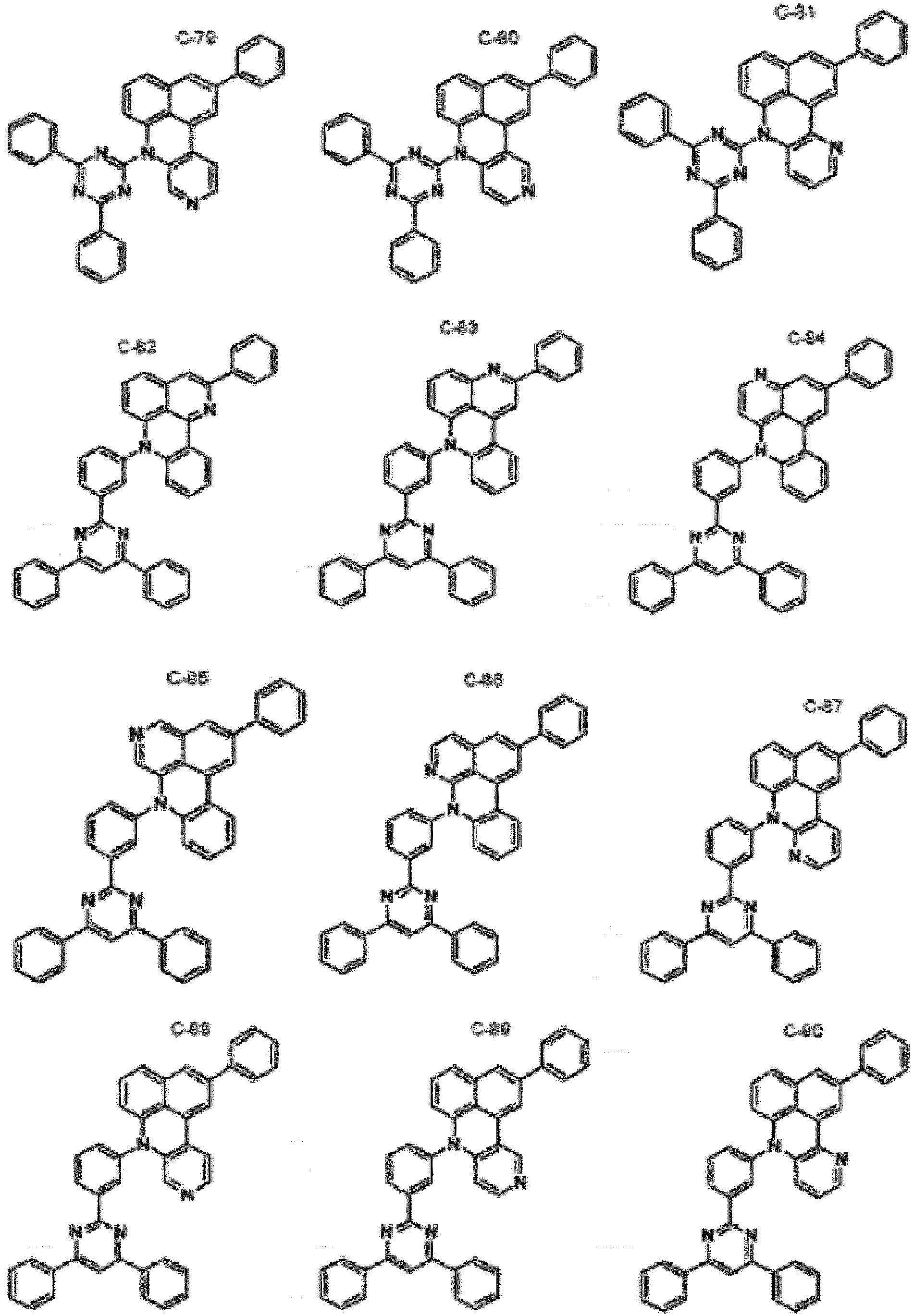

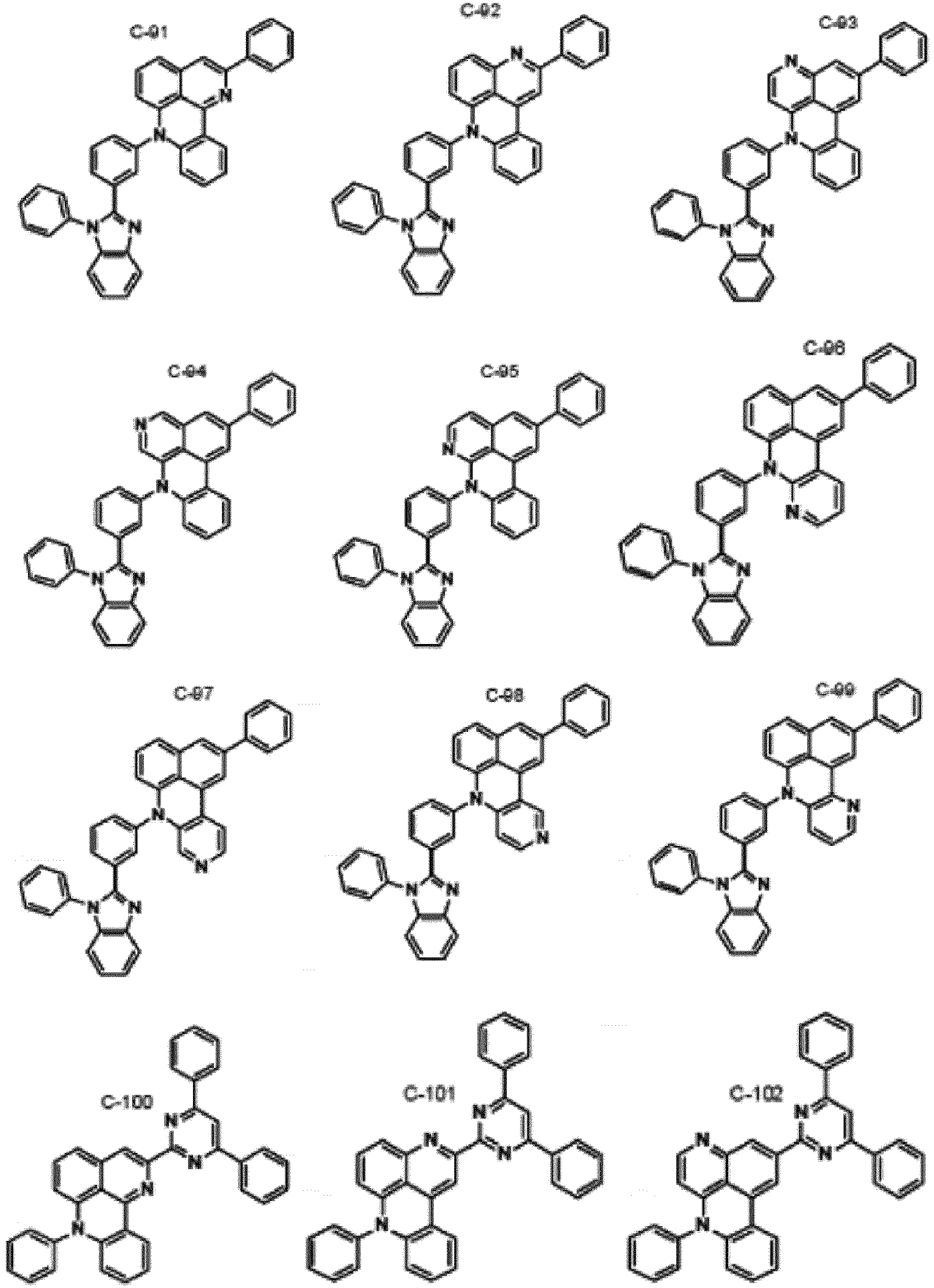

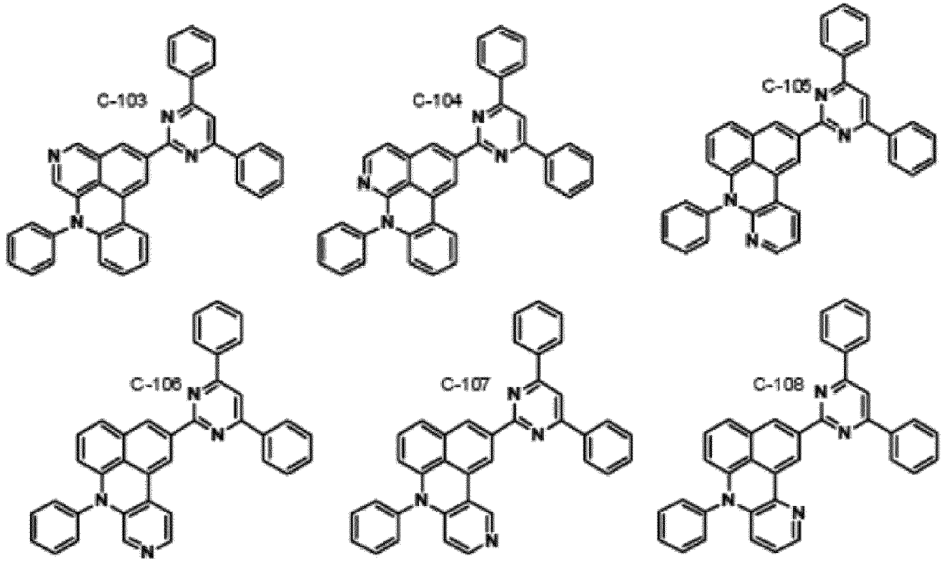

4. Dispositif optoélectronique organique comprenant
une anode (120) et une cathode (110) se faisant face, et
au moins une couche organique (130) positionnée entre l'anode et la cathode
dans lequel, la couche organique comprend le composé selon l'une quelconque des revendications 1 et 2.

5. Dispositif optoélectronique organique selon la revendication 4, dans lequel la couche organique comprend une couche d'émission,
dans lequel la couche d'émission comprend le composé.

6. Dispositif optoélectronique organique selon la revendication 5, dans lequel le composé est inclus en tant qu'hôte de la couche d'émission.

7. Dispositif optoélectronique organique selon la revendication 4, dans lequel la couche organique comprend au moins une couche auxiliaire sélectionnée parmi une couche d'injection de trous, une couche de transport de trous, une couche de blocage d'électrons, une couche de transport d'électrons, une couche d'injection d'électrons et une couche de blocage de trous,
dans lequel la couche auxiliaire comprend le composé.

8. Dispositif optoélectronique organique comprenant
une anode (120) et une cathode (110) se faisant face, et
au moins une couche organique (130) positionnée entre l'anode et la cathode
dans lequel, la couche organique comprend le composé selon la revendication 3.

9. Dispositif d'affichage comprenant le dispositif optoélectronique organique selon la revendication 4.

10. Dispositif d'affichage comprenant le dispositif optoélectronique organique selon la revendication 8.
